(19) Europäisches Patentamt
European Patent Office
Office européen des brevets

(11) EP 4 682 173 A1

(12) **EUROPEAN PATENT APPLICATION**
published in accordance with Art. 153(4) EPC

(43) Date of publication:
**21.01.2026 Bulletin 2026/04**

(21) Application number: **24770998.3**

(22) Date of filing: **15.03.2024**

(51) International Patent Classification (IPC):
***C07K 19/00*** *(2006.01)* ***C07K 7/08*** *(2006.01)*
***C07K 16/00*** *(2006.01)*

(52) Cooperative Patent Classification (CPC):
**C07K 7/08; C07K 16/00; C07K 19/00**

(86) International application number:
**PCT/JP2024/010181**

(87) International publication number:
**WO 2024/190896 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2023 JP 2023040529**

(71) Applicants:
- **Kagoshima University**
  **Kagoshima-shi, Kagoshima 890-8580 (JP)**
- **Peptide Institute, Inc.**
  **Ibaraki-shi, Osaka 567-0085 (JP)**

(72) Inventors:
- **KISHIMOTO Satoshi**
  **Tokyo 136-0075 (JP)**
- **KOTANI Hiroshi**
  **Tokyo 136-0075 (JP)**
- **ITO Yuji**
  **Kagoshima-shi, Kagoshima 890-8580 (JP)**
- **YOSHIYA Taku**
  **Ibaraki-shi, Osaka 567-0085 (JP)**
- **TSUDA Shugo**
  **Ibaraki-shi, Osaka 567-0085 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **METHOD FOR PRODUCING RADIOLABELED ANTIBODY, RADIOLABELED ANTIBODY, AND RADIOACTIVE MEDICINE**

(57) The method for producing a radiolabeled antibody includes a conjugation step of reacting a compound represented by Formula (1): P1-S-Y-B or a salt thereof with IgG. In Formula (1), P1 is a labeling site labeled with a radioisotope or a labeling site that can be labeled with a radioisotope. S is a spacer. Y is a reaction site that reacts with IgG. B is an antibody binding site including an IgG-binding peptide that specifically binds to the Fc region of IgG. Y in Formula (1) has a group represented by Formula I. $R^1$ is a substituent in which a pKa of $R^1$-H is 4 to 14. $R^2$ is bonded to S in Formula (1). $R^3$ is bonded to B in Formula (1). $R^4$ is H or a substituted or unsubstituted alkyl group having 1 to 8 carbon atoms.

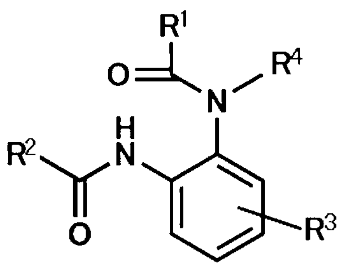

Formula I



Processed by Luminess, 75001 PARIS (FR)

## Description

Technical Field

The present invention relates to a method for producing a radiolabeled antibody, a radiolabeled antibody, and a radiopharmaceutical.

Background Art

Development of antibody drugs that have enhanced functionality by adding a drug, for example, an antibody drug conjugate (ADC) and the like is in progress. For the modification of antibodies, various methods such as random modification of amino groups and thiol modification at the hinge region have been used.

Patent Literature 1 to 3 disclose the chemical conjugation by affinity peptide (CCAP) method, which is a site-specific modification method for preparing conjugates (complexes) of antibodies and drugs or the like.

In the CCAP method, an antibody-modifying reagent having an IgG-binding peptide (IgG-BP) that specifically binds to the Fc region of IgG is reacted with the antibody. A predetermined amino acid residue of the IgG-BP is modified with disuccinimidyl glutarate (DSG) or dithiobis(succinimidyl propionate) (DSP), and has an N-hydroxysuccinimide (NHS) group. In the reaction between the antibody-modifying reagent and the antibody, IgG-BP binds to a specific site in the Fc region via an NHS group.

Since the structure of the Fc region is symmetrical, the Fc region typically has two IgG-BP binding sites. Therefore, in the CCAP method, a bivalent modified antibody in which IgG-BP is bound to both IgG-BP binding sites, and a monovalent modified antibody in which IgG-BP is bound to one of the IgG-BP binding sites, are obtained.

Patent Literature 2 discloses that, by using the CCAP method to label an antibody with a radioactive metal nuclide, a monovalent radiolabeled antibody modified with one molecule of a chelate-conjugated peptide per one molecule of antibody, and a bivalent radiolabeled antibody modified with two molecules of a chelate-conjugated peptide per one molecule of antibody, were obtained. Furthermore, Patent Literature 2 discloses that positron emission tomography (PET) imaging of tumor-bearing mice was performed using each of monovalent $^{89}$Zr-trastuzumab and bivalent $^{89}$Zr-trastuzumab.

Citation List

Patent Literature

Patent Literature 1: International Patent Publication 2016/186206
Patent Literature 2: International Patent Publication 2017/217347
Patent Literature 3: International Patent Publication 2018/230257
Patent Literature 4: International Patent Publication 2021/080008

Summary of Invention

In the images obtained from the PET imaging of tumor-bearing mice disclosed in Patent Literature 2, the monovalent $^{89}$Zr-trastuzumab exhibited higher radioactivity accumulation in HER2-overexpressing tumors compared to the bivalent $^{89}$Zr-trastuzumab. The present inventors consider the following regarding this result. In the monovalent radiolabeled modified antibody, IgG-BP remains in the Fc chain on one side, but does not remain in the Fc chain on the other side, and the binding ability to FcRn is maintained. On the other hand, in the case of the bivalent modified antibody, since IgG-BP remains on both Fc chains, the binding ability to FcRn is lost. As a result, the bivalent radiolabeled modified antibody shows reduced blood retention and is rapidly eliminated from the body. On the other hand, in the case of the monovalent radiolabeled modified antibody, although the binding ability to FcRn is retained, a reduction in blood retention is observed compared to the unmodified antibody. Based on this consideration, in order to improve blood retention in radiolabeled modified antibodies prepared using the CCAP method, purification of the monovalent radiolabeled modified antibody using methods disclosed in Patent Literature 4 and the like has been conducted to increase the purity of the monovalent radiolabeled modified antibody. In the radiolabeled modified antibody prepared using the CCAP method described in Patent Literature 2, improvement in blood retention and improvement in productivity were in a trade-off relationship.

The present invention has been made in view of the above circumstances, and an object thereof is to provide a method for producing a radiolabeled antibody, a radiolabeled antibody, and a radiopharmaceutical having higher blood

retention compared to conventional bivalent radiolabeled antibodies prepared using the CCAP method.

According to a first aspect of the present invention, there is provided a method for producing a radiolabeled antibody, including:

a conjugation step of reacting a compound represented by the following Formula (1) or a salt thereof, with IgG,

P1-S-Y-B Formula (1)

wherein
P1 is a labeling site labeled with a radioisotope or a labeling site capable of being labeled with a radioisotope,
S is a spacer,
Y is a reaction site that reacts with IgG, and
B is an antibody binding site containing an IgG-binding peptide that specifically binds to an Fc region of IgG), and wherein Y in the above Formula (1) has a group represented by Formula I below:

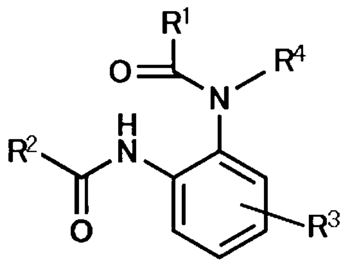

Formula I

wherein
$R^1$ is a substituent such that a pKa of $R^1$-H is 4 to 14,
$R^2$ is bound to S in the above Formula (1),
$R^3$ is bound to B in the above Formula (1), and
$R^4$ is H or a substituted or unsubstituted alkyl group having 1 to 8 carbon atoms.

The radiolabeled antibody according to a second aspect of the present invention is represented by Formula (4) below:

(P2-S)n-Ab Formula (4)

wherein

P2 contains a radioisotope,
S is a spacer,
Ab is IgG, and
n is 1 or 2, wherein S in the above Formula (4) includes Lys-Gly-Gly, an amino group of a side chain of Lys in S in the above Formula (4) is substituted with P2, and
S in the above Formula (4) is specifically bound to Lys in an Fc region of IgG.

The radiolabeled antibody according to a third aspect of the present invention is represented by Formula (4) below:

(P2-S)n-Ab Formula (4)

wherein

P2 contains a radioisotope,
S is a spacer,
Ab is IgG, and
n is 1 or 2, wherein
S in the above Formula (4) is selected from the group consisting of a polyethylene glycol chain, an alkylene chain, and a combination thereof, and

S in the above Formula (4) is specifically bound to Lys in an Fc region of IgG.

A radiopharmaceutical according to a fourth aspect of the present invention contains,
as an active ingredient, the radiolabeled antibody according to the second or third aspect of the present invention.

The radiolabeled antibody and the radiopharmaceutical according to the present invention have higher blood retention than a bivalent radiolabeled antibody using the conventional CCAP method.

Brief Description of Drawings

Fig. 1 is a diagram schematically showing a reaction of modifying an antibody with a compound according to an embodiment of the present invention.
Fig. 2 is a diagram showing an elution chromatogram obtained by liquid chromatography mass spectrometry (LC-MS) of an antibody solution according to Example 1.
Fig. 3A is a diagram showing a result of mass spectrometry of a peak of IgG in an antibody solution before addition of Compound A according to Example 1.
Fig. 3B is a diagram showing a result of mass spectrometry of a peak of IgG in a reaction solution after 2 hours from the start of the reaction according to Example 1.
Fig. 4A is a diagram showing corrected count values regarding antigen binding activities of Example 1 and Comparative Example 1 according to Test Example 1.
Fig. 4B is a diagram showing a ratio between an average value of corrected values of SK-OV-3 and an average value of corrected values of MDA-MB-231 regarding the antigen binding activities of Example 1 and Comparative Example 1 according to Test Example 1.
Fig. 5 is a diagram showing time-dependent change in the accumulation rate of administered radioactivity per unit weight(%ID/g) of blood of mice according to Test Example 2.
Fig. 6A is a diagram showing time-dependent change in the accumulation rate of administered radioactivity per unit weight of the liver of mice according to Test Example 2.
Fig. 6B is a diagram showing time-dependent change in the accumulation rate of administered radioactivity per unit weight of the kidney of mice according to Test Example 2.
Fig. 6C is a diagram showing time-dependent change in the accumulation rate of administered radioactivity per unit weight of the femur of mice according to Test Example 2.
Fig. 6D is a diagram showing time-dependent change in the accumulation rate of administered radioactivity per unit weight of the thigh muscle of mice according to Test Example 2.
Fig. 6E is a diagram showing time-dependent change in the accumulation rate of administered radioactivity per unit weight of the spleen of mice according to Test Example 2.
Fig. 7A is a diagram showing a result of mass spectrometry of a peak of IgG in an antibody solution before addition of Compound A according to Example 2.
Fig. 7B is a diagram showing a result of mass spectrometry of a peak of IgG in a reaction solution containing Compound A according to Example 2.
Fig. 8A is a diagram showing a result of mass spectrometry of a peak of IgG in an antibody solution before addition of Compound B according to Example 2.
Fig. 8B is a diagram showing a result of mass spectrometry of a peak of IgG in a reaction solution containing Compound B according to Example 2.
Fig. 9A is a diagram showing a result of mass spectrometry of a peak of IgG in an antibody solution before addition of Compound C according to Example 2.
Fig. 9B is a diagram showing a result of mass spectrometry of a peak of IgG in a reaction solution containing Compound C according to Example 2.
Fig. 10A is a diagram showing a result of mass spectrometry of a peak of IgG in an antibody solution before addition of Compound D according to Example 2.
Fig. 10B is a diagram showing a result of mass spectrometry of a peak of IgG in a reaction solution containing Compound D according to Example 2.
Fig. 11A is a diagram showing a result of mass spectrometry of a peak of IgG in an antibody solution before addition of Compound A according to Example 3.
Fig. 11B is a diagram showing a result of mass spectrometry of a peak of IgG in a reaction solution containing Compound A according to Example 3.
Fig. 12A is a diagram showing a result of mass spectrometry of a peak of IgG in an antibody solution before addition of Compound B according to Example 3.

Fig. 12B is a diagram showing a result of mass spectrometry of a peak of IgG in a reaction solution containing Compound B according to Example 3.
Fig. 13A is a diagram showing a result of mass spectrometry of a peak of IgG in an antibody solution before addition of Compound C according to Example 3.
Fig. 13B is a diagram showing a result of mass spectrometry of a peak of IgG in a reaction solution containing Compound C according to Example 3.
Fig. 14 is a diagram showing results of size exclusion chromatography (SEC) analysis in Test Example 4.
Fig. 15 is a diagram showing corrected count values regarding the antigen binding activity of a radioconjugate according to Example 2 in Test Example 5.
Fig. 16A is a diagram showing time-dependent change in accumulation of the radioconjugate according to Example 2 in a tumor in Test Example 6.
Fig. 16B is a diagram showing time-dependent change in accumulation of the radioconjugate according to Example 2 in the heart in Test Example 6.
Fig. 16C is a diagram showing time-dependent change in accumulation of the radioconjugate according to Example 2 in the liver in Test Example 6.
Fig. 16D is a diagram showing time-dependent change in accumulation of the radioconjugate according to Example 2 in the kidney in Test Example 6.
Fig. 17 is a diagram showing corrected count values regarding the antigen binding activity of a radioconjugate according to Example 3 in Test Example 7.
Fig. 18A is a diagram showing time-dependent change in accumulation of the radioconjugate according to Example 3 in the tumor in Test Example 8.
Fig. 18B is a diagram showing time-dependent change in accumulation of the radioconjugate according to Example 3 in the heart in Test Example 8.
Fig. 18C is a diagram showing time-dependent change in accumulation of the radioconjugate according to Example 3 in the liver in Test Example 8.
Fig. 18D is a diagram showing time-dependent change in accumulation of the radioconjugate according to Example 3 in the kidney in Test Example 8.
Fig. 19 is a diagram showing corrected count values regarding the antigen binding activity of a radioconjugate according to Example 4 in Test Example 10.
Fig. 20 is a diagram showing the time-dependent change in tumor volume in tumor-bearing mice administered with the radioconjugate according to Example 4 in Test Example 11.

Description of Embodiments

Hereinafter, embodiments according to the present invention will be described with reference to the drawings. In the drawings, the same or equivalent parts will be given the same reference numerals. Note that the present invention is not limited by the following embodiments and drawings. In the following embodiments, the expression "having", "including" or "containing" also includes the meaning of "consisting of" or "composed of".

(Method for Producing Radiolabeled Antibody)

The method for producing a radiolabeled antibody according to the present embodiment includes a conjugation step of reacting a compound represented by the following Formula (1) or a salt thereof with IgG.

P1-S-Y-B Formula (1)

In Formula (1), P1 is a labeling site labeled with a radioisotope or a labeling site that can be labeled with a radioisotope. In the present embodiment, the radioisotope is not particularly limited as long as the radioisotope is an element that emits radiation and can be tracked as a radiolabeled antibody. As the radioisotope, a radionuclide that emits radiation of $\alpha$ rays, $\beta$ rays, $\gamma$ rays, or a combination thereof can be used. Examples of such a radioisotope include a radioisotope of a halogen element, an alkali metal, an alkaline earth metal, a lanthanoid, an actinoid, a transition metal, or a metal other than these.

The radioisotope is preferably a radioactive metal nuclide. From the viewpoint of being commercially available and improving the complexation ability, it is preferable to use $^{44}Sc$, $^{51}Cr$, $^{57}Co$, $^{58}Co$, $^{60}Co$, $^{59}Fe$, $^{67}Ga$, $^{68}Ga$, $^{64}Cu$, $^{67}Cu$, $^{89}Sr$, $^{89}Zr$, $^{90}Y$, $^{99m}Tc$, $^{103}Ru$, $^{111}In$, $^{153}Sm$, $^{165}Dy$, $^{166}Ho$, $^{177}Lu$, $^{186}Re$, $^{188}Re$, $^{198}Au$, $^{201}Tl$, $^{197}Hg$, $^{203}Hg$, $^{212}Bi$, $^{213}Bi$, $^{212}Pb$, $^{227}Th$, or, $^{225}Ac$. Examples of the radioisotope include radioactive halogen atoms such as a radioactive fluorine atom ($^{18}F$), a radioactive chlorine atom ($^{36}Cl$), a radioactive bromine atom ($^{75}Br$, $^{76}Br$, $^{77}Br$, $^{80}Br$), a radioactive iodine atom ($^{123}I$, $^{124}I$, $^{125}I$), and a radioactive astatine atom ($^{221}At$), $Al^{18}F$, $^{64}Cu$, $^{68}Ga$, $^{89}Zr$, 90y, $^{99m}Tc$, $^{111}In$, $^{177}Lu$, and $^{225}Ac$.

When the labeling site is labeled with a radioisotope, P1 has a reactive functional group, and after the conjugation step, the labeling site is labeled with a radioisotope. Examples of reactive functional groups include imidazole group, hydroxyl group, amino group, thiol group, halogen atom, sulfonate ester group, epoxy group, isocyanato group, azide group, vinyl group, maleimide group, ethynyl group (-C=CH), diene, alkyne, bicyclo 6.1.0 nonene (BCN), dibenzocyclooctyne (DBCO) group, trans-cyclooctene (TCO), and tetrazine. Preferably, the reactive functional group is an azide group, and a radioisotope is added to P1 by a click reaction with an alkyne, a DBCO group or the like.

S in Formula (1) is a spacer. The spacer is, for example, selected from the group consisting of -C-, -NH-, - O-, -S-, -C(=O)-NH-, -NH-C(=O)-, -C(=O)-O-, -O-C(=O)-, - C(=O)-, polyoxyalkylene groups, amino acid residues, peptide chains, polyethylene glycol (PEG) chains, and combinations thereof. The spacer may be selected from the group consisting of a substituted or unsubstituted alkyl group having 1 to 8 carbon atoms, a substituted or unsubstituted alkenyl group having 2 to 8 carbon atoms, a substituted or unsubstituted alkynyl group having 2 to 8 carbon atoms, a substituted or unsubstituted peptide chain having 2 to 50 amino acid residues, a substituted or unsubstituted polymer chain having a polymerization degree of 2 to 50, and combinations thereof. The number of amino acid residues in the peptide chain is preferably 2 to 30, 2 to 20, 2 to 10, or 2 to 5, and more preferably 3. The amino acid residue in the peptide chain may be an amino acid residue of a natural amino acid or an amino acid residue of an artificial amino acid, but may be, for example, one or more glycine residues or one or more lysine residues, or a combination thereof. Specific examples of the peptide chain include Lys-Gly-Gly. The polymer chain is not particularly limited as long as the polymer is a known polymer chain, and examples thereof include PEG. Specifically, the polymer chain may be a PEG chain having a polymerization degree of 2 to 50. The substituted peptide chain includes a peptide chain in which a substituent is bound to a main chain or a side chain. Substituted polymer chains include those in which a substituent is bound to a main chain or a side chain. Preferably, the spacer contains Lys-Gly-Gly, and the amino group of the side chain of Lys is substituted with P1 in the above Formula (1).

"Alkyl group" means a saturated hydrocarbon group. The alkyl group includes a cyclic (including fused bicyclic) alkyl group satisfying the condition of having a required number of carbon atoms, a linear and branched alkyl group, and a linear or branched alkyl group substituted with a cyclic alkyl group. Examples of alkyl groups include methyl group, ethyl group, n-propyl group, iso-propyl group, cyclopropyl group, n-butyl group, isobutyl group, sec-butyl group, t-butyl group, n-pentyl group, 1,1-dimethylpropyl group, 1,2-dimethylpropyl group, 2,2-dimethylpropyl group, 1-ethylpropyl group, 2-ethylpropyl group, n-hexyl group, cyclohexyl group, cyclooctyl group, and 1-methyl-2-ethylpropyl group.

The term "alkenyl group" means a hydrocarbon group having at least one double bond. The term alkenyl includes both linear alkenyl groups and branched alkenyl groups. Examples of alkenyl groups include ethenyl group, n-propenyl group, iso-propenyl group, n-butenyl group, isobutenyl group, sec-butenyl group, t-butenyl group, n-pentenyl group, 1,1-dimethylpropenyl group, 1,2-dimethylpropenyl group, 2,2-dimethylpropenyl group, 1-ethylpropenyl group, 2-ethylpropenyl group, n-hexenyl group, and 1-methyl-2-ethylpropenyl group.

The term "alkynyl group" means a hydrocarbon group having at least one triple bond. The term alkynyl includes both linear alkynyl groups and branched chain alkynyl groups. Examples of alkynyl groups include ethynyl group, n-propynyl group, iso-propynyl group, n-butynyl group, iso-butynyl group, sec-butynyl group, t-butynyl group, and n-pentynyl group.

The chain length of the PEG chain contained in the spacer is not particularly limited. The polymerization degree of the PEG chain is, for example, 2 to 30, 3 to 28, 3 to 27, 3 to 25, or 3 to 24. The spacer may have an alkylene chain (-$C_mH2_m$-) having m carbon atoms. The carbon number m of the alkylene chain is, for example, 1 to 10, 1 to 8, 1 to 6, or 1 to 4. The spacer may contain a PEG chain and an alkylene chain, and for example, the PEG chain bound to Y may be bound to P1 via the alkylene chain. In this case, when the polymerization degree of the PEG chain is n, the above Formula (1) is represented by P1-$C_mH2_m$-(PEG)n-Y-B.

Y in Formula (1) is a reaction site that reacts with IgG. Here, "IgG" may be IgG derived from a mammal, such as a primate including humans and chimpanzees; laboratory animals such as rats, mice, and rabbits; livestock animals such as pigs, cattle, horses, sheep, and goats; or companion animals such as dogs and cats. As the IgG, a chimeric antibody of a monoclonal antibody, a humanized antibody, or a human antibody is further preferable. Also, subclasses of these antibodies can be used without particular limitation. It is preferably human IgG (IgG1, IgG2, IgG3, or IgG4). When human IgG is used as IgG, at least one of IgG1, IgG2, IgG3, and IgG4 can be used as IgG. The IgG may be a rabbit IgG. The antibody may be a full-length antibody or an antibody fragment (F(ab')2, Fab', Fab, Fv, single chain antibody), and is preferably a full-length antibody.

Y in Formula (1) has a group represented by the following Formula I.

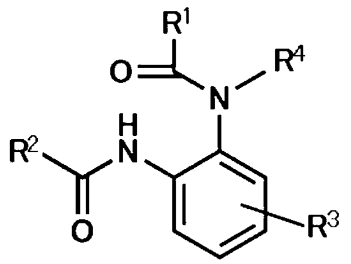

Formula I

In Formula I, $R^1$ is a substituent having an acid dissociation constant (pKa) of $R^1$-H of 4 to 14, 4 to 12, or 4 to 10, and preferably 5 to 9. Here, the pKa is a pKa at room temperature (for example, 25°C). The compound becomes an activated intermediate by the leaving of $R^1$. The pKa of $R^1$-H determines the ease of leaving, that is, the ease of transition to an activated intermediate. When the pKa is high, the transition becomes difficult, and when the pKa is low, the transition becomes easier. However, when the pKa is too low, the compound may become unstable and undergo hydrolysis. Preferably, $R^1$-H is a phenol. Examples of $R^1$-H include salicylic acid, phenol, 4-fluorophenol, 4-nitrophenol, 2,6-dichlorophenol, N-hydroxysuccinimide, 2,3,5,6-tetrafluorophenol, pentafluorophenol, aminophenol, and dinitrophenol. Suitably, $R^1$-H is 4-nitrophenol and $R^1$ is a nitrophenoxy group.

$R^2$ in the above Formula I is bound to S in the above Formula (1). $R^3$ in the above Formula I is bound to B in the above Formula (1). B is an antibody binding site including an IgG-BP that specifically binds to the Fc region of IgG. The "Fc region of IgG" means a C-terminal fragment obtained by digestion of IgG with the proteolytic enzyme papain.

$R^3$ may be IgG-BP. $R^3$ may have a linker and IgG-BP, where * is the carbon of the benzene ring of Formula I, and $R^3$ may be *-(linker)-(IgG-BP). The linker is, for example, selected from the group consisting of -NH-, -O-, -S-, - C(=O)-NH-, -NH-C(=O)-, -C(=O)-O-, -O-C(=O)-, -C(=O)-, polyoxyalkylene groups, amino acid residues, peptide chains, PEG chains, and combinations thereof.

IgG-BP in $R^3$ is bound to a carbon of the benzene ring of Formula I or a linker via a main chain or a side chain of any amino acid residue. Preferably, the IgG-BP in $R^3$ is bound to the carbon of the carbonyl group in Formula I or to a linker via the amino group of the N-terminal main chain or side chain.

Preferably, B in Formula (1) is represented by any one of the following Formulas II, III, and IV with IgG-BP as $R^{3a}$. In Formulas II, III, and IV, * represents a carbon of the benzene ring of Formula I.

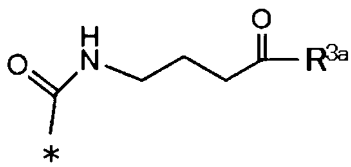

Formula II

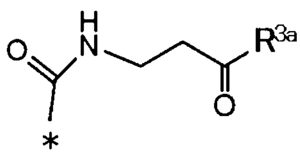

Formula III

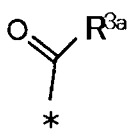

Formula IV

IgG-BP is a peptide bound to a site selected from Lys246, Lys248, Lys288, Lys290, Lys338, Lys360, Lys414, and Lys439 according to Eu numbering in Fc, or a proximal region thereof, preferably bound to Lys248 or a proximal region thereof, or bound to a binding region of protein A. For example, the IgG-BP may be a partial peptide of protein A having Fc-binding ability, or a variant thereof. Specific examples of such peptides are described in International Patent Publication 2008/054030, International Patent Publication 2013/027796, Patent Literature 1 to 3, and the like. IgG-BP can be appropriately prepared according to a known peptide synthesis method, for example, a peptide solid phase synthesis method or a method described in each of the above Literature.

More specifically, $R^3$ is exemplified in the following (i) to (iii).

(i) A substituent containing IgG-BP represented by the following Formula (PI)

$NH_2$- (Linker) - ($X^1_{1-3}$)-C-($X^2$)-($X^3$)- ($X^4$)-($X^5$)-G-($X^6$)-L-($X^7$)-W-C-($X^8_{1-3}$)-(Linker) (SEQ ID NO. 155)... Formula (PI)

wherein, each (Linker) is independently the same or different linker or is absent, and here, the linker is RRRGS (SEQ ID NO. 156), EEGGS (SEQ ID NO. 157), $(GSGGS)_{1-3}$ (SEQ ID NO. 158 to 160) or $(PEG)_{1-10}$, preferably $(PEG)_{1-8}$ or $(PEG)_{2-10}$, more preferably $(PEG)_4$,
1 to 3 $X^1$, $X^2$, $X^3$, $X^4$, $X^5$, $X^6$, $X^7$, and 1 to 3 $X^8$ each independently represent the same or different amino acid residues,
$X^1$, $X^2$, $X^3$, and each $X^8$ each independently represent the same or different and any amino acid residue other than C,
$X^4$ is H, R, S, or D,
$X^5$ is one amino acid residue selected from K, C, D, E, R, V, F, L, 2-aminosuberic acid, Dpr, Orn, AceOrn, AceDab, Dab, Nle, Nva, Tle, Ala(t-Bu), and Cha,
$X^6$ is E, N, R, or D, and
$X^7$ is I or V.

Dab, Tle, and Cha mean 2,4-diaminobutanoic acid, tert-leucine, and β-cyclohexyl-L-alanine, respectively. Ace and t-Bu mean having an acetyl group and a tert-butyl group, respectively.
In Formula (PI), for the purpose of bonding, an amino group may be bound to the terminal (-COOH) of the C-terminal amino acid residue of Formula (PI) to form a $(-C(=O)NH_2)$ group. In Formula (PI), the N-terminal amino group may be acetylated. In this case, a Lys residue may be introduced at an appropriate position near the N-terminal in the linker.
Preferred examples of the IgG-BP contained in the substituent represented by Formula (PI) include the following peptides.

1. $X^1_{1-3}$ is an amino acid sequence represented by (S, G, F, or none)-(D, G, A, S, P, Hcy, or none)-(S, D, T, N, E, or R).
2. $X^1_{1-3}$ is D, GPD, R, GPR, SPD, GDD, GPS, SDD, RGN, G-Hcy-D, RGP, or GPD.
3. $X^1_{1-3}$ is D or GPD.
4. $X^2$ is A, S, or T.
5. $X^2$ is A or T.
6. $X^2$ is A.
7. $X^3$ is Y or W.
8. $X^3$ is Y.
9. $X^4$ is H.
10. $X^5$ is one amino acid residue selected from A, R, K, C, D, E, L, 2-aminosuberic acid, Dpr, R, F, 2-aminosuberic acid, Dpr, AceOrn, AceDab, Dab, Nle, Nva, Ala(t-Bu), and Cha.
11. $X^5$ is K, R, or AceOrn.
12. $X^5$ is one amino acid residue selected from V, Dab, F, R, L, Nva, Nle, Ala(t-Bu), and Cha.
13. $X^5$ is one amino acid residue selected from F, R, L, Nva, Nle, Ala(t-Bu), and Cha.
14. $X^5$ is one amino acid residue selected from L, Ala(t-Bu), and Cha.
15. $X^6$ is E or N.
16. $X^6$ is E.
17. $X^7$ is V.
18. $X^8_{1-3}$ is (S, T, or D)-(H, G, Y, T, N, D, F, Hcy, or none) - (Y, F, H, M, or none) .
19. $X^8_{1-3}$ is T, TFH, S, SFH, THH, TFY, TYH, or T-Hcy-H.
20. $X^8_{1-3}$ is T or TFH.

The IgG-BP contained in the substituent represented by Formula (PI) may be any one or combination of two or more of the above conditions, and for example, may be a peptide satisfying the conditions described below: 8. and 9., 8. and 17., 9. and 17., 8., 9., and 17., or a combination of these with any one of 10. to 14.
More specifically, the amino acid sequence of IgG-BP may be an amino acid sequence shown in any one of SEQ ID NOs. 1 to 151. $X^5$ is the same as described above, and may have an $NH_2$-(Linker)-group at the N-terminal, and may have a -NH2 group or a -(Linker)-$NH_2$ group at the C-terminal.

1) DCAYH$X^5$GELVWCT (SEQ ID NO. 1)
2) GPDCAYH$X^5$GELVWCTFH (SEQ ID NO. 2)
3) RCAYH$X^5$GELVWCS (SEQ ID NO. 3)
4) GPRCAYH$X^5$GELVWCSFH (SEQ ID NO. 4)

5) SPDCAYHX$^5$GELVWCTFH (SEQ ID NO. 5)
6) GDDCAYHX$^5$GELVWCTFH (SEQ ID NO. 6)
7) GPSCAYHX$^5$GELVWCTFH (SEQ ID NO. 7)
8) GPDCAYHX$^5$GELVWCSFH (SEQ ID NO. 8)
9) GPDCAYHX$^5$GELVWCTHH (SEQ ID NO. 9)
10) GPDCAYHX$^5$GELVWCTFY (SEQ ID NO. 10)
11) SPDCAYHX$^5$GELVWCTFY (SEQ ID NO. 11)
12) SDDCAYHX$^5$GELVWCTFY (SEQ ID NO. 12)
13) RGNCAYHX$^5$GQLVWCTYH (SEQ ID NO. 13)
14) G-Hcy-DCAYHX$^5$GELVWCT-Hcy-H (SEQ ID NO. 14)
15) RRGPDCAYHX$^5$GELVWCTFH (SEQ ID NO. 15)
16) DCTYHX$^5$GNLVWCT (SEQ ID NO. 16)
17) DCAYHX$^5$GNLVWCT (SEQ ID NO. 17)
18) DCTYHX$^5$GELVWCT (SEQ ID NO. 18)
19) DCAWHX$^5$GELVWCT (SEQ ID NO. 19)
20) DCTYTX$^5$GNLVWCT (SEQ ID NO. 20)
21) DCAYTX$^5$GNLVWCT (SEQ ID NO. 21)
22) DCSYTX$^5$GNLVWCT (SEQ ID NO. 22)
23) DCTWTX$^5$GNLVWCT (SEQ ID NO. 23)
24) DCTYHX$^5$GNLVWCT (SEQ ID NO. 24)
25) DCTYRX$^5$GNLVWCT (SEQ ID NO. 25)
26) DCTYSX$^5$GNLVWCT (SEQ ID NO. 26)
27) DCTYTX$^5$GNLVWCT (SEQ ID NO. 27)
28) DCTYTX$^5$GELVWCT (SEQ ID NO. 28)
29) DCTYTX$^5$GRLVWCT (SEQ ID NO. 29)
30) DCTYTX$^5$GDLVWCT (SEQ ID NO. 30)
31) DCTYTX$^5$GNLIWCT (SEQ ID NO. 31)
32) DCAYHRGELVWCT (SEQ ID NO. 32)
33) GPDCAYHRGELVWCTFH (SEQ ID NO. 33)
34) RCAYHRGELVWCS (SEQ ID NO. 34)
35) GPRCAYHRGELVWCSFH (SEQ ID NO. 35)
36) SPDCAYHRGELVWCTFH (SEQ ID NO. 36)
37) GDDCAYHRGELVWCTFH (SEQ ID NO. 37)
38) GPSCAYHRGELVWCTFH (SEQ ID NO. 38)
39) GPDCAYHRGELVWCSFH (SEQ ID NO. 39)
40) GPDCAYHRGELVWCTHH (SEQ ID NO. 40)
41) GPDCAYHRGELVWCTFY (SEQ ID NO. 41)
42) SPDCAYHRGELVWCTFY (SEQ ID NO. 42)
43) SDDCAYHRGELVWCTFY (SEQ ID NO. 43)
44) DCTYHRGNLVWCT (SEQ ID NO. 44)
45) DCAYHRGNLVWCT (SEQ ID NO. 45)
46) DCTYHRGELVWCT (SEQ ID NO. 46)
47) DCAWHRGELVWCT (SEQ ID NO. 47)
48) DCTYTNGNLVWCT (SEQ ID NO. 48)
49) DCAYTNGNLVWCT (SEQ ID NO. 49)
50) DCSYTNGNLVWCT (SEQ ID NO. 50)
51) DCTWTNGNLVWCT (SEQ ID NO. 51)
52) DCTYHNGNLVWCT (SEQ ID NO. 52)
53) DCTYRNGNLVWCT (SEQ ID NO. 53)
54) DCTYSNGNLVWCT (SEQ ID NO. 54)
55) DCTYTRGNLVWCT (SEQ ID NO. 55)
56) DCTYTNGELVWCT (SEQ ID NO. 56)
57) DCTYTNGRLVWCT (SEQ ID NO. 57)
58) DCTYTNGDLVWCT (SEQ ID NO. 58)
59) DCTYTNGNLIWCT (SEQ ID NO. 59)

Examples of $R^3$ include substituents having the following structures.

60) GSGGS-GPDCAYHRGELVWCTFH-$NH_2$ (SEQ ID NO. 60)
$(PEG)_4$-GPDCAYHRGELVWCTFH-$NH_2$ (SEQ ID NO. 33)
61) GSGGS-DCAYHRGELVWCT-$NH_2$ (SEQ ID NO. 61)
$(PEG)_4$-DCAYHRGELVWCT-$NH_2$ (SEQ ID NO. 32)

In the present specification, IgG-BP includes a peptide to which a functional group Z is bound. For example, in the substituent represented by Formula (PI), a functional group Z may be bound to the end of (Linker). Examples of such a substituent include the following substituents.

62) Acetyl-K(Z)-RRRGS-GPDCAYHKGELVWCTFH-$NH_2$ (SEQ ID NO. 62)
63) Acetyl-K(Z)-EEGGS-GPDCAYHKGELVWCTFH-$NH_2$ (SEQ ID NO. 63)
64) Acetyl-K(Z)-$(PEG)_4$-GPDCAYHKGELVWCTFH-$NH_2$ (SEQ ID NO. 64)

$R^3$ may be a substituent in which a maleimide group, a DBCO group, a tetrazine group, or a TCO group is bound to the N-terminus or PEG of the following peptide, or may be a substituent in which an $NH_2$ group is bound to the C-terminus.

65) RRRGS-GPDCAYHKGELVWCTFH (SEQ ID NO. 65)
66) EEGGS-GPDCAYHKGELVWCTFH (SEQ ID NO. 66)
$(PEG)_4$-GPDCAYHKGELVWCTFH (SEQ ID NO. 64)

Examples of the substituent in which the functional group Z is bound to the end of (Linker) include the following peptides.

67) Acetyl-K(Z)RRRGS-DCAYHKGELVWCT-$NH_2$ (SEQ ID NO. 67)
68) Acetyl-K(Z)EEGGS-DCAYHKGELVWCT-$NH_2$ (SEQ ID NO. 68)
69) Acetyl-K(Z)-$(PEG)_4$-DCAYHKGELVWCT-$NH_2$ (SEQ ID NO. 69)

$R^3$ may be a substituent in which a maleimide group, a DBCO group, a tetrazine group, or a TCO group is bound to the N-terminus or PEG of the following peptide, or may be a substituent in which an $NH_2$ group is bound to the C-terminus.

70) RRRGS-DCAYHKGELVWCT (SEQ ID NO. 70)
71) EEGGS-DCAYHKGELVWCT (SEQ ID NO. 71)
$(PEG)_4$-DCAYHKGELVWCT (SEQ ID NO. 69)
(ii) A substituent containing an IgG-BP represented by the following Formula (PII), or a substituent containing an IgG-BP having an amino acid sequence in which one or several amino acids are added, deleted, and/or substituted at positions other than $X^9$ to $X^{14}$ in the amino acid sequence of Formula (PII)

$$X^9_{1-2}NMQX^{10}QX^{14}RFYEALHDPNLNEEQRNAX^{11}IX^{12}SIRDDX^{13}\text{-(Linker2)-}CONH_2\ldots \quad (PII)$$

wherein, (Linker2) is $(GSGGS)_{1-3}$ (SEQ ID NOs. 158 to 160), $(SGSGS)_{1-3}$ (SEQ ID NOs. 161 to 163), $(PEG)_{2-10}$-Lys (preferably $(PEG)_4$-Lys), SGSGSK (SEQ ID NO. 164), SRRCR (SEQ ID NO. 165), SRRK(Z)R (SEQ ID NO. 166), SRRCRRCRRC (SEQ ID NO. 167), SRRK(Z)RRK(Z)RRK(Z) (SEQ ID NO. 168), $(PEG)_{1-8}$-Lys (preferably $(PEG)_4$-Lys), or may be absent,
$X^9_{1-2}$ is selected from the group consisting of GF, AF, VF, LF, IF, MF, PF, FF, WF, KF, Orn-F, CF, DF, EF, β-alanine-F, 2-aminosuberinic acid-F, Dpr-F, $NH_2$-(PEG)n-CO(where n is 1 to 50)-F, F, K, Orn, C, D, E, 2-aminosuberinic acid, Dpr, and Acetyl-K,
$X^{10}$ is C or Q,
$X^{11}$ and $X^{12}$ are each independently selected from the group consisting of R, H, D, E, S, T, N, Q, Y, and C,
$X^{13}$ is C or P, or is absent, and
$X^{14}$ is R, C, K, or K(Z).

The end ($-NH_2$) of the N-terminal amino acid of Formula (PII) may be acetylated to form a ($CH_3$-C(=O)-NH-) group. In addition, the Cys residue (C) contained in the linker may be bound to another functional molecule via a maleimide group as necessary.

Preferred examples of the IgG-BP contained in the substituent represented by Formula (PII) include the following peptides.

21. $X^9$ is selected from the group consisting of GF, AF, βAlaF, $NH_2$-$(PEG)_n$-CO(n is 2 to 10)-F, F, K, Orn, C, Dpr, and Acetyl-K.
22. $X^9$ is selected from the group consisting of GF, F, K, and Acetyl-K.
23. $X^{10}$ is Q.
24. $X^{11}$ and $X^{12}$ are each independently selected from the group consisting of R, H, and E.
25. $X^{11}$ is R.
26. $X^{12}$ is R or K(Z) (preferably, Z is azide).

More specific examples of the substituent represented by Formula (PII) include the following substituents (here, a functional group may be bound to the contained Lys residue as necessary).

72) FNMQCQRRFYEALHDPNLNEEQRNARIRSIRDDC (SEQ ID NO. 72)
73) GFNMQCQRRFYEALHDPNLNEEQRNARIRSIRDDC (SEQ ID NO. 73)
74) KNMQCQRRFYEALHDPNLNEEQRNARIRSIRDDC (SEQ ID NO. 74)
75) GFNMQCQKRFYEALHDPNLNEEQRNARIRSIRDDC (SEQ ID NO. 75)
76) KNMQCQKRFYEALHDPNLNEEQRNARIRSIRDDC (SEQ ID NO. 76)
77) FNMQQQRRFYEALHDPNLNEEQRNARIRSIRDD (SEQ ID NO. 77)
78) GKNMQCQRRFYEALHDPNLNEEQRNARIRSIRDDC (SEQ ID NO. 78)

79) Acetyl-FNMQQQRRFYEALHDPNLNEEQRNARIRSIRDDP-SGSGSK-$NH_2$ (SEQ ID NO. 79)

80) Acetyl-FNMQCQRRFYEALHDPNLNEEQRNARIRSIRDDC-SGSGSK-$NH_2$ (SEQ ID NO. 80)

81) Acetyl-FNMQQQRRFYEALHDPNLNEEQRNARIRSIRDDP-$(PEG)_4$-Lys-$NH_2$ (SEQ ID NO. 81)

72-2) Acetyl-FNMQCQRRFYEALHDPNLNEEQRNARIRSIRDDC-$(PEG)_4$-Lys-$NH_2$ (SEQ ID NO. 72)

82) FNMQQQCRFYEALHDPNLNEEQRNARIRSIRDD-$NH_2$ (SEQ ID NO. 82)
83) FNMQQQRRFYEALHDPNLNEEQRNARIRSIRDDC-$NH_2$ (SEQ ID NO. 83)
84) FNMQQQRRFYEALHDPNLNEEQRNARIRSIRDDP-SRRK(Z)R-$NH_2$ (SEQ ID NO. 84)
85) FNMQQQRRFYEALHDPNLNEEQRNARIRSIRDDP-SRRCR-$NH_2$ (SEQ ID NO. 85)
86) FNMQQQCRFYEALHDPNLNEEQRNARICSIRDDP-SRRCRRCRRC-$NH_2$ (SEQ ID NO. 86)

87) FNMQQQK(Z)RFYEALHDPNLNEEQRNARIK(Z)SIRDDP-SRRK(Z)RRK(Z)RRK(Z)-$NH_2$ (SEQ ID NO. 87)

88) Acetyl-KNMQQQCRFYEALHDPNLNEEQRNARIRSIRDD-$NH_2$ (SEQ ID NO. 88)
89) Acetyl-KNMQQQRRFYEALHDPNLNEEQRNARIRSIRDDC-$NH_2$ (SEQ ID NO. 89)

90) Acetyl-KNMQQQRRFYEALHDPNLNEEQRNARIRSIRDDP-SRRK(Z)R-$NH_2$ (SEQ ID NO. 90)

91) Acetyl-KNMQQQRRFYEALHDPNLNEEQRNARIRSIRDDP-SRRCR-$NH_2$ (SEQ ID NO. 91)

92) Acetyl-KNMQQQCRFYEALHDPNLNEEQRNARICSIRDDP-SRRCRRCRRC-$NH_2$ (SEQ ID NO. 92)

93) Acetyl-KNMQQQK(Z)RFYEALHDPNLNEEQRNARIK(Z)SIRDDP-SRRK(Z)RRK(Z)RRK(Z)-$NH_2$ (SEQ ID NO. 93)

94) GFNMQQQCRFYEALHDPNLNEEQRNARIRSIRDD-$NH_2$ (SEQ ID NO. 94)
95) GFNMQQQRRFYEALHDPNLNEEQRNARIRSIRDDC-$NH_2$ (SEQ ID NO. 95)
96) GFNMQQQRRFYEALHDPNLNEEQRNARIRSIRDDP-SRRK(Z)R-$NH_2$ (SEQ ID NO. 96)

97) GFNMQQQRRFYEALHDPNLNEEQRNARIRSIRDDP-SRRCR-$NH_2$ (SEQ ID NO. 97)

98) GFNMQQQCRFYEALHDPNLNEEQRNARICSIRDDP-SRRCRRCRRC-$NH_2$ (SEQ ID NO. 98)

99) GFNMQQQK(Z)RFYEALHDPNLNEEQRNARIK(Z)SIRDDP-SRRK(Z)RRK(Z)RRK(Z)-$NH_2$ (SEQ ID NO. 99)

100) FNMQCQZRFYEALHDPNLNEEQRNARIRSIRDDC-$NH_2$ (SEQ ID NO. 100)
101) Acetyl-KNMQCQZRFYEALHDPNLNEEQRNARIRSIRDDC-$NH_2$ (SEQ ID NO. 101)

102) GFNMQCQK(Z)RFYEALHDPNLNEEQRNARIRSIRDDC-SRRK(Z)R-$NH_2$ (SEQ ID NO. 102)

103) FNMQCQK(Z)RFYEALHDPNLNEEQRNARIRSIRDDC-$NH_2$ (SEQ ID NO. 103)

104) Acetyl-KNMQCQK(Z)RFYEALHDPNLNEEQRNARIRSIRDDC-SRRK(Z)R-$NH_2$ (SEQ ID NO. 104)

105) GFNMQCQK(Z)RFYEALHDPNLNEEQRNARIRSIRDDC-SRRK(Z)RRK(Z)RRK(Z)-$NH_2$ (SEQ ID NO. 105)

106) Acetyl-KNMQCQK(Z)RFYEALHDPNLNEEQRNARIRSIRDDC-SRRK(Z)RRK(Z)RRK(Z)-$NH_2$ (SEQ ID NO. 106)

107) GFNMQCQK(Z)RFYEALHDPNLNEEQRNARIRSIRDDC-SRRK(Z)RRK(Z)RRK(Z)-$NH_2$ (SEQ ID NO. 107)

(iii) The following peptides
108) CAWHLGELVWC (SEQ ID NO. 108)
109) DCAWHLGELVWCT (SEQ ID NO. 109)
110) DCAWHLGELVFCT (SEQ ID NO. 110)

111) DCAWHLGELVX$^{15}$CT (SEQ ID NO. 111)
X$^{15}$ = 1-naphtoyl, 2-naphtoyl, benzyl, or benzothiophene
112) CDCAWHLGELVWCTC (SEQ ID NO. 112)
113) CAYHLGELVWC (SEQ ID NO. 113)
114) DCAYHLGELVWCTF (2-Pya) (SEQ ID NO. 114)
115) Acetyl-(Lys Azide )RRRGSGPDCAYHKGELVWCTFH-CONH$_2$ (SEQ ID NO. 115)

In consideration of the steric structures of IgG-BP and IgG, the amino acid sequence of IgG-BP may be the amino acid sequence shown in any one of SEQ ID NOs. 116 to 151, which lacks the N-terminal amino acid residue of the amino acid sequence shown in SEQ ID NOs. 72 to 107.

R$^3$ may be bound to a carbon of the benzene ring of Formula I or a linker via a Lys residue contained in IgG-BP, particularly the side chain of X$^5$ described above.

In addition, when R$^3$ is 79), 80), 81), 72-2), 88), 89), 90), 91), 92), 93), 101), 104), and 106) as described above, the side chain of any Lys residue may be bound to a carbon of the benzene ring of Formula I or a linker.

In the IgG-BP described above, any two cysteine residues may form an intramolecular (peptide) bond via a disulfide bond.

R$^4$ in Formula (1) is H or a substituted or unsubstituted alkyl group having 1 to 8 carbon atoms. R$^4$ is, for example, an alkyl group having 1 to 6, 1 to 4, or 1 to 3 carbon atoms, preferably an ethyl group, and more preferably a methyl group.

The compound can be synthesized by a known method. For example, when IgG-BP is synthesized on a protected peptide resin by a peptide solid phase synthesis method, a MeDbz residue is inserted using Fmoc-MeDbz(3- (9-Fluorenylmethoxycarbonyl)amino -4-(methylamino)benzoic acid) in the middle of extending the peptide, whereby the skeleton represented by the above Formula (I) can be constructed at the MeDbz residue site by 4-nitrophenyl chloroformate treatment on the protected peptide resin.

The compound according to the present embodiment or a salt thereof does not have an NHS group that is easily hydrolyzed, and therefore can be chemically stable and stored for a long period. Since the compound or a salt thereof has an active precursor represented by Formula I, a predetermined site of the Fc region can be specifically modified by being mixed with IgG. When IgG is modified with a reactive functional group, a drug, a labeling substance, or the like can be bound to IgG via the reactive functional group. When the drug is carried on IgG, an antibody drug conjugate in which the drug is bound site-specifically is obtained. Thereby, since the properties of the drug can be imparted to IgG, it can be applied to a drug transport system (DDS) and the like. In addition, since the labeling substance can be quantitatively controlled by modification of IgG with a site-specific labeling substance, it is useful for tracking, distribution, detection, and the like of IgG.

As an example of the above compounds, structures of compounds A to D are shown below. All of the compounds A to D have IgG-BP in which R$^3$ is the amino acid sequence shown in SEQ ID NO. 152. In the structures of the following compounds A to D, N, M, and Q in -C(=O)-N-M-Q-NH-, and QRRFYEALHDPNLNEEQRNARIRSIRDD represent one-letter amino acid code.

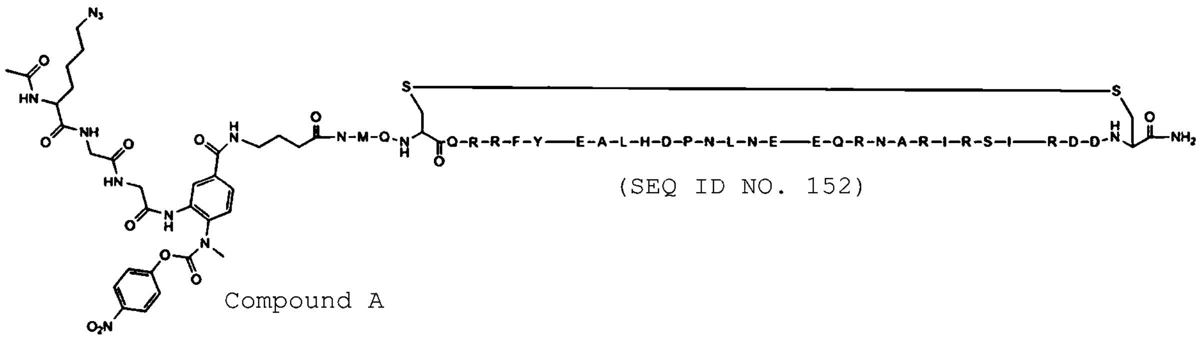

(SEQ ID NO. 152)

Compound A

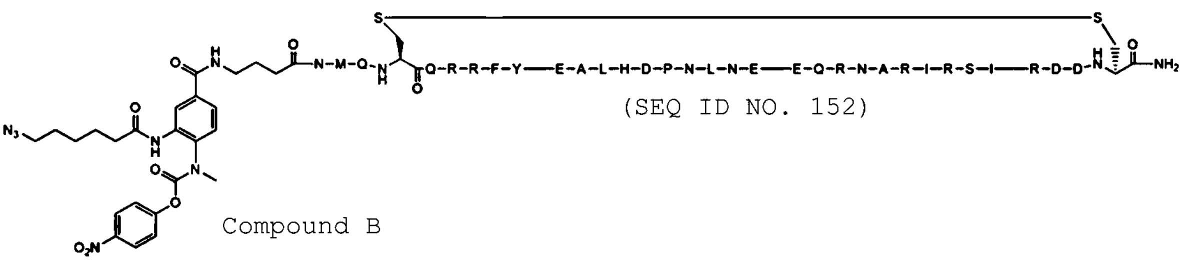

(SEQ ID NO. 152)

Compound B

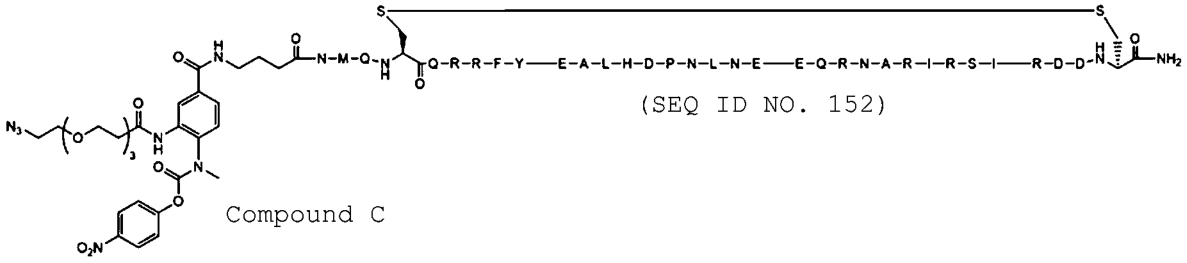

(SEQ ID NO. 152)

Compound C

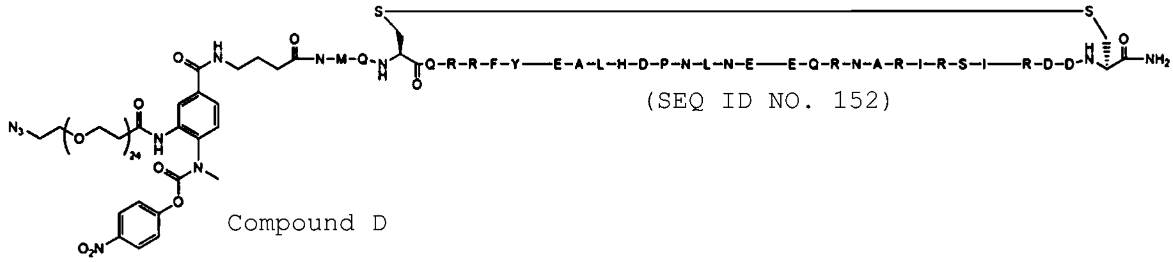

(SEQ ID NO. 152)

Compound D

The salt of the compound represented by the above Formula (1) is not particularly limited as long as the salt is a pharmacologically acceptable salt, and may be either an acidic salt or a basic salt. Examples of salts include alkali metal salts such as lithium salts, sodium salts, and potassium salts; alkaline earth metal salts such as magnesium salts and calcium salts; inorganic acid salts such as hydrochlorides, hydrobromides, sulfates, nitrates, oxalates, and phosphates; and organic acid salts such as acetates, propionates, hexanoates, cyclopentanepropionates, glycolates, pyruvates, lactates, malonates, succinates, malates, fumarates, tartrates, citrates, benzoates, o-(4-hydroxybenzoyl)benzoates, cinnamates, mandelates, methanesulfonates, ethanesulfonates, 1,2-ethanedisulfonates, 2-hydroxyethanesulfonates, benzenesulfonates, p-chlorobenzenesulfonates, 2-naphthalenesulfonates, p-toluenesulfonates, camphorsulfonates, glucoheptonates, 3-phenylpropionates, trimethylacetates, tert-butylacetates, lauryl sulfates, gluconates, glutamates, hydroxynaphthoates, salicylates, stearates, trifluoroacetates (TFA salts), maleates, and muconates.

In the conjugation step, a modified antibody represented by the following Formula (2) is obtained.

$(P1\text{-}S)_n\text{-}Ab...$ Formula (2)

In Formula (2), Ab is IgG. n is 1 or 2.

A reaction of modifying IgG with a compound in which P1 in the above Formula (1) is a labeling site that can be labeled with a radioisotope is illustrated in Fig. 1. Fig. 1 shows a modified antibody in which n is 1. Since the compound has an active precursor, the compound binds to IgG via an activated compound. By this reaction, a substituent including $R^2$ is added to a predetermined amino acid residue of IgG, and $R^3$ including IgG-BP is not added to IgG. Thereby, IgG can be easily modified with P1 in the above Formula (1).

In the reaction in the conjugation step, the compound may be exposed to IgG. As conditions of the reaction in the conjugation step, for example, the compound and IgG may be mixed in a buffer solution having a pH of 7.0 to 9.0. The concentrations of the compound and IgG in the buffer solution are appropriately adjusted. The molar ratio of the compound to the IgG in the buffer solution is, for example, 1:1 to 10, 1:2 to 8, or 1:3 to 6, preferably 1:6.

When P1 in the above Formula (1) is a labeling site that can be labeled with a radioisotope, the method for producing a radiolabeled antibody according to the present embodiment may further include a labeling step of labeling the labeling site with a radioisotope.

Preferably, P1 in the above Formula (1) is a labeling site having a click reactive group 1 (first click reactive group) and capable of being labeled with a radioisotope. In this case, the method for producing a radiolabeled antibody according to the present embodiment may further include a complex formation step of obtaining a labeling compound by reacting a compound represented by the following Formula (3) with a radioisotope to form a complex between the radioisotope and a chelating agent before the labeling step.

Ch-L1 Formula (3)

In Formula (3), Ch is a chelating agent. L1 is a linker having a click reactive group 2 (second click reactive group) that reacts with the click reactive group 1. Examples of the linker in L1 include -C-, -NH-, -O-, - S-, -C(=O)-NH-, -NH-C(=O)-, -C(=O)-O-, -O-C(=O)-, -C(=O)-, polyoxyalkylene groups, amino acid residues, peptide chains, PEG chains, and combinations thereof.

For example, the chelating agent has a structure derived from a compound represented by the following Formula V or a salt thereof.

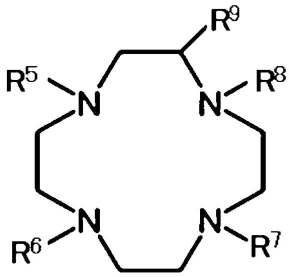

Formula V

In Formula V, $R^5$, $R^6$, and $R^7$ are each independently - $(CH_2)_pCOOH$, $-(CH_2)_pC_5H_4N$, $-(CH_2)_pPO_3H_2$, $-(CH_2)_pCONH_2$, or - $(CHCOOH)$ $(CH_2)_pCOOH$. One of $R^8$ and $R^9$ is a hydrogen atom, a carboxyl group, or a carboxyalkyl group having 2 or 3 carbon atoms, and the other is a substituent for binding to S in the above Formula (2). p is an integer of 0 or more and 3 or less. When $R^8$ is a substituent for binding to S in the above Formula (2), $R^9$ is a hydrogen atom. When $R^8$ is not a substituent for binding to S in the above Formula (2), $R^9$ is a substituent for binding to S in the above Formula (2). The structure derived from the compound represented by the above Formula V or a salt thereof is a structure in which at least one atom constituting Formula V is substituted with a linker represented by L1.

In the complex formation step, a complex of a radioisotope and a chelating agent can be formed by a known method. For example, an aqueous solution containing a compound represented by the above Formula (3) and sodium acetate, an aqueous solution containing gentisic acid and sodium acetate, and a hydrochloric acid solution containing a radioisotope may be mixed and reacted at 50 to 90°C, 60 to 80°C, and preferably 70°C, for 30 to 90 minutes, 40 to 80 minutes, and preferably 60 minutes. As a result, a labeling compound in which a chelating agent (ligand) is coordinated to a radioisotope is obtained.

When the method for producing a radiolabeled antibody includes a complex formation step, the method may further include a labeling step of labeling the modified antibody with a radioisotope by reacting the click reactive group 1 of the modified antibody obtained in the conjugation step with the click reactive group 2 of the labeling compound obtained in the complex formation step. The click reactive group 1 and the click reactive group 2 can be reacted by a known method.

As described above, since the compound represented by the above Formula (1) binds to IgG via the activated compound, IgG-BP is not added to IgG. Therefore, according to the modified antibody obtained by the method for producing a radiolabeled antibody according to the present embodiment, it is possible to avoid a decrease in blood retention caused by the remaining IgG-BP. Therefore, even when a bivalent modified antibody is contained, the modified antibody has sufficient blood retention. The modified antibody is useful as a radiopharmaceutical.

(Radiolabeled Antibody)

In another embodiment, a radiolabeled antibody is provided. That is, the radiolabeled antibody is represented by the following Formula (4).

(P2-S)n-Ab Formula (4)

In Formula (4), P2 is a complex site containing a radioisotope, and preferably, containing a complex formed by complex formation between a radioactive metal nuclide as the radioisotope and a chelating agent. S is a spacer.

In one embodiment, the spacer (S) contains Lys-Gly-Gly, and the amino group of the side chain of Lys in the spacer in the above Formula (4) is substituted with P2. The spacer in the above Formula (4) specifically binds to Lys of the Fc region of IgG.

In certain aspects, S in Formula (4) is selected from the group consisting of a polyethylene glycol (PEG) chain, an alkylene chain ($-CH_2-$), and combinations thereof. The polymerization degree (p) of the PEG chain is, for example, 2 to 30, 3 to 28, 3 to 27, 3 to 25, or 3 to 24. The number of carbon atoms q in the alkylene chain is, for example, 1 to 10, 1 to 8, 1 to 6, or 1 to 4. The spacer may contain a PEG chain and an alkylene chain, and for example, when the polymerization degree of (P2-S) is n, the above Formula (4) is represented by (P2-$(CH_2)$q-(PEG)p)n-Ab.

Ab is IgG. As the IgG, an antibody that specifically binds to any antigen can be used. Examples of the antigen include proteins, sugar chains, nucleic acids, and low molecular weight compounds. Preferably, the antigen is a protein. Examples of the protein include a cell membrane receptor, a cell membrane protein other than the cell membrane receptor, a ligand, and a soluble receptor, and more specifically, may be a target protein associated with a disease.

For example, an antibody that recognizes an antigen specifically expressed in a tumor cell can be used. Specific

examples of such antigens include PD-L1, GD2, PDGFR$\alpha$ (platelet-derived growth factor receptor), CD3, CD16A, CD19, CD20, CD22, CD25, CD27, CD30, CD32B, CD33, CD37, CD38, CD39, CD40, CD70, CD73, CD74, CD79b, CD100 (SEMA4D), CD105 (endoglin), CD123, CD137 (4-1BB), CD138, CD157 (Bst1), CD166, CD200, HER2, HER3, phosphatidylserine (PS), EpCAM, fibronectin, PD-1, VEGF, VEGFR-2, VEGF-A, HGF, gpNMB, DEC-205, folate receptor, Trop2, CEACAM5, S1P, IGF-1R, DLL4, TNT-1/B, CPAAs, PSMA, ICAM-1, MUC1, EGFR, EGFRvIII, KIR2DL1, KIR3DL2, NKG2A, tenascin-C, IGF (insulin-like growth factor), CTLA-4, mesothelin, c-Met, Ang2, ENPD3, folate receptor $\alpha$, TEM-1, GM2, glypican 3, macrophage inhibitory factor, Notch1, Notch2, Notch3, TLR-2, CSF-1R, FGFR2b, HLA-DR, GM-CSF, EphA3, B7-H3, gpA33, Frizzled7 receptor, DLL4, RSPO, LIV-1, SLITRK6, Nectin-4, MET, tissue factor, IL-8, P-cadherin, CEA, GITR, TAM (tumor associated macrophage), DLL4, Ang2, FGFR2, FGFR3, FGFR4, CXCR4, LAG-3, GITR, fucosyl GM1, IGF-1, angiopoietin 2, CSF-1R, OX40, BCMA, ErbB3, PTK7, EFNA4, FAP, DR5, CEA, Ly6E, CA6, CAIX, CA72-4 (TAG-72)CEACAM5, LAMP1, tissue factor, EPHA2, DR5, B7-H3, $\alpha$2-PI, A33, GDF15, ROR1, GITR, BCMA, TBA, LAG-3, EphA2, TIM-3, PIGF, Axl, MICA/B, Thomsen-Friedenreich, CLEC12A, Lgr3, transferrin receptor, TGF$\beta$, IL-17, 5T4, RTK, immune suppressor protein, NaPi2b, Lewis blood group B antigen, A34, lysyl oxidase, DLK-1, and $\alpha$9 integrin. As an example, the IgG is an anti-HER2 antibody (trastuzumab and pertuzumab) and an anti-EGFR antibody (panitumumab and cetuximab).

n is 1 or 2, preferably n is 2.

In certain aspects, the spacer (S) in Formula (4) is bound to, for example, Lys248 or Lys246, preferably Lys248, according to Eu numbering in human IgG Fc.

P2 in the above Formula (4) may contain a group represented by the following Formula (5).

ChM-L2 Formula (5)

In Formula (5), ChM is the above complex site, and L2 is a linker. The linker in the above Formula (5) may contain any one group represented by the following Formula VI-1, VI-2 or VI-3.

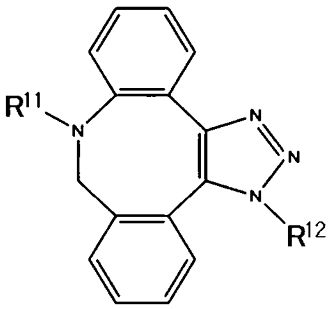

Formula VI-1

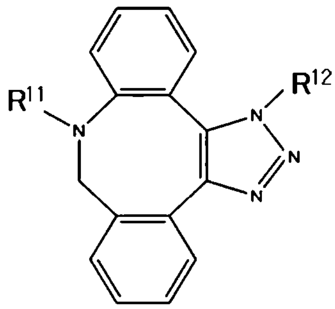

Formula VI-2

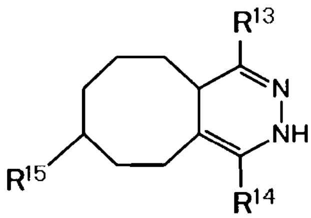

Formula VI-3

In Formulas VI-1 and VI-2, $R^{11}$ represents a binding site to the ChM side in the above Formula (5). $R^{12}$ represents a binding site to the S side in the above Formula (4). In Formula VI-3, one of $R^{13}$ and $R^{14}$ is a hydrogen atom, a methyl group, a phenyl group, or a pyridyl group, and the other represents a binding site to the ChM side in Formula (5). $R^{15}$ represents a binding site to the S side in the above Formula (4).

As an example, the radiolabeled antibody represented by Formula (4) is a radiolabeled antibody in which a radiolabeled linker represented by the following Formula (4-1) or (4-2) is bound to a lysine residue (for example, Lys248 or Lys246, preferably Lys248, according to Eu numbering in human IgG Fc) of human IgG Fc. Here, the radiolabeled linker represented by Formula (4-1) or Formula (4-2) is bound to a human IgG1 molecule by one molecule or two molecules, preferably two molecules. In this example, the human IgG is a human antibody represented by Ab of the radiolabeled antibody represented by Formula (4).

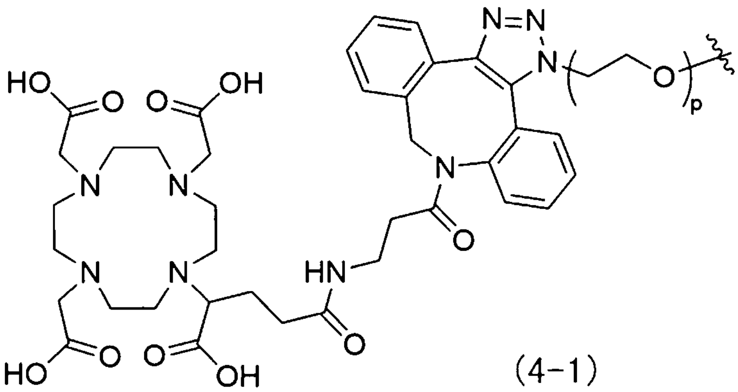

(4-1)

In Formula (4-1), p is, for example, 2 to 30, 3 to 28, 3 to 27, 3 to 25, or 3 to 24. The wavy line indicates the binding site with a lysine residue of human IgG Fc.

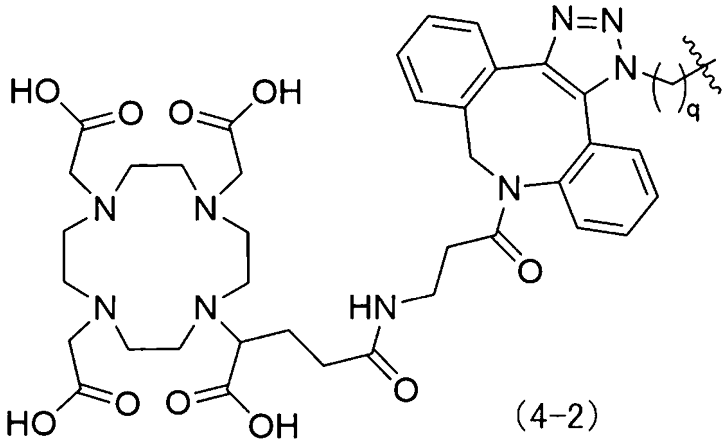

(4-2)

In Formula (4-2), q is, for example, 1 to 10, 1 to 8, 1 to 6, or 1 to 4. The wavy line indicates the binding site with a lysine residue of human IgG Fc. In the present specification, the human IgG or human antibody may be partially a human-derived antibody, and includes not only a fully human antibody but also a chimeric antibody and a humanized antibody.

When the radiolabeled antibody according to the present embodiment is used for the purpose of treating a disease, it is preferable to use an $\alpha$-ray-emitting nuclide or a $\beta^-$-ray-emitting nuclide as the radioisotope from the viewpoint of enhancing the therapeutic effect. The $\alpha$-ray-emitting nuclide may be any nuclide that emits $\alpha$ rays in the process of radioactive decay of the radioisotope, and is preferably a radioactive metal nuclide that emits $\alpha$ rays. Specifically, the radioactive metal nuclide that emits $\alpha$ rays is preferably $^{212}$Bi, $^{213}$Bi, $^{227}$Th, $^{225}$Ac, or the like, more preferably $^{227}$Th or $^{225}$Ac, and still more preferably $^{225}$Ac.

The $\beta^-$-ray-emitting nuclide may be a nuclide that emits a $\beta^-$-ray in the process of radioactive decay of a radioisotope, and is preferably a radioactive metal nuclide that emits a $\beta^-$-ray. Specifically, the radioactive metal nuclide that emits a $\beta^-$-ray is preferably $^{60}$Co, $^{59}$Fe, $^{64}$Cu, $^{67}$Cu, $^{89}$Sr, $^{90}$Y, $^{99m}$Tc, $^{103}$Ru, $^{153}$Sm, $^{165}$Dy, $^{166}$Ho, $^{177}$Lu, $^{186}$Re, $^{188}$Re, $^{198}$Au, $^{203}$Hg, $^{212}$Bi, $^{213}$Bi, $^{212}$Pb, or the like, more preferably $^{64}$Cu, $^{67}$Cu, $^{89}$Sr, $^{90}$Y, or $^{177}$Lu.

When the radiolabeled antibody according to the present embodiment is used for the purpose of diagnosis of a disease or detection of a lesion, it is preferable to use a $\beta^+$-ray-emitting nuclide, an electron-capturing decay nuclide, or a $\gamma$-ray-emitting nuclide as the radioisotope from the viewpoint of improving the diagnostic performance. The $\beta^+$-ray-emitting nuclide may be a nuclide that emits a positron in the process of radioactive decay of the radioisotope, and is preferably a metal nuclide that emits a positron, and $^{44}$Sc, $^{58}$Co, $^{68}$Ga, $^{64}$Cu, $^{89}$Zr, or the like is preferably used, and more preferably $^{64}$Cu or $^{89}$Zr. The electron-capturing decay nuclide may be any nuclide that emits Auger electrons or characteristic X-rays in the process of radioactive decay of the radioisotope, and a radioactive metal nuclide such as $^{51}$Cr, $^{57}$Co, $^{58}$Co, $^{67}$Ga, $^{68}$Ga, $^{64}$Cu, 89Zr, 111In, $^{186}$Re, $^{201}$Tl, or $^{197}$Hg is preferably used. The $\gamma$-ray-emitting nuclide may be a nuclide that emits $\gamma$ rays by $\gamma$ decay, and as the nuclide that emits $\gamma$ rays by $\gamma$ decay, a radioactive metal nuclide of $^{99m}$Tc, $^{68}$Ga, or $^{201}$Tl is preferably used.

When the radioactive metal nuclide coordinated to the radioactive metal complex in an ionic state is selected as the radioisotope based on the ionic radius, examples of the radioactive metal having an ionic radius of approximately 70 to 130 pm include $^{67}$Ga, $^{68}$Ga, $^{64}$Cu, $^{67}$Cu, 89Zr, 90y, $^{99m}$Tc, $^{103}$Ru, 111In, $^{153}$Sm, $^{165}$Dy, 166Ho, $^{177}$Lu, $^{186}$Re, $^{188}$Re, $^{198}$Au,

$^{201}Tl$, $^{197}Hg$, $^{203}Hg$, $^{212}Bi$, $^{213}Bi$, $^{212}Pb$, and $^{225}Ac$. These radioactive metal nuclides can form a complex of the chelating agent and the radioactive metal nuclide in the structure represented by the above Formula (4).

The above-described radioactive metal nuclide is preferably one or more selected from $Al^{18}F$, $^{64}Cu$, $^{67}Cu$, $^{68}Ga$, 89Zr, $^{90}Y$, 99mTc, 111In, 177Lu, and 225Ac.

(Radiopharmaceutical)

In another embodiment, a radiopharmaceutical is provided. The radiopharmaceutical contains the radiolabeled antibody as an active ingredient. The radiopharmaceutical can be formulated according to a conventional method.

The radiopharmaceutical may contain optional pharmacologically acceptable component in addition to the radiolabeled antibody. The optional components include, for example, carriers, excipients, lubricants, binders, disintegrants, solvents, solubilizing agents, suspending agents, isotonic agents, buffering agents, and anesthetizing agents. Additionally, if necessary, additives such as preservatives, antioxidants, colorants, and sweeteners may be blended with the radiolabeled antibody.

The administration route of the radiopharmaceutical is not particularly limited. The radiopharmaceutical may be administered, for example, parenterally or orally. In the case of parenteral administration, it may be intravenous injection, subcutaneous injection, intraperitoneal injection, intramuscular injection, transdermal administration, nasal administration, pulmonary administration, enteral administration, transmucosal administration, and the like. The radiopharmaceutical may be administered via drip infusion. The radiopharmaceutical can be in any formulation form.

The administration target of the radiopharmaceutical is preferably a vertebrate, and more preferably a mammalian animal. Examples of mammalian animals include, for instance, humans, chimpanzees and other primates, pigs, and horses, in addition to birds such as ducks and chickens. A particularly preferred administration target is a human. For example, the radiopharmaceutical is used for radionuclide therapy of cancer or diagnosis of cancer. When used in cancer, the radiolabeled antibody is preferably specifically bound to a target expressed in the cancer, particularly a marker molecule of the cancer. The cancer is not particularly limited, and examples thereof include breast cancer, brain tumor, prostate cancer, pancreatic cancer, gastric cancer, lung cancer, colon cancer, rectal cancer, colorectal cancer, small intestine cancer, esophageal cancer, duodenal cancer, tongue cancer, pharyngeal cancer, salivary gland cancer, neurilemmoma, liver cancer, kidney cancer, bile duct cancer, endometrial cancer, cervical cancer, ovarian cancer, bladder cancer, skin cancer, hemangioma, malignant lymphoma, malignant melanoma, thyroid cancer, parathyroid cancer, nasal cavity cancer, paranasal sinus cancer, bone tumor, angiofibroma, retinoblastoma, penile cancer, testicular tumor, pediatric solid cancer, sarcoma, and leukemia. These cancers may be primary or metastatic. The IgG may be appropriately selected according to the cancer type. For example, trastuzumab may be used for breast cancer, and cetuximab may be used for colorectal cancer.

The above embodiments include the following technical ideas.

1. A method for producing a radiolabeled antibody, comprising:

a conjugation step of reacting a compound represented by the following Formula (1) or a salt thereof, with IgG

P1-S-Y-B    Formula (1)

wherein
P1 is a labeling site labeled with a radioisotope or a labeling site capable of being labeled with a radioisotope,
S is a spacer,
Y is a reaction site that reacts with IgG, and
B is an antibody binding site containing an IgG-binding peptide that specifically binds to an Fc region of IgG, and in which
Y in the above Formula (1) has a group represented by the above Formula I.

2. The method for producing a radiolabeled antibody according to 1., in which
$R^1$ is a nitrophenoxy group.
3. The method for producing a radiolabeled antibody according to 1. or 2., in which

B in the above Formula (1) is
represented by any one of the above Formulas II, III, and IV, with the IgG-binding peptide being $R^{3a}$.

4. The method for producing a radiolabeled antibody according to any one of 1. to 3., in which

an amino acid sequence of the IgG-binding peptide is
an amino acid sequence shown in any one of SEQ ID NOs. 1 to 151.

5. The method for producing a radiolabeled antibody according to any one of 1. to 4., in which

S in the above Formula (1) includes Lys-Gly-Gly, and
an amino group of a side chain of Lys is substituted with P1 in the above Formula (1).

6. The method for producing a radiolabeled antibody according to any one of 1. to 4., in which S in the above Formula (1) is selected from the group consisting of a polyethylene glycol chain, an alkylene chain, and a combination thereof.
7. The method for producing a radiolabeled antibody according to any one of 1. to 6., in which

the radioisotope is
a radioactive halogen atom, $Al^{18}F$, $^{64}Cu$, $^{68}Ga$, 89Zr, $^{90}Y$, $^{99m}Tc$, 111In, $^{177}Lu$, or $^{225}Ac$.

8. The method for producing a radiolabeled antibody according to any one of 1. to 6., in which

the radioisotope is
a radioactive metal nuclide.

9. The method for producing a radiolabeled antibody according to any one of 1. to 8., in which

P1 in the above Formula (1) is a labeling site labeled with a radioisotope, and
in the conjugation step,
a modified antibody represented by the following Formula (2) is obtained,

$(P1\text{-}S)_n\text{-}Ab$ Formula (2)

wherein
Ab is IgG, and
n is 1 or 2.

10. The method for producing a radiolabeled antibody according to any one of 1. to 8., in which

P1 in the above Formula (1) is a labeling site capable of being labeled with a radioisotope, and
in the conjugation step,
a modified antibody represented by the following Formula (2) is obtained,

$(P1\text{-}S)_n\text{-}Ab$ Formula (2)

wherein
Ab is IgG, and
n is 1 or 2.

11. The method for producing a radiolabeled antibody according to 10., further comprising:
a labeling step of labeling the labeling site with the radioisotope.
12. The method for producing a radiolabeled antibody according to any one of 1. to 8., in which

P1 in the above Formula (1) is a labeling site having a first click reactive group and capable of being labeled with a radioisotope, and
in the conjugation step,
a modified antibody represented by the following Formula (2) is obtained,

$(P1\text{-}S)_n\text{-}Ab$ Formula (2)

wherein
Ab is IgG, and

n is 1 or 2.

13. The method for producing a radiolabeled antibody according to 12., further comprising:

a complex formation step of reacting a compound represented by the following Formula (3) with the radioisotope,

Ch-L1 Formula (3)

wherein
Ch is a chelating agent, and
L1 is a linker having a second click reactive group that reacts with the first click reactive group,
to form a complex of the radioisotope and the chelating agent, thereby obtaining a labeling compound.

14. The method for producing a radiolabeled antibody according to 13., further comprising:
a labeling step of labeling the modified antibody with the radioisotope by reacting the first click reactive group possessed by the modified antibody obtained in the conjugation step with the second click reactive group possessed by the labeling compound obtained in the complex formation step.
15. The method for producing a radiolabeled antibody according to 13. or 14., in which
the chelating agent has a structure derived from a compound represented by the above Formula V above or a salt thereof.
16. A radiolabeled antibody represented by Formula (4) below:

(P2-S)n-Ab Formula (4)

wherein

P2 contains a radioisotope,
S is a spacer,
Ab is IgG, and
n is 1 or 2, in which
S in the above Formula (4) includes Lys-Gly-Gly, an amino group of a side chain of Lys in S in the above Formula (4) is substituted with P2, and
S in the above Formula (4) is specifically bound to Lys in an Fc region of IgG.

17. A radiolabeled antibody represented by Formula (4) below:

(P2-S)n-Ab Formula (4)

wherein

P2 contains a radioisotope,
S is a spacer,
Ab is IgG, and
n is 1 or 2, in which
S in the above Formula (4) is selected from the group consisting of a polyethylene glycol chain, an alkylene chain, and a combination thereof, and
S in the above Formula (4) is specifically bound to Lys in an Fc region of IgG.

18. The radiolabeled antibody according to 16. or 17., in which
P2 is a complex site that contains a complex formed between a radioisotope and a chelating agent.
19. The radiolabeled antibody according to any one of 16. to 18., in which

the radioisotope is
a radioactive halogen atom, $Al^{18}F$, $^{64}Cu$, $^{68}Ga$, 89Zr, $^{90}Y$, $^{99m}Tc$, $^{111}In$, $^{177}Lu$, or $^{225}Ac$.

20. The radiolabeled antibody according to 18., in which
the chelating agent has a structure derived from a compound represented by the above Formula V above or a salt thereof.

21. The radiolabeled antibody according to any one of 16. to 20., in which in the above Formula (4), n is 2.
22. The radiolabeled antibody according to any one of 16. to 21., in which the IgG is trastuzumab or panitumumab.
23. The radiolabeled antibody according to 18., in which

P2 in the above Formula (4) includes a group represented by Formula (5) below:

ChM-L2 (5)

wherein ChM is the complex site, and L2 is a linker.

24. The radiolabeled antibody according to 23., in which

L2 in the above Formula (5) is a group represented by any one of Formulas VI-1, VI-2, or VI-3.

25. A radiopharmaceutical comprising, as an active ingredient, the radiolabeled antibody according to any one of 16. to 24..
26. The radiopharmaceutical according to 25., which is used for radionuclide therapy of cancer or for diagnosis of cancer.

The present invention will be described more specifically with reference to the following examples, but the present invention is not limited by the examples.

Examples

Example 1: Production of Radioactive Metal Nuclide Labeled Trastuzumab Conjugate

(Azide Group Linker Modification of Trastuzumab)

First, the above Compound A was synthesized as follows. Using 0.25 mmol of Rink Amide PEG resin (0.55 mmol/g), a protected peptide resin was constructed by repeating Fmoc deprotection with piperidine/1-methyl-2-pyrrolidone (1:4) and Fmoc-protected amino acids/DIC/Oxyma (1 mmol/1 mmol/1 mmol) on a PurePep Chorus peptide solid-phase synthesizer (manufactured by Gyros Protein Technologies). However, the coupling of Fmoc-MeDbz was performed using Fmoc-MeDbz/HCTU/6-Cl HOBt/DIEA (1 mmol/1 mmol/1 mmol/2 mmol), and the coupling of Fmoc-Lys($N_3$) was performed using Fmoc-Lys($N_3$)/DIC/HOAt (0.375 mmol/0.375 mmol/0.375 mmol).
The obtained protected peptide resin (0.25 mmol) was reacted with 4-nitrophenyl chloroformate (0.5 g) in dichloromethane for 1 hour to construct the PMD (phenoxycarbonylated N-methyl-o-diaminobenzene) ($NO_2$) framework. The obtained resin was treated with a TFA solution to deresinate and deprotect the resin, and solidified with diethyl ether to obtain 966 mg of a crude peptide (2SH form). The obtained crude peptide was purified with a reverse phase HPLC (high performance liquid chromatograph) column and freeze-dried to obtain 147 mg of a 2SH form. Thereafter, the crude peptide (2SH form) was dissolved in acetic acid/water (1:1), and under ice cooling, a 0.1 M iodine-methanol solution was slowly added for 1 equivalent. After 30 seconds, the reaction was quenched with an aqueous ascorbic acid solution, and purified and freeze-dried with a reverse phase HPLC column to obtain 87 mg of Azide KGG-PMD($NO_2$)-$\alpha$Z34 C(F1Gaba,K7R) (Compound A) as the desired SS form peptide.
Compound A was reacted with an antibody (human IgG1 antibody drug Herceptin, trastuzumab, manufactured by Roche). Trastuzumab is an antibody directed against the HER2 antigen. To 300 $\mu$L of a 20 mg/mL IgG solution, 600 $\mu$L of a 0.5 mol/L bicarbonate buffer solution (pH 8.9) was added, and 2075.7 $\mu$L of distilled water was added. Finally, 8.1 $\mu$L of 10 mmol/L of Compound A dissolved in dimethyl sulfoxide (hereinafter, DMSO) was added thereto, and the mixture was rapidly stirred and then reacted at room temperature for 20 minutes. 8.1 $\mu$L of 10 mmol/L of Compound A dissolved in DMSO was added, and the mixture was rapidly stirred. After 20 minutes, 8.1 $\mu$L of 10 mmol/L of Compound A was added again to make the total amount 3,000 $\mu$L (the IgG concentration in the final reaction product is 13.5 $\mu$M, and the reagent is 81 $\mu$mol/L in a 6-fold molar ratio). After the reaction for a total of 1 hour, the linker modification ratio was analyzed by LC-MS analysis. The reaction solution was gel-filtered using a PD-10 column (manufactured by Cytiva), then subjected to solvent exchange into PBS by centrifugal concentration, and concentrated to 5 mL.

(Solvent Exchange of Azide Group Linker Modified Trastuzumab)

The entire volume of the collected solution obtained above was added to an ultrafiltration filter (Amicon Ultra-15, manufactured by Merck) preloaded with 10 mL of trastuzumab formulation buffer solution (19 g/L trehalose hydrate, 470 mg/L L-histidine hydrochloride, 300 mg/L L-histidine), and centrifuged using a centrifuge (centrifugation conditions: 25°C, 3,000xg, 20 minutes). After centrifugation, the filtrate was discarded, 13.5 mL of trastuzumab formulation buffer solution was added again, and the mixture was centrifuged under the same conditions. This centrifugation was performed twice in total to obtain an azide group linker-modified trastuzumab solution (hereinafter also referred to as "Solution A") having an antibody concentration of 9.3 mg/mL.

(LC-MS Analysis of Reactant)

After quenching the reaction, the reaction solution was diluted 5-fold with 0.1% formic acid, and 20 µL was analyzed using a BioAccord LC-MS system (manufactured by Waters) equipped with a ProteinBEH C4 column (300 Å, 1.7 µm, 2.1 x 500 mm, manufactured by Waters) (flow rate: 0.4 mL/min, elution: linear gradient from 1% $CH_3CN$ containing 0.1% formic acid to 50% $CH_3CN$, column temperature: 80°C).

The results of LC-MS of the reaction solution after 2 hours are shown in Fig. 2. Fig. 2 is an elution chromatogram (280 nm) of the antibody solution. The peak at 1.92 minutes is a peak corresponding to the elution of Compound A. The peak at 2.25 minutes is a peak corresponding to the elution of IgG. Fig. 3A shows the result of mass spectrometry of a peak of IgG in an antibody solution before addition of Compound A. Fig. 3B shows the result of mass spectrometry of a peak of IgG in the reaction solution. After the linker modification, the molecular species 1 (148,221 Da) corresponding to the molecular weight of the antibody as a raw material decreased, and the molecular species of 148,532 Da and 148,843 Da increased. This difference of 311 Da and 622 Da was consistent with the addition of azidated KGG (312 Da) added by reaction of Compound A. From the result of the mass spectrum in Fig. 3B, it was confirmed that the content of the monovalent modified form (148,532 Da) was 25%, and the content of the bivalent modified form (148,843 Da) was 75%.

(Production of $^{89}$Zr-Labeled DOTAGA-DBCO)

$^{89}$Zr obtained by the nuclear reaction of $^{89}$Y(p,n)$^{89}$Zr and prepared into an aqueous solution of $^{89}Zr^{4+}$ ions (manufactured by Nihon Medi-Physics Co., Ltd.) was added to the vial, and the solvent was distilled off. 0.1 mol/L hydrochloric acid was added to the vial, and the bottom surface was thoroughly washed by pipetting to obtain 30 µL of 0.1 mol/L hydrochloric acid containing 101.3 MBq of $^{89}Zr^{4+}$ ions as radioactivity. To this $^{89}$Zr solution, 40 µL of an aqueous solution containing DOTAGA (1,4,7,10-tetrakis(carboxymethyl)-1,4,7,10-tetraazacyclododecane glutaric acid)-DBCO (concentration: 0.3 mmol/L) and sodium acetate (concentration: 0.156 mol/L), which contained 12 nmol of ligand, and 30 µL of an aqueous solution containing gentisic acid (concentration: 0.15 mol/L) and sodium acetate (concentration: 0.156 mol/L) were added. The mixture was reacted at 70°C for 60 minutes to obtain a solution containing DOTAGA-DBCO coordinated with $^{89}Zr^{4+}$ ($^{89}$Zr-labeled DOTAGA-DBCO) (hereinafter also referred to as "Solution B"). The structure of $^{89}$Zr-labeled DOTAGA-DBCO is shown below.

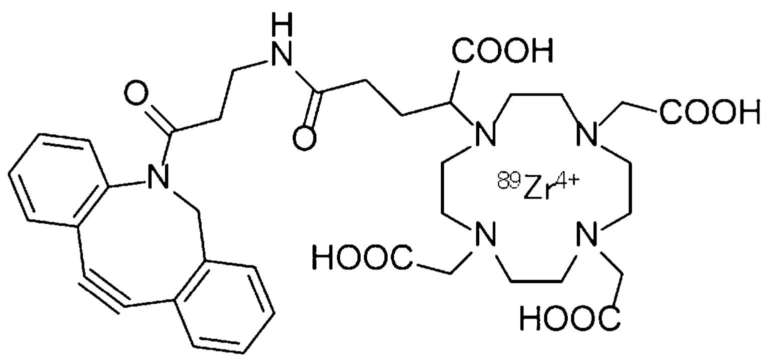

0.5 µL of the obtained reaction mixture of $^{89}$Zr-labeled DOTAGA-DBCO was extracted and separated by thin layer chromatography (TLC, iTLC SG, Agilent, developing solvent: mixed solution of water for injection and acetonitrile (volume ratio 1:1)). The developed thin layer chromatogram was introduced into a TLC analyzer (GITAStar, manufactured by MS Equipment Co., Ltd.) to measure the total $^{89}$Zr radioactivity count (including unreacted $^{89}$Zr) and the radioactivity count of $^{89}$Zr-labeled DOTAGA-DBCO in the reaction mixture, respectively. The percentage of radioactivity count of $^{89}$Zr-labeled DOTAGA-DBCO to the total $^{89}$Zr radioactivity count was 97.4%.

(Production of $^{89}$Zr-Trastuzumab Conjugate)

The $^{89}$Zr-trastuzumab conjugate was obtained by a production method including 2 steps of the $^{89}$Zr labeling step to the ligand and the click reaction of the $^{89}$Zr-ligand. That is, 72.4 μL (containing 4.5 nmol of the antibody) of Solution A was added to 30 μL (containing 4.5 nmol of $^{89}$Zr-labeled DOTAGA-DBCO) of Solution B and reacted at 37°C for 90 minutes, and the antibody and $^{89}$Zr-labeled DOTAGA-DBCO were bound by a click reaction.

The reaction rate of the obtained $^{89}$Zr-trastuzumab conjugate was calculated as follows. The total $^{89}$Zr radioactivity count (including unreacted $^{89}$Zr) and the radioactivity count of $^{89}$Zr-trastuzumab conjugate in the reaction mixture were separated by TLC (iTLC SG, manufactured by Agilent, developing solvent: mixed solution of aqueous EDTA aqueous solution (0.1 mol/L) and acetonitrile (volume ratio 1:1)). As a result of measuring the developed thin layer chromatogram with a TLC analyzer (GITAStar, manufactured by MS Equipment Co., Ltd.), the percentage of the radioactivity count of the $^{89}$Zr-trastuzumab conjugate to the total $^{89}$Zr radioactivity count was 95.3%.

(Purification of $^{89}$Zr-Trastuzumab Conjugate)

The entire volume of the $^{89}$Zr-labeled antibody obtained above was added to an ultrafiltration filter (Amicon Ultra-0.5, manufactured by Merck) preloaded with 0.4 mL of trastuzumab formulation buffer solution, and centrifuged using a centrifuge (centrifugation conditions: 4°C, 7,000xg, 10 minutes). After centrifugation, the filtrate was discarded, 0.4 mL of trastuzumab formulation buffer solution was added again, and the mixture was centrifuged under the same conditions.

Subsequently, by adding a trastuzumab formulation buffer solution to the solution obtained as described above, the radioactivity concentration of the $^{89}$Zr-trastuzumab conjugate was adjusted to 10 MBq/mL.

As a result of measuring the total $^{89}$Zr radioactivity count (including unreacted $^{89}$Zr) and the radioactivity count of the $^{89}$Zr-trastuzumab conjugate in the purified solution by the same TLC condition as described above, the radiochemical purity (RCP) of the $^{89}$Zr-trastuzumab conjugate (hereinafter also referred to as "Example 1") was 97.8%.

Comparative Example 1: Production of $^{89}$Zr-Trastuzumab Conjugate (Monovalent) by CCAP Method

(Synthesis of Peptide-Modified Trastuzumab)

IgG-BP containing 17 amino acid residues represented by the following Formula VII was prepared by the method described in International Patent Publication 2017/217347. The amino acid sequence of IgG-BP is an amino acid sequence (SEQ ID NO. 153) in which $X^5$ in SEQ ID NO. 2 is K. The side chain terminal amino group of the lysine residue of the IgG-BP is modified with a structure represented by $R^{16}$ of the following Formula VII. In addition, a disulfide bond is formed between two cysteine residues, and an azide group is bound to the N-terminal of the peptide via a linker structure having diglycolic acid and eight PEGs. The C-terminus of IgG-BP is amidated.

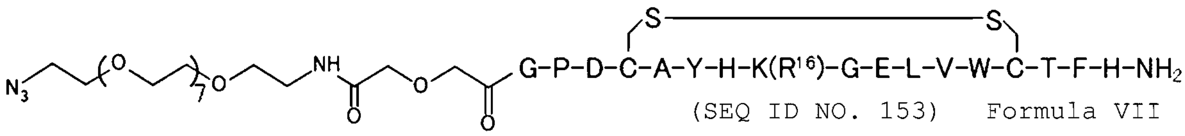

(SEQ ID NO. 153) Formula VII

$R^{16}$=

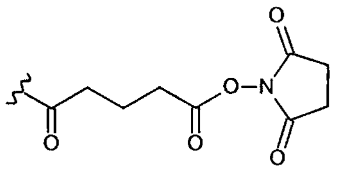

In Formula VII, GPDCAYHKGELVWCTFH (SEQ ID NO. 153) in -G-P-D-C-A-Y-H-K($R^{16}$)-G-E-L-V-W-C-T-F-H- represents a one-letter amino acid.

50 mg of trastuzumab was dissolved in 25 mL of 20 mmol/L acetic acid-sodium acetate buffer solution (pH 6.0) to obtain a 2 mg/mL antibody solution. Thereafter, 233 μL (1.7 equivalent to the antibody) of a DMSO solution (peptide concentration: 2.4 mmol/L) of the prepared IgG-BP was added, pipetting was immediately performed, and the mixture was allowed to stand at room temperature and reacted for 60 minutes to obtain a solution containing peptide-modified trastuzumab.

Next, the solution containing the peptide-modified trastuzumab obtained as described above was diluted with 25 mL of 20 mmol/L acetic acid-sodium acetate buffer solution (pH 6.0), and the diluted solution was passed through an IgG-BP column (a column obtained by immobilizing 5,632 nmol of the peptide having the amino acid sequence shown in SEQ

ID NO. 154 on a Hitrap NHS-activated HP column (manufactured by Cytiva) having a volume of 5 mL, in which the peptide-cysteine residues are crosslinked by a disulfide bond) to retain the peptide-modified trastuzumab. A 0.10 mol/L acetic acid-sodium acetate buffer solution containing 0.15 mol/L sodium chloride (pH 5.7) was passed through the column to remove the peptide-modified antibody modified with two molecules of peptide per molecule of trastuzumab (hereinafter referred to as "bivalent peptide-modified trastuzumab"), and then a 0.1 mol/L glycine hydrochloride buffer solution (pH 3.5) was passed through the column to collect the fraction containing peptide-modified trastuzumab modified with one molecule of peptide per molecule of trastuzumab(hereinafter referred to as "monovalent peptide-modified trastuzumab").

The entire volume of the collected solution obtained above was added to an ultrafiltration filter (Amicon Ultra-15, manufactured by Merck) preloaded with 10 mL of trastuzumab formulation buffer solution, and centrifuged using a centrifuge (centrifugation conditions: 25°C, 3,000xg, 20 minutes). After centrifugation, the filtrate was discarded, 13.5 mL of trastuzumab formulation buffer solution was added again, and the mixture was centrifuged under the same conditions. This centrifugation was performed twice in total.

Subsequently, by adding a trastuzumab formulation buffer solution to the solution obtained as described above, a monovalent peptide-modified trastuzumab solution (hereinafter also referred to as "Solution C") prepared such that the concentration of the monovalent peptide-modified trastuzumab was 15 mg/mL was obtained.

45 μL (containing 4.5 nmol of trastuzumab) of Solution C was added to 30 μL (containing 4.5 nmol of $^{89}$Zr-labeled DOTAGA-DBCO) of Solution B obtained in Example 1, and the mixture was reacted at 37°C for 90 minutes to bind the monovalent peptide-modified trastuzumab and $^{89}$Zr-labeled DOTAGA-DBCO by click reaction, thereby obtaining a $^{89}$Zr-trastuzumab conjugate (monovalent) by the CCAP method.

The reaction rate of the obtained $^{89}$Zr-trastuzumab conjugate (monovalent) was calculated as follows. As a result of evaluating the total $^{89}$Zr radioactivity count, including unreacted $^{89}$Zr and the radioactivity count of the $^{89}$Zr-trastuzumab conjugate (monovalent) in the reaction mixture of the $^{89}$Zr-trastuzumab conjugate (monovalent) in the same manner as in Example 1, the percentage of the radioactivity count of the $^{89}$Zr-trastuzumab conjugate to the total $^{89}$Zr radioactivity count was 86.2%.

(Purification of $^{89}$Zr-Trastuzumab Conjugate (Monovalent) by CCAP Method)

The entire volume of the $^{89}$Zr-trastuzumab conjugate (monovalent) obtained above was added to an ultrafiltration filter (Amicon Ultra-0.5, manufactured by Merck) preloaded with 0.4 mL of trastuzumab formulation buffer solution, and centrifuged using a centrifuge (centrifugation conditions: 4°C, 7,000xg, 10 minutes). After centrifugation, the filtrate was discarded, 0.4 mL of trastuzumab formulation buffer solution was added again, and the mixture was centrifuged under the same conditions.

Then, by adding a trastuzumab formulation buffer solution to the obtained solution, the radioactivity concentration of the $^{89}$Zr-trastuzumab conjugate (monovalent) was adjusted to 10 MBq/mL.

The RCP of the obtained $^{89}$Zr-trastuzumab conjugate (monovalent) was calculated as follows. As a result of measuring the total $^{89}$Zr radioactivity count (including unreacted $^{89}$Zr) and the radioactivity count of the $^{89}$Zr-trastuzumab conjugate (monovalent) in the purified solution by the TLC in the same manner as in Example 1, the RCP of the $^{89}$Zr-trastuzumab conjugate (monovalent) (hereinafter also referred to as "Comparative Example 1") was 98.7%.

Comparative Example 2: Production of $^{89}$Zr-Trastuzumab Conjugate (Bivalent) by CCAP Method

(Synthesis of Peptide-Modified Trastuzumab)

10 mg of trastuzumab was dissolved in 2.5 mL of 20 mmol/L acetic acid-sodium acetate buffer solution (pH 6.0) to obtain a 4 mg/mL antibody solution. Thereafter, 164 μL (6 equivalent to the antibody) of a DMSO solution (peptide concentration: 2.4 mmol/L) of the IgG-BP was added, and pipetting was immediately performed, and the mixture was allowed to stand at room temperature and reacted for 60 minutes to obtain a solution containing peptide-modified trastuzumab.

Next, the entire volume of the solution containing the peptide-modified trastuzumab was added in advance to an ultrafiltration filter (Amicon Ultra-15, manufactured by Merck) preloaded with 10 mL of trastuzumab formulation buffer solution, and centrifuged using a centrifuge (centrifugation conditions: 25°C, 3,000xg, 20 minutes). After centrifugation, the filtrate was discarded, 13.5 mL of trastuzumab formulation buffer solution was added again, and the mixture was centrifuged under the same conditions. This centrifugation was performed twice in total.

By adding a trastuzumab formulation buffer solution to the solution obtained as described above, a bivalent peptide-modified trastuzumab solution (hereinafter also referred to as "Solution D") prepared such that the concentration of the bivalent peptide-modified trastuzumab was 15 mg/mL was obtained.

69 μL (containing 4.5 nmol of antibody) of Solution D was added to 30 μL (containing 4.5 nmol of $^{89}$Zr-labeled DOTAGA-DBCO) of Solution B obtained in Example 1, and the mixture was reacted at 37°C for 90 minutes to bind the

bivalent peptide-modified trastuzumab and $^{89}Zr$-labeled DOTAGA-DBCO by click reaction, thereby obtaining a $^{89}Zr$-trastuzumab conjugate (bivalent) by the CCAP method.

The reaction rate of the obtained $^{89}Zr$-trastuzumab conjugate (bivalent) was calculated as follows. As a result of evaluating the total $^{89}Zr$ radioactivity count (including unreacted $^{89}Zr$) and the radioactivity count of the $^{89}Zr$-trastuzumab conjugate (bivalent) in the reaction mixture of the $^{89}Zr$-trastuzumab conjugate (bivalent) in the same manner as in Example 1, the percentage of the radioactivity count of the $^{89}Zr$-trastuzumab conjugate to the total $^{89}Zr$ radioactivity count was 95.4%.

(Purification of $^{89}Zr$-Trastuzumab Conjugate (Bivalent) by CCAP Method)

The entire volume of the $^{89}Zr$-trastuzumab conjugate (bivalent) obtained above was added to an ultrafiltration filter (Amicon Ultra-0.5, manufactured by Merck) preloaded with 0.4 mL of trastuzumab formulation buffer solution, and centrifuged using a centrifuge (centrifugation conditions: 4°C, 7,000xg, 10 minutes). After centrifugation, the filtrate was discarded, 0.4 mL of trastuzumab formulation buffer solution was added again, and the mixture was centrifuged under the same conditions.

By adding a trastuzumab formulation buffer solution to the solution obtained as described above, the radioactive concentration of the $^{89}Zr$-trastuzumab conjugate (bivalent) was adjusted to 10 MBq/mL.

The RCP of the $^{89}Zr$-trastuzumab conjugate (bivalent) was calculated as follows. As a result of measuring the total $^{89}Zr$ radioactivity count (including unreacted $^{89}Zr$) and the radioactivity count of the $^{89}Zr$-trastuzumab conjugate (bivalent) in the purified solution by the TLC in the same manner as in Example 1, the RCP of the $^{89}Zr$-trastuzumab conjugate (bivalent) (hereinafter also referred to as "Comparative Example 2") was 99.8%.

Test Example 1: Antigen Binding Activity

The antigen binding activity on the production date (day 0) and the last day of storage (day 3) was confirmed by in vitro autoradiography (ARG). SK-OV-3 cells, which are HER2-positive human ovarian cancer cell lines purchased from American Type Culture Collection (ATCC), and MDA-MB-231 cells, which are HER2-negative human breast cancer cell lines, were subcutaneously implanted to the flank of female BALB/c-nu/nu mice (manufactured by Charles River Laboratories Japan, Inc.) at 5 x $10^6$ cells and 1 x $10^7$ cells, respectively, to prepare tumor-bearing mice. Thereafter, the SK-OV-3 tumor and the MDA-MB-231 tumor were extracted and embedded in Tissue-Tek O.C.T. Compound (manufactured by Sakura Finetek Japan Co., Ltd.) to prepare a cryosection. Each of Example 1 and Comparative Example 1 was added to PBS containing 1% bovine serum albumin to be 5 kBq/mL, and the SK-OV-3 tumor section and the MDA-MB-231 tumor section were immersed. After bringing the sections into contact with an imaging plate, the plate was read using a scanner-type image analysis device (Typhoon FLA 7000, manufactured by GE Healthcare Japan), and the radioactivity bound to the sections was evaluated.

(Results)

Fig. 4A shows a corrected value (mean $\pm$ standard deviation) obtained by correcting a value obtained by dividing the count value in the ROI set in the used tumor section by the area of the ROI by a value obtained by dividing the count value of the standard radiation source by the area of the ROI. In Example 1 and Comparative Example 1, the binding activity for HER2 was confirmed. Example 1 and Comparative Example 1 bound only to SK-OV-3 tumor sections, and the HER2-selective binding activity was confirmed. Fig. 4B shows an average value of corrected values of SK-OV-3/an average value of corrected values of MDA-MB-231. In Example 1, there was no difference as compared with Comparative Example 1, and the binding ability to the positive section was maintained even after 3 days from production.

Test Example 2: In Vivo Distribution Evaluation

In Example 1, Comparative Example 1, and Comparative Example 2, biodistribution in each tissue in vivo was evaluated using mice. 6-week-old BALB/c mice (manufactured by the Jackson Laboratory Japan, Inc.) were intravenously administered via the tail vein with Example 1, Comparative Example 1, or Comparative Example 2 at a dose of 0.4 to 0.6 MBq per mouse (n = 5 per group). After 3, 24, 72, and 168 hours after administration of each of the radioconjugates, blood was collected from the heart under isoflurane anesthesia, and after euthanasia treatment, the heart, the lung, the spleen, the pancreas, the stomach, the small intestine, the large intestine, the ovary, the uterus, the thigh muscle, the femur, the liver, and the kidney were collected, and the other tissues were collected as the remaining whole body. Mice to which Example 1, Comparative Example 1, or Comparative Example 2 was administered were kept in a metabolic cage (manufactured by Shinano Machinery Co., Ltd.) until the dissection time, and feces and urine were collected. The weight of each collected tissue and blood was measured, and the counting rate of each tissue, blood and collected feces and urine

was measured using a γ-ray scintillation measuring apparatus (JDC-1712, manufactured by Hitachi, Ltd.). From the obtained tissue weight and counting rate, the administered radioactivity accumulation rate per unit weight of tissue (%ID/g) was calculated.

(Results)

Fig. 5 shows the accumulation rate of administered radioactivity per unit weight of blood (mean ± standard deviation). In Example 1, accumulation in blood was higher than that in Comparative Example 1 and Comparative Example 2. From this, it was shown that the blood retention of Example 1 was high. Figs. 6A, 6B, 6C, 6D, and 6E show the accumulation rate of administered radioactivity(mean ± standard deviation) per unit weight of the liver, the kidney, the femur, the thigh muscle and the spleen, respectively. In Example 1, it is considered that the accumulation in the whole body including the liver, the kidney, the femur, the thigh muscle, and the spleen was increased because the accumulation in the blood was high and the blood retention was increased. Therefore, in Example 1, it was shown that the retention in the whole body was improved.

Example 2: Production of Radioactive Metal Nuclide Labeled Trastuzumab Conjugate

(Synthesis of Azide Group Linker)

The above-described Compound B (6-azidohexanoic acid), Compound C (azido-PEG3-acid), and Compound D (azido-PEG24-acid) were synthesized as follows. Using 0.3 mmol of Compound B and Compound C, and 0.15 mmol of Compound D with Rink Amide PEG resin (0.5 mmol/g), a protected peptide resin was constructed by repeating Fmoc deprotection with piperidine/1-methyl-2-pyrrolidone (1:4) and amino acid coupling using Fmoc-protected amino acids/DIC/Oxyma (1:1:1) on an ABI 433A peptide solid-phase synthesizer (manufactured by Applied Biosystems). However, for Compound B, the coupling of 6-azidohexanoic acid was performed using 6-azidohexanoic acid/EDC/HOAt (0.9 mmol/0.9 mmol/0.9 mmol); for Compound C, the coupling of azido-PEG3-acid was performed using azido-PEG3-acid/EDC/HOAt (0.9 mmol/0.9 mmol/0.9 mmol); and for Compound D, the coupling of azido-PEG24-acid was performed using azido-PEG24-acid/EDC/HOAt (0.2 mmol/0.2 mmol/0.3 mmol).

The obtained protected peptide resin was reacted with 4-nitrophenyl chloroformate (2 g/mmol) in dichloromethane for 1 hour to construct the PMD($NO_2$) framework. The obtained resin was treated with a TFA solution to deresinate and deprotect the resin, and solidified with diethyl ether to obtain a crude peptide (2SH form). The obtained crude peptide was purified with a reverse phase HPLC column and freeze-dried to obtain a 2SH form. Thereafter, the crude peptide (2SH form) was dissolved in acetic acid/water (1:1), and under ice cooling, a 0.1 M iodine-methanol solution was slowly added for 1 equivalent. After 30 seconds, the reaction was quenched with an aqueous ascorbic acid solution, and purified and freeze-dried on a reverse phase HPLC column to obtain 69 mg of Azide-Hexanoyl-PMD($NO_2$)-αZ34C (Compound B), 198 mg of Azide-PEG3-PMD (NO2)-αZ34 C (Compound C), and 217 mg of Azide-PEG24-PMD($NO_2$)-αZ34C (Compound D) as the desired SS form peptide.

(Synthesis of High Purity Bivalent Azide Group Linker (Compound A) Modified Trastuzumab (Hereinafter also Referred to as "Tmab_N3"))

Compound A was reacted with an antibody (human IgG1 antibody drug Herceptin, trastuzumab, manufactured by Roche). To 2,310 μL of a 7.8 mg/mL IgG solution, 1,000 μL of a 0.5 mol/L bicarbonate buffer solution (pH 8.9) was added, and 1629.3 μL of distilled water was added. Further, 60.7 μL of 10 mmol/L of Compound A dissolved in DMSO was added to make the total amount 5,000 μL, and the mixture was rapidly stirred and then reacted at room temperature for 30 minutes. After dialysis, an additional 36.4 μL of 10 mmol/L of Compound A dissolved in DMSO was added to make the total amount 5,036 μL (IgG concentration in final reaction is 24.1 μM, and reagent is 192.8 μmol/L with 8 fold molar ratio). After the reaction for a total of 1 hour, the linker modification ratio was analyzed by LC-MS analysis. The reaction solution was gel-filtered using a PD-10 column (manufactured by Cytiva), then subjected to solvent exchange into PBS by centrifugal concentration, and concentrated to 5 mL.

(Synthesis of High Purity Bivalent Azide Group Linker (Compound B) Modified Trastuzumab (Hereinafter also Referred to as "Tmab_6N3"))

Compound B and trastuzumab were reacted in the same manner as in Tmab_N3 except that no additional reaction after dialysis was performed (IgG concentration in final reaction is 24.3 μM, and reagent is 121.4 μmol/L with 5 fold molar ratio).

(Synthesis of High Purity Bivalent Azide Group Linker (Compound C) Modified Trastuzumab (Hereinafter also Referred to as "Tmab_11N3"))

Compound C was reacted with trastuzumab. After dialysis, a reaction was performed in the same manner as in Tmab_N3, except that 60.7 μL of 10 mmol/L of Compound C dissolved in DMSO was added to make total amount 5,060.7 μL (IgG concentration in final reaction is 24.0 μM, reagent is 239.9 μmol/L with 10 fold molar ratio).

(Synthesis of High Purity Bivalent Azide Group Linker (Compound D) Modified Trastuzumab (Hereinafter also Referred to as "Tmab_75N3"))

Compound D was reacted with trastuzumab. To 1,350 μL of a 7.8 mg/mL IgG solution, 600 μL of a 0.5 mol/L bicarbonate buffer solution (pH 8.9) was added, and 1,014 μL of distilled water was added. Further, 36 μL of 10 mmol/L of Compound D dissolved in DMSO was added to make the total amount 3,000 μL, and the mixture was rapidly stirred and then reacted at room temperature for 30 minutes. After dialysis, an additional 21 μL of 10 mmol/L of Compound D dissolved in DMSO was added to make the total amount 3,021 μL (IgG concentration in final reaction is 23.6 μM, and reagent is 188.7 μmol/L with 8 fold molar ratio).

(Solvent Exchange of High Purity Bivalent Azide Group Linker Modified Trastuzumab)

By performing solvent exchange into the trastuzumab formulation buffer solution in the same manner as in Example 1, Tmab_N3 solution (hereinafter also referred to as "Solution E"), Tmab_6N3 solution (hereinafter also referred to as "Solution F"), Tmab_11N3 solution (hereinafter also referred to as "Solution G"), and Tmab_75N3 solution (hereinafter also referred to as "Solution H"), each having an antibody concentration of 15 mg/mL, were obtained.

(LC-MS Analysis of Reactant)

The reaction solution, after quenching the reaction, was analyzed by LC-MS in the same manner as described in Example 1.

Fig. 7A shows the result of mass spectrometry of a peak of IgG in an antibody solution before addition of Compound A. Fig. 7B shows the result of mass spectrometry of a peak of IgG in the reaction solution of Tmab_N3. After the linker modification, the molecular species (148,220 Da) corresponding to the molecular weight of the antibody as a raw material decreased, and the molecular species of 148,532 Da and 148,840 Da increased. This difference of 312 Da and 620 Da was consistent with the addition of one and two molecules, respectively, of azide-modified KGG (312 Da) added by the reaction with Compound A. Based on the result of the mass spectrum in Fig. 7B, it was confirmed that the content of the monovalent modified form (148,532 Da) was 13%, and the content of the bivalent modified form (148,840 Da) was 87%.

Fig. 8A shows the result of mass spectrometry of a peak of IgG in an antibody solution before addition of Compound B. Fig. 8B shows the result of mass spectrometry of a peak of IgG in the reaction solution of Tmab_6N3. After the linker modification, the molecular species (148,220 Da) corresponding to the molecular weight of the antibody as a raw material decreased, and the molecular species of 148,498 Da increased. This difference of 278 Da was consistent with the addition of azidated hexanoyl (141 Da) 2 molecules added by reaction of Compound B. Based on the result of the mass spectrum in Fig. 8B, it was confirmed that the content of the bivalent modified form (148,498 Da) was 100%.

Fig. 9A shows the result of mass spectrometry of a peak of IgG in an antibody solution before addition of Compound C. Fig. 9B shows the result of mass spectrometry of a peak of IgG in the reaction solution of Tmab_11N3. After the linker modification, the molecular species (148,220 Da) corresponding to the molecular weight of the antibody as a raw material decreased, and the molecular species of 148,650 Da increased. This difference of 430 Da was consistent with the addition of azidated PEG3 (217 Da) 2 molecules added by reaction of Compound C. Based on the result of the mass spectrum in Fig. 9B, it was confirmed that the content of the monovalent modified form (148,435 Da) was 8%, and the content of the bivalent modified form (148,650 Da) was 92%.

Fig. 10A shows the result of mass spectrometry of a peak of IgG in an antibody solution before addition of Compound D. Fig. 10B shows the result of mass spectrometry of a peak of IgG in the reaction solution of Tmab_75N3. After the linker modification, the molecular species (148,220 Da) corresponding to the molecular weight of the antibody as a raw material decreased, and the molecular species of 150,532 Da increased. This difference of 2,312 Da was consistent with the addition of azidated PEG24 (1,156 Da) 2 molecules added by reaction of Compound D. Based on the result of the mass spectrum in Fig. 10B, it was confirmed that the content of the bivalent modified form (150,532 Da) was 100%.

(Production of $^{89}$Zr-Labeled DOTAGA-DBCO)

$^{89}$Zr obtained by the nuclear reaction of $^{89}$Y(p,n)$^{89}$Zr and prepared into an aqueous solution of $^{89}Zr^{4+}$ ions

(manufactured by Nihon Medi-Physics Co., Ltd.) was added to the vial, and the solvent was distilled off. 200 μL of an aqueous solution containing DOTAGA-DBCO (concentration: 0.3 mmol/L) and sodium acetate (concentration: 0.1 mol/L) (containing 60 nmol of the ligand) and 300 μL of an aqueous solution containing gentisitic acid (concentration: 0.075 mol/L) and sodium acetate (concentration: 0.1 mol/L) were added to 387 MBq of $^{89}Zr^{4+}$ ions as radioactivity of the vial, and the mixture was reacted at 70°C for 60 minutes to obtain a solution containing $^{89}$Zr-labeled DOTAGA-DBCO (hereinafter also referred to as "solution I"). The RCP evaluated by the same TLC as in Example 1 was 99.8%.

(Production of $^{89}$Zr-Trastuzumab Conjugate)

A $^{89}$Zr-trastuzumab conjugate was obtained by the same production method as in Example 1. That is, 145 μL (containing 14.4 nmol of the antibody) of each of Solutions E, F, G, and H was added to 100 μL (containing 12 nmol of $^{89}$Zr-labeled DOTAGA-DBCO) of Solution I and reacted at 37°C for 90 minutes, and the antibody and $^{89}$Zr-labeled DOTAGA-DBCO were bound via a click reaction. From Solutions E, F, G, and H, $^{89}$Zr-Tmab_N3, $^{89}$Zr-Tmab_6N3, $^{89}$Zr-Tmab_11N3, and $^{89}$Zr-Tmab_7SN3 were obtained, respectively, as $^{89}$Zr-trastuzumab conjugates.

In $^{89}$Zr-Tmab_N3, which is a radiolabeled antibody represented by Formula (4), S is Lys-Gly-Gly, the amino group on the side chain of the Lys in S of Formula (4) is substituted with P2, P2 is a group represented by Formula (5), L2 is a group represented by Formula VI-1, Ch is a group represented by Formula V, wherein $R^5$, $R^6$, $R^7$, and $R^8$ are each independently $-CH_2COOH$, $R^9$ is a substituent for binding to Formula VI-1, M is 89Zr, n is 2, and Ab is trastuzumab.

$^{89}$Zr-Tmab_6N3 is a radiolabeled linker represented by Formula (4-2) in which $^{89}$Zr forms a complex, in which q is 5, to which 2 molecules of trastuzumab are bound.

$^{89}$Zr-Tmab 11N3 is a radiolabeled linker represented by Formula (4-1) in which $^{89}$Zr forms a complex, in which p is 3, to which 2 molecules of trastuzumab are bound.

$^{89}$Zr-Tmab_75N3 is a radiolabeled linker represented by Formula (4-1) in which $^{89}$Zr forms a complex, in which p is 24, to which 2 molecules of trastuzumab are bound.

The reaction rate of the obtained $^{89}$Zr-trastuzumab conjugate was calculated in the same manner as in Example 1. The percentage of the radioactivity count of the $^{89}$Zr-trastuzumab conjugate to the total $^{89}$Zr radioactivity count was 94.8% for $^{89}$Zr-Tmab_N3, 95.8% for $^{89}$Zr-Tmab_6N3, 94.5% for $^{89}$Zr-Tmab_11N3, and 82.8% for $^{89}$Zr-Tmab_75N3.

(Purification of $^{89}$Zr-Trastuzumab Conjugate)

Except for changing the final radioactivity concentration of the $^{89}$Zr-trastuzumab conjugate to 50 MBq/mL, purification was performed in the same manner as in Example 1. As measured by TLC, the RCP was 99.2% for $^{89}$Zr-Tmab_N3, 99.7% for $^{89}$Zr-Tmab_6N3, 99.5% for $^{89}$Zr-Tmab_11N3, and 99.1% for $^{89}$Zr-Tmab_75N3.

Example 3: Production of Radioactive Metal Nuclide Labeled Panitumumab Conjugate

(Synthesis of High Purity Bivalent Azide Group Linker (Compound A) Modified Panitumumab (Hereinafter also Referred to as "Pmab_N3"))

Compound A was reacted with an antibody (human IgG2 antibody drug Vectibix, panitumumab, manufactured by Amgen). To 650 μL of a 20 mg/mL IgG solution, 600 μL of a 0.5 mol/L bicarbonate buffer solution (pH 8.9) was added, and 1705.8 μL of distilled water was added. Finally, 44.2 μL of 10 mmol/L of Compound A dissolved in DMSO was added to make the total amount 3,000 μL, and the mixture was rapidly stirred and then reacted at room temperature for 30 minutes. After dialysis, an additional 26.5 μL of 10 mmol/L of Compound A dissolved in DMSO was added to make the total amount 5,026.5 μL (IgG concentration in final reaction is 29.2 μM, and reagent is 233.6 μmol/L with 8 fold molar ratio). After the reaction for a total of 1 hour, the linker modification ratio was analyzed by LC-MS analysis. The reaction solution was gel-filtered using a PD-10 column (manufactured by Cytiva), then subjected to solvent exchange into PBS by centrifugal concentration, and concentrated to 5 mL.

(Synthesis of High Purity Bivalent Azide Group Linker (Compound B) Modified Panitumumab (Hereinafter also Referred to as "Pmab_6N3"))

Compound B and panitumumab were reacted in the same manner as in Pmab_N3 except that no additional reaction after dialysis was performed (IgG concentration in final reaction is 29.5 μM, and reagent is 147.3 μmol/L with 5 fold molar ratio).

(Synthesis of High Purity Bivalent Azide Group Linker (Compound C) Modified Panitumumab (Hereinafter also Referred to as "Pmab_11N3"))

Compound C and panitumumab were reacted in the same manner as in Pmab_N3 (IgG concentration in the final reaction product is 29.2μM, and the reagent is 233.6 μmol ol/L with 8 fold molar ratio.).

(Solvent Exchange of High Purity Bivalent Azide Group Linker Modified Panitumumab)

By performing solvent exchange in the same manner as in Example 1, except that the formulation buffer solution was replaced with an aqueous solution of 50 mmol/L sodium acetate containing 0.1 mol/L sodium chloride, Pmab_N3 solution (hereinafter also referred to as "Solution J"), Pmab_6N3 solution (hereinafter also referred to as "Solution K"), and Pmab_11N3 solution (hereinafter also referred to as "Solution L"), each having an antibody concentration of 15 mg/mL, were obtained.

(LC-MS Analysis of Reactant)

The reaction solution, after quenching the reaction, was analyzed by LC-MS in the same manner as described in Example 1.

Fig. 11A shows the result of mass spectrometry of a peak of IgG in an antibody solution before addition of Compound A. Fig. 11B shows the result of mass spectrometry of a peak of IgG in the reaction solution of Pmab_N3. After the linker modification, the molecular species (147,103 Da) corresponding to the molecular weight of the antibody as a raw material decreased, and the molecular species of 147,710 Da increased. This difference of 607 Da was consistent with the addition of azidated KGG (312 Da) 2 molecules added by reaction of Compound A. Based on the result of the mass spectrum in Fig. 11B, it was confirmed that the content of the bivalent modified form was 100%.

Fig. 12A shows the result of mass spectrometry of a peak of IgG in an antibody solution before addition of Compound B. Fig. 12B shows the result of mass spectrometry of a peak of IgG in the reaction solution of Pmab_6N3. After the linker modification, the molecular species (147,103 Da) corresponding to the molecular weight of the antibody as a raw material decreased, and the molecular species of 147,373 Da increased. This difference of 270 Da was consistent with the addition of azidated hexanoyl (141 Da) 2 molecules added by reaction of Compound B. Based on the result of the mass spectrum in Fig. 12B, it was confirmed that the content of the bivalent modified form was 100%.

Fig. 13A shows the result of mass spectrometry of a peak of IgG in an antibody solution before addition of Compound C. Fig. 13B shows the result of mass spectrometry of a peak of IgG in the reaction solution of Pmab 11N3. After the linker modification, the molecular species (147,103 Da) corresponding to the molecular weight of the antibody as a raw material decreased, and the molecular species of 147,320 Da and 147,522 Da increased. This difference of 217 Da and 419 Da corresponded to the addition of one and two molecules, respectively, of azide-modified PEG3 (217 Da) added by the reaction with Compound C. From the result of the mass spectrum in Fig. 13B, it was confirmed that the content of the monovalent modified form (147,320 Da) was 1%, and the content of the bivalent modified form (147,522 Da) was 99%.

(Production of $^{89}$Zr-Labeled DOTAGA-DBCO)

$^{89}$Zr obtained by the nuclear reaction of $^{89}$Y(p,n)$^{89}$Zr and prepared into an aqueous solution of $^{89}Zr^{4+}$ ions (manufactured by Nihon Medi-Physics Co., Ltd.) was added to the vial, and the solvent was distilled off. 120 μL of an aqueous solution containing 150 μL of 0.1 mol/L hydrochloric acid, DOTAGA-DBCO (concentration: 0.3 mmol/L), and sodium acetate (concentration: 0.156 mol/L) (containing 36 nmol of the ligand) and 150 μL of an aqueous solution containing gentisitic acid (concentration: 0.15 mol/L) and sodium acetate (concentration: 0.156 mol/L) were added to 452 MBq of $^{89}Zr^{4+}$ ions as radioactivity of the vial, and the mixture was reacted at 70°C for 60 minutes to obtain a solution containing $^{89}$Zr-labeled DOTAGA-DBCO (hereinafter also referred to as "solution M"). The RCP evaluated by the same TLC as in Example 1 was 93.9%.

(Production of $^{89}$Zr-Panitumumab Conjugate)

A $^{89}$Zr-panitumumab conjugate was obtained by the same production method as in Example 1. That is, 110 μL (containing 10.9 nmol of the antibody) of each of Solutions J, K, and L was added to 100 μL (containing 9 nmol of $^{89}$Zr-labeled DOTAGA-DBCO) of Solution M and reacted at 37°C for 90 minutes, and the antibody and $^{89}$Zr-labeled DOTAGA-DBCO were bound via a click reaction. From the solutions J, K, and L, $^{89}$Zr-Pmab_N3, $^{89}$Zr-Pmab_6N3, and $^{89}$Zr-Pmab_11N3 were obtained as $^{89}$Zr-panitumumab conjugates, respectively.

In $^{89}$Zr-Pmab_N3, which is a radiolabeled antibody represented by Formula (4), S is Lys-Gly-Gly, the amino group on the side chain of the Lys in S of Formula (4) is substituted with P2, P2 is a group represented by Formula (5), L2 is a

group represented by Formula VI-1, Ch is a group represented by Formula V, wherein $R^5$, $R^6$, $R^7$, and $R^8$ are each independently $-CH_2COOH$, $R^9$ is a substituent for binding to Formula VI-1, M is 89Zr, n is 2, and Ab is panitumumab.

$^{89}$Zr-Pmab_6N3 is a radiolabeled linker represented by Formula (4-2) in which $^{89}$Zr forms a complex, in which q is 5, to which 2 molecules of panitumumab are bound.

$^{889}$Zr-Pmab_11N3 is a radiolabeled linker represented by Formula (4-1) in which $^{89}$Zr forms a complex, in which p is 3, to which 2 molecules of panitumumab are bound.

The reaction rate of the obtained $^{89}$Zr-panitumumab conjugate was calculated in the same manner as in Example 1. The percentage of the radioactivity count of the $^{89}$Zr-panitumumab conjugate to the total $^{89}$Zr radioactivity count was 93.3% for $^{89}$Zr-Pmab_N3, 91.8% for $^{89}$Zr-Pmab_6N3, and 93.5% for $^{89}$Zr-Pmab11N3.

(Purification of $^{89}$Zr-Panitumumab Conjugate)

Except for changing the final radioactivity concentration of the $^{89}$Zr-panitumumab conjugate to 50 MBq/mL and changing the buffer solution to an aqueous solution of 50 mmol/L sodium acetate containing 0.1 mol/L sodium chloride, purification was performed in the same manner as in Example 1. As measured by TLC, the RCP was 98.5% for $^{89}$Zr-Pmab_N3, 98.6% for $^{89}$Zr-Pmab_6N3, and 98.2% for $^{89}$Zr-Pmab11N3.

Comparative Example 3: Production of $^{89}$Zr-Panitumumab Conjugate (Monovalent) by CCAP Method

(Synthesis of Peptide-Modified Panitumumab)

50 mg of panitumumab was diluted with 20 mmol/L acetic acid-sodium acetate buffer solution (pH 6.0) to obtain a 4 mg/mL antibody solution. Thereafter, similar to Comparative Example 1, 27.4 μL (1.7 equivalent to the antibody) of a DMSO solution (peptide concentration: 16.5 mmol/L) of the IgG-BP was added, pipetting was immediately performed, and the mixture was allowed to stand at room temperature and reacted for 60 minutes to obtain a solution containing peptide-modified panitumumab.

Next, in the solution containing the peptide-modified panitumumab obtained as described above, a fraction containing peptide-modified panitumumab (hereinafter referred to as "monovalent peptide-modified panitumumab") modified with one molecule of peptide per one molecule of panitumumab was collected using an IgG-BP column in the same manner as in Comparative Example 1.

Next, the entire volume of the solution containing the monovalent peptide-modified panitumumab was added in advance to an ultrafiltration filter (Amicon Ultra-15, manufactured by Merck) preloaded with 10 mL of an aqueous solution of 50 mmol/L sodium acetate containing 0.1 mol/L sodium chloride, and centrifuged using a centrifuge (centrifugation conditions: 25°C, 3,000xg, 20 minutes). After centrifugation, the filtrate was discarded, 13.5 mL of a 50 mmol/L sodium acetate aqueous solution containing 0.1 mol/L sodium chloride was added again, and the mixture was centrifuged under the same conditions. This centrifugation was performed twice in total.

By adding an aqueous solution of 50 mmol/L sodium acetate containing 0.1 mol/L sodium chloride to the solution obtained as described above, a monovalent peptide-modified panitumumab solution with a concentration of monovalent peptide-modified panitumumab adjusted to 12 mg/mL (hereinafter also referred to as "Solution N") was obtained.

138 μL (containing 10.9 nmol of antibody) of Solution N was added to 100 μL (containing 9 nmol of $^{89}$Zr-labeled DOTAGA-DBCO) of Solution M obtained in Example 3, and the mixture was reacted at 37°C for 90 minutes to bind the monovalent peptide-modified panitumumab and $^{89}$Zr-labeled DOTAGA-DBCO via click reaction, thereby obtaining a $^{89}$Zr-panitumumab conjugate (monovalent) by the CCAP method.

As a result of calculating the reaction rate of the obtained $^{89}$Zr-panitumumab conjugate (monovalent) in the same manner as in Example 1, the percentage of the radioactivity count of the $^{89}$Zr-panitumumab conjugate to the total $^{89}$Zr radioactivity count was 82.6%.

(Purification of $^{89}$Zr-Panitumumab Conjugate (Monovalent) by CCAP Method)

Except for changing the final radioactive concentration of the $^{89}$Zr-panitumumab conjugate to 50 MBq/mL and changing the buffer solution to an aqueous solution of 50 mmol/L sodium acetate containing 0.1 mol/L sodium chloride, purification was performed in the same manner as in Example 1. As measured by TLC, the RCP was 98.3% for $^{89}$Zr-panitumumab conjugate (monovalent) (hereinafter, referred to as "Comparative Example 3").

Test Example 3: Stability Evaluation

Each of the radioconjugates ($^{89}$Zr-Tmab_N3, $^{89}Z_r$-Tmab_6N3, $^{89}$Zr-Tmab_11N3, $^{89}$Zr-Tmab_75N3, $^{89}$Zr-Pmab_N3, $^{89}$Zr-Pmab_6N3, and $^{89}$Zr-Pmab_11N3) produced according to the description of Examples 2 and 3 was

stored in a refrigerator (5°C) for 2 weeks, and the ratio of RCP and aggregates was evaluated at each time point (Day 0, Day 7, and Day 14). The period of 14 days from the end of production corresponds to approximately 5 half-life when the radioactive metal nuclide is $^{89}$Zr.

Test Example 3-1: RCP

RCP was analyzed by TLC. The conditions for TLC were the same as those used for examining the reaction rate in Example 1.

(Results)

The RCP of each radioconjugate is shown in Table 1.

Table 1

| | RCP (%) | | |
|---|---|---|---|
| | Day 0 | Day 7 | Day 14 |
| $^{89}$ Zr - Tmab_N3 | 99.2 | 99.1 | 99.1 |
| $^{89}$ Zr -Tmab_6N3 | 99.7 | 98.8 | 99.4 |
| $^{89}$Z r - Tmab_11N3 | 99.5 | 99.1 | 99.1 |
| $^{89}$Zr - Tmab_75N3 | 99.1 | 98.9 | 99.2 |
| $^{89}$ Zr - Pmab_N3 | 98.5 | 99.1 | 95.6 |
| $^{89}$Zr - Pmab_6N3 | 98.6 | 99.4 | 96.7 |
| $^{89}$Zr - Pmab_11N3 | 98.2 | 98.6 | 96.1 |

When the $^{89}$Zr-labeled antibody according to the present embodiment was stored for 7 days under refrigeration after the end of production, the RCP was maintained at 98% or more, and even when the antibody was stored for 14 days, a significant decrease in RCP was not confirmed.

Test Example 3-2: Aggregation Stability

The percentage of aggregates was confirmed by size exclusion chromatography (SEC). A Waters 2695 Separation Module or e2695 Separation Module was used as the liquid chromatography system, and a Waters 2489 UV/Vis detector was used as the UV detector. Analysis was performed under the following conditions.

HPLC Conditions

Column: TOSOH TSKgel Guardcolumn SWXL (6 mm x 4 cm), TOSOH TSKgel G3000SWXL (5 $\mu$m, 7.8 x 30 cm)
Column temperature: constant temperature around 25°C
Mobile phase: 0.1 mol/L sodium dihydrogen phosphate buffer solution (pH 6.8) containing 0.2 mol/L arginine hydrochloride
Flow rate: 1.0 mL per minute
Area measurement range: 15 minutes
UV detection wavelength: 280 nm

(Results)

The ratio of each component when stored for 14 days after the end of production is shown in Table 2. No significant increase in aggregates was observed when stored for 14 days after the end of production.

Table 2

| | | Proportion of main peak (%) | Proportion of aggregate peaks (%) |
|---|---|---|---|
| **$^{89}$ Zr - Tmab_N3** | Day 0 | **96.3** | **3.7** |
| | Day 7 | **96.4** | **3.7** |
| | Day 14 | **96.4** | **3.7** |
| **$^{89}$Zr-Tmab_6N3** | Day 0 | **96.8** | **3.2** |
| | Day 7 | **96.8** | **3.2** |
| | Day 14 | **96.9** | **3.2** |
| **$^{89}$Zr-Tmab_11N3** | Day 0 | **96.3** | **3.7** |
| | Day 7 | **96.4** | **3.6** |
| | Day 14 | **96.4** | **3.6** |
| **$^{89}$Zr-Tmab_75N3** | Day 0 | **92.9** | **7.0** |
| | Day 7 | **93.0** | **7.0** |
| | Day 14 | **93.0** | **7.0** |
| **$^{89}$Zr-Pmab_N3** | Day 0 | **91.2** | **8.8** |
| | Day 7 | **91.7** | **8.2** |
| | Day 14 | **91.5** | **8.5** |
| **$^{89}$Zr-Pmab_6N3** | Day 0 | **90.4** | **9.5** |
| | Day 7 | **91.3** | **8.6** |
| | Day 14 | **91.8** | **8.1** |
| **$^{89}$Zr-Pmab_11N3** | Day 0 | **88.2** | **11.7** |
| | Day 7 | **89.1** | **10.9** |
| | Day 14 | **89.2** | **10.8** |

Test Example 4: Evaluation of Heavy Chain Specificity Of Modification Reaction

The heavy chain specificity of the modification reaction was confirmed by SEC analysis. A Waters 2695 Separation Module or e2695 Separation Module was used as the liquid chromatography system, and a Waters 2489 UV/Vis detector was used as the UV detector. The sample was prepared and analyzed under the following conditions.

(Sample Preparation)

$^{89}$Zr-Tmab_N3, $^{89}$Zr-Tmab_6N3, and $^{89}$Zr-Tmab_11N3 prepared in Example 3 were diluted to 2 mg/mL with trastuzumab formulation buffer solution. In a 1.5 mL tube, 40 μL of a 7 mol/L guanidine hydrochloride-100 mmol/L Tris aqueous solution was mixed with 10 μL of the antibody solution. 1 μL of a 500 mmol/L DTT aqueous solution was mixed with this solution, and the mixture was allowed to stand at room temperature for 30 minutes to prepare a reduced sample (final DTT concentration: 10 mmol/L). A sample to which DTT was not added was used as a non-reduced sample.

A Waters 2695 Separation Module or e2695 Separation Module was used as the liquid chromatography system, a Waters 2489 UV/Vis detector was used as the UV detector, and a Gabi Star detector (manufactured by Raytest) was used as the RI detector. Analysis was performed under the following conditions.

HPLC Conditions
Column: TOSOH TSKgel Guardcolumn SWXL (6 mm x 4 cm), TOSOH TSKgel G3000SWXL (5 μm, 7.8 x 30 cm)
Column temperature: constant temperature around 25°C
Mobile phase: a mixed solution of water and acetonitrile (80:20) containing 0.1% TFA and 0.1% formic acid
Flow rate: 1.0 mL per minute
Area measurement range: 20 minutes
UV detection wavelength: 280 nm
Radioactivity detection range: 50 to 803 keV

The results of SEC analysis of the reduced sample and the non-reduced sample under the above conditions are shown in Fig. 14. In the non-reduced sample, both UV and RI were detected at a single peak. Meanwhile, in the reduced sample, two peaks derived from each of the heavy chain and the light chain were detected by the UV detector, and only a

single peak derived from the heavy chain was detected by the RI detector. Accordingly, it was confirmed that the RI labeling according to Example 3 was heavy chain specific.

Test Example 5: Antigen Binding Activity ($^{89}$Zr-Labeled Tmab)

The antigen binding activity of each of the radioconjugates according to Example 2 on the day after production was confirmed by in vitro ARG. Evaluation was performed in the same manner as in Test Example 1.

(Results)

Fig. 15 shows a corrected value (mean ± standard deviation) obtained by correcting a value obtained by dividing the count value in the ROI set in the used tumor section by the area of the ROI by a value obtained by dividing the count value of the standard radiation source by the area of the ROI. In each of the radioconjugates of Example 2, the binding activity for HER2 was confirmed. Each of the radioconjugates strongly bound only to SK-OV-3 tumor sections, and the HER2-selective binding activity was confirmed. No significant difference was confirmed in the binding activity among the four types of evaluated radioconjugates.

Test Example 6: Biodistribution Evaluation in Tumor-Bearing Mice ($^{89}$Zr-labeled Tmab)

The biodistribution of each radioconjugate according to Example 2 and Comparative Example 1 in each tissue in vivo was evaluated using tumor-bearing mice

(Preparation of Tumor-Bearing Mice)

The HER2-positive SK-OV-3 used in Test Example 1 was suspended in phosphate buffered saline (PBS), and 5 x $10^6$ cells was subcutaneously implanted to the flank of a 5-week-old female BALB/c-nu/nu (manufactured by Jackson Laboratory Japan, Inc) to prepare a tumor-bearing mice. 3 weeks after tumor implantation, it was confirmed that the tumor volume was 100 to 200 $mm^3$. The tumor volume was calculated according to the following formula.

Tumor volume ($mm^3$) = (tumor long diameter x (tumor short diameter)$^2$) x 1/2

(Biokinetic Evaluation)

Each of the radioconjugates according to Example 2 and Comparative Example 1 were administered to the tail vein at a dose of approximately 5 MBq/mouse (n = 3 in each group). PET imaging was performed under isoflurane anesthesia using a small-animal PET imaging device (PET/CT Si78, manufactured by Bruker) at 3, 24, 72, 168, and 240 hours (for Example 2 only) after administration of each radioconjugate and Comparative Example 1. As imaging conditions of PET, the acquisition time was set to 900 seconds, and the energy window was set to 357.7 to 664.3 keV. Image reconstruction was performed using the Maximum Likelihood-Expectation Maximization method (Iterations: 12) incorporating each correction (scatter correction, random correction, and attenuation correction), and PET images were obtained. After completion of the PET imaging, the obtained PET image was converted into a ratio of the radioactive concentration (Standard Uptake Value: SUV) of each part when the radioactive concentration in a case where it was assumed that the administered radioactive substance was uniformly distributed and not excreted was 1, using image analysis software PMOD (manufactured by PMOD). For the PET images converted to SUV, volumes of interest (VOIs) were set for the tumor, heart (intracardiac blood), liver, and kidneys, and quantitative analysis was performed.

(Results)

The tumor volume and body weight of each mouse when each radioconjugate and Comparative Example 1 were administered are shown in Table 3.

Table 3

| | Tumor volume ($mm^3$)<br>Mean ± Standard deviation | Body weight (g)<br>Mean ± Standard deviation |
|---|---|---|
| Comparative Example 1 | **154.4±23.4** | **19.9±0.7** |
| **$^{89}$Zr-Tmab_N3** | **184.5±47.6** | **20.1±1.2** |
| **$^{89}$Zr-Tmab_6N3** | **175.5±13.1** | **20.5±1.2** |

(continued)

| | Tumor volume ($mm^3$)<br>Mean ± Standard deviation | Body weight (g)<br>Mean ± Standard deviation |
|---|---|---|
| **$^{89}$Zr-Tmab_11N3** | **125.6±8.7** | **22.6±0.4** |
| **$^{89}$Zr-Tmab 75N3** | **121.5±9.6** | **21.8±1.1** |

The time-dependent changes in SUV (mean ± standard deviation) for the tumor, heart (intracardiac blood), liver, and kidneys are shown in Figs. 16A, 16B, 16C, and 16D, respectively. The accumulation of each radioconjugate according to Example 2 in each organ was higher than that in Comparative Example 1. From this, it was shown that in each of the radioconjugates according to Example 2, the retention in the whole body was increased due to the improvement in blood retention, and the accumulation in the tumor was also improved.

Test Example 7: Antigen Binding Activity ($^{89}$Zr-Labeled Pmab)

The antigen binding activity on the day after production of each radioconjugate according to Example 3 and Comparative Example 3 was confirmed by in vitro ARG. Evaluation was performed in the same manner as in Test Example 1 except that the tumor section used was A431 cells which were EGFR-positive human epithelioid cancer-derived cell lines purchased from the European Collection of Authenticated Cell Cultures (ECACC), A549 cells which were EGFR-positive human lung cancer-derived cell lines purchased from ECACC, SW48 cells which were EGFR-positive colon cancer-derived cell lines purchased from ATCC, HCT116 cells which were EGFR-positive human colon cancer-derived cell lines purchased from ATCC, and CAMA-1 cells which were EGFR-negative human breast cancer cell lines purchased from ATCC.

(Results)

Fig. 17 shows a corrected value (mean ± standard deviation) obtained by correcting a value obtained by dividing the count value in the ROI set in the used tumor section by the area of the ROI by a value obtained by dividing the count value of the standard radiation source by the area of the ROI. In each of the radioconjugates according to Example 3 and Comparative Example 3, the binding activity for EGFR was confirmed. Each of the radioconjugates and Comparative Example 3 strongly bound to the EGFR-positive tumor section, and the EGFR-selective binding activity was confirmed. No significant difference was confirmed in the binding activity among the four types of evaluated radioconjugates.

Test Example 8: Biodistribution Evaluation in Tumor-Bearing Mice ($^{89}$Zr-labeled Pmab)

The distribution of each of the radioconjugates according to Example 3 and that of each of tissues in the living body of Comparative Example 3 were evaluated using tumor-bearing mice.

(Preparation of Tumor-Bearing Mouse)

A431 cells, an EGFR-positive human epithelial cancer-derived cell line purchased from ECACC, were suspended in PBS and subcutaneously implanted to the flank of 5-week-old female BALB/c-nu/nu mice (purchased from Jackson Laboratory Japan, Inc.) at 5 x $10^6$ cells per mouse to prepare tumor-bearing mice. 2 weeks after tumor implantation, it was confirmed that the tumor volume was approximately 100 $mm^3$. The tumor volume was calculated in the same manner as in Test Example 6.

(Biokinetic Evaluation)

Each of the radioconjugates according to Example 3 and Comparative Example 3 were administered to the tail vein at a dose of approximately 5 MBq/mouse (n = 3 in each group). At 3, 24, 168, and 240 hours after administration of each radioconjugate, PET imaging under isoflurane anesthesia was performed using a small-animal PET imaging device (Si78) under the same conditions as in Test Example 6. After completion of PET imaging, image reconstruction was performed in the same manner as in Test Example 6, VOIs were set for the tumor, heart (intracardiac blood), liver, and kidneys, and quantitative analysis was performed.

(Results)

The tumor volume and body weight of each mouse when each radioconjugate according to Example 3 and Comparative Example 3 were administered are shown in Table 4.

Table 4

| | Tumor volume ($mm^3$) Mean ± Standard deviation | Body weight (g) Mean ± Standard deviation |
|---|---|---|
| Comparative Example 3 | **105.2±11.1** | **21.2±0.6** |
| **$^{89}$Zr-Pmab_N3** | **99.1±16.6** | **21.4±0.6** |
| **$^{89}$Zr-Pmab_6N3** | **105.9±17.4** | **21.3±1.4** |
| **$^{89}$Zr-Pmab 11N3** | **94.6±18.0** | **21.5±0.8** |

The time-dependent changes in SUV (mean ± standard deviation) for the tumor, heart (intracardiac blood), liver, and kidneys are shown in Figs. 18A, 18B, 18C, and 18D, respectively. The accumulation of each radioconjugate according to Example 3 in each organ was higher than that in Comparative Example 3. From this, it was shown that in each of the radioconjugates according to Example 3, the retention in the whole body was increased due to the improvement in blood retention, and the accumulation in the tumor was also improved.

Example 4: Production of Radioactive Metal Nuclide ($^{225}$Ac) Labeled Trastuzumab Conjugate

(Production of $^{225}$Ac-Labeled DOTAGA-DBCO)

DOTAGA-DBCO was dispersed in 0.1 mol/L sodium acetate buffer solution (pH 5.0) as a solvent to form a dispersion containing 0.3 mmol/L of a chelating agent. A reaction solution in which 94.1 μL of this dispersion and approximately 8.87 MBq of a $^{225}$Ac ion-containing solution (0.1 mol/L hydrochloric acid aqueous solution, radioactive concentration: 217.4 MBq/mL, prepared from material manufactured by State Atomic Energy Corporation Rosatom, liquid volume: 40.8 μL) as a radioactive metal source were mixed was reacted under heating conditions to obtain a $^{225}$Ac complex solution. The reaction solution was heated at a heating temperature of 70°C for a heating time of 90 minutes.

The RCP of the obtained $^{225}$Ac complex was measured by the following method. That is, a part of the $^{225}$Ac complex solution was developed by TLC (manufactured by Agilent, model number: SGI0001, developing solvent: acetonitrile/water mixed solution (volume ratio 1:1)), and then measured by a radio γ-TLC analyzer (MODEL GITA Star from Raytest). The percentage of the radioactivity (counts) of the peak detected near the origin relative to the total detected radioactivity (counts) was defined as the RCP of the $^{225}$Ac complex. As a result, the RCP of the $^{225}$Ac complex was 95.1%. The obtained $^{225}$Ac complex solution was used as it was in the step of labeling the antibody.

(Labeling Step to Antibody)

To the solution of the unpurified $^{225}$Ac complex obtained through the above steps, a solution containing Tmab_N3, Tmab_6N3, and Tmab_11N3 with an azide group linker introduced therein was added in the same manner as in Example 3, and a click reaction was performed at 37°C for 120 minutes to obtain a $^{225}$Ac-labeled antibody.

In $^{225}$Ac-Tmab_N3, which is a radiolabeled antibody represented by Formula (4), S is Lys-Gly-Gly, the amino group on the side chain of the Lys in S of Formula (4) is substituted with P2, P2 is a group represented by Formula (5), L2 is a group represented by Formula VI-1, Ch is a group represented by Formula V, wherein $R^5$, $R^6$, $R^7$, and $R^8$ are each independently $-CH_2COOH$, $R^9$ is a substituent for binding to Formula VI-1, M is $^{225}$Ac, n is 2, and Ab is trastuzumab.

$^{225}$Ac-Tmab_6N3 is a radiolabeled linker represented by Formula (4-2) in which $^{225}$Ac forms a complex, in which q is 5, to which 2 molecules of trastuzumab are bound.

$^{225}$Ac-Tmab_11N3 is a radiolabeled linker represented by Formula (4-1) in which $^{225}$Ac forms a complex, in which p is 3, to which 2 molecules of trastuzumab are bound.

The amount of the $^{225}$Ac complex and the amount of the azide group linker-modified antibody (bivalent) were 5.7 nmol and 10.1 nmol, respectively. The reaction rates of the unpurified $^{225}$Ac-labeled antibody were 41.3% (Tmab_N3), 48.0% (Tmab_6N3), and 44.6% (Tmab_11N3), respectively. The obtained $^{225}$Ac-labeled antibody was purified using an ultrafiltration filter (manufactured by Merck, model number: UFC505096). The RCP of the $^{225}$Ac-labeled antibody after purification was 86.8% ($^{225}$Ac-Tmab_N3), 88.7% ($^{225}$Ac-Tmab_6N3), and 89.8% ($^{225}$Ac-Tmab_11N3), respectively.

Comparative Example 4: Production of $^{225}$Ac-Trastuzumab Conjugate (Monovalent) by CCAP Method

(Labeling Step to Antibody)

To the unpurified $^{225}$Ac complex solution obtained in Example 4, Solution C obtained in Comparative Example 1 was added, and a click reaction was performed at 37°C for 120 minutes to obtain the $^{225}$Ac-labeled antibody. The amount of the $^{225}$Ac complex and the amount of the azide group linker-modified antibody (bivalent) were 5.7 nmol and 10.1 nmol, respectively. The reaction rate of the unpurified $^{225}$Ac-labeled antibody was 38.4%. The RCP of the purified $^{225}$Ac-trastuzumab conjugate (monovalent) (hereinafter also referred to as "Comparative Example 4") was 93.4%.

Test Example 9: Stability Evaluation

Each of the radioconjugates ($^{225}$Ac-Tmab_N3, $^{225}$Ac-Tmab_6N3, $^{225}$Ac-Tmab_11N3) produced in Example 4 was stored in a refrigerator (5°C) for 2 weeks, and RCP and the ratio of aggregates were evaluated at each time point (day 0, day 7, and day 14). The period of 14 days from the end of production corresponds to approximately 1.5 half-life when the radioactive metal nuclide is $^{225}$Ac.

Test Example 9-1: RCP

RCP was analyzed by TLC. The conditions for TLC were the same as the conditions for calculating the reaction rate in Example 1.

(Results)

The RCP of each radioconjugate is shown in Table 5.

Table 5

| | RCP (%) | | |
|---|---|---|---|
| | Day 0 | Day 7 | Day 14 |
| $^{225}$Ac-Tmab_N3 | 86.8 | 90.9 | 86.9 |
| $^{225}$Ac-Tmab_6N3 | 88.7 | 86.4 | 84.2 |
| $^{225}$Ac -Tmab_11N3 | 89.8 | 89.0 | 83.7 |

When stored for 14 days under refrigeration after the end of production, the RCP of each of the radioconjugates according to Example 4 was maintained at 83.7% or more, and a significant decrease in RCP was not confirmed.

Test Example 9-2: Aggregation Stability

The ratio of aggregates was confirmed by SEC. The measurement conditions were the same as in Test Example 3-2.

(Results)

The ratio of each component when stored for 22 days after the end of production is shown in Table 6. No significant increase in aggregates was observed when stored for 22 days after the end of production.

Table 6

| | | Proportion of main peak (%) | Proportion of aggregate peaks (%) |
|---|---|---|---|
| **$^{225}$Ac-Tmab_N3** | Day 0 | **96.6** | **3.4** |
| | Day 22 | **96.0** | **1.0** |
| **$^{225}$Ac-Tmab_6N3** | Day 0 | **96.9** | **3.1** |
| | Day 22 | **96. 1** | **3.9** |
| **$^{225}$Ac-Tmab_11N3** | Day 0 | **96.8** | **3.2** |
| | Day 22 | **96.0** | **1.0** |

Test Example 10: Antigen Binding Activity ($^{225}$Ac-Labeled Tmab)

The antigen binding activity on the day after production of each radioconjugate according to Example 4 and Comparative Example 4 was confirmed by in vitro ARG. Evaluation was performed in the same manner as in Test Example 1 except that the radioactive concentration of the used reaction solution was 1 kBq/mL. Fig. 19 shows a corrected value (mean ± standard deviation) obtained by correcting a value obtained by dividing the count value in the ROI set in the used tumor section by the area of the ROI by a value obtained by dividing the count value of the standard radiation source by the area of the ROI. In each of the radioconjugates according to Example 4 and Comparative Example 4, the binding activity for HER2 was confirmed. Each of the radioconjugates and Comparative Example 4 bound only to SK-OV-3 tumor sections, and the HER2-selective binding activity was confirmed.

Test Example 11: In Vivo Drug Efficacy Evaluation ($^{225}$Ac-Labeled Tmab)

The antitumor effect of each radioconjugate according to Example 4 and Comparative Example 4 was evaluated using tumor-bearing mice.

(Preparation of Tumor-Bearing Mice)

SK-OV-3 tumor-bearing mice were prepared in the same manner as in Test Example 6. 2 weeks after tumor implantation, animals with tumors of shapes suitable for tumor diameter measurement were randomly assigned to groups. Each of the radioconjugates according to Example 4 and Comparative Examples 4 were intravenously administered to the tail at a dose of 5 kBq/mouse (15 μg/mouse as trastuzumab). As a control group, a vehicle group to which a preservation buffer solution was intravenously administered was set. There were 6 animals in each group, and over time until 40 days after administration, the general condition was observed and body weight and tumor volume were measured. In the vehicle group, due to fighting among the animals during the housing period, some animals reached the humane endpoint, and from day 8 after administration onward, only one animal remained in the group.

(Results)

The tumor volume and body weight of each mouse when each radioconjugate according to Example 4 and Comparative Example 4 were administered are shown in Table 7.

Table 7

| | Tumor volume ($mm^3$)<br>Mean ± Standard deviation | Body weight (g)<br>Mean ± Standard deviation |
|---|---|---|
| Vehicle group | **481.0±61.7** | **16.6±1.7** |
| Comparative Example 4 | **468.8±113.1** | **16.7±1.3** |
| **$^{225}$Ac-Tmab_N3** | **514.9± 107.5** | **18.0±2.4** |
| **$^{225}$Ac-Tmab_6N3** | **499.2±114.2** | **17.0±1.1** |
| **$^{225}$Ac-Tmab_11N3** | **488.6±68. 6** | **17.0±1.6** |

The change in tumor volume over time is shown in Fig. 20. The vertical axis in Fig. 20 represents a relative value when the tumor volume at the time of administration of each drug is 1. The horizontal axis in Fig. 20 indicates the number of days elapsed from administration of each drug. Graphs represent mean ± standard deviation of tumor volume for each group. In the group to which the radioconjugate was administered, the increase in the tumor volume was moderate as compared with the vehicle group, and the drug efficacy by the administration of the radioconjugate was confirmed. In addition, each of the radioconjugates according to Example 4 exhibited an antitumor effect equivalent to or more than that of Comparative Example 4. There was no significant change in general condition in each individual, and no toxic signs such as significant body weight loss were observed.

The above-described embodiments are for describing the present invention, and do not limit the scope of the present invention. That is, the scope of the present invention is indicated by the claims rather than the embodiments. Various modifications made within the scope of the claims and the meaning of the invention equivalent thereto are regarded as being within the scope of the present invention.

The present invention is useful for producing a radiolabeled antibody.

## Claims

1. A method for producing a radiolabeled antibody, comprising:

a conjugation step of reacting a compound represented by the following Formula (1) or a salt thereof, with IgG,

P1-S-Y-B Formula (1)

wherein
P1 is a labeling site labeled with a radioisotope or a labeling site capable of being labeled with a radioisotope,
S is a spacer,
Y is a reaction site that reacts with IgG, and
B is an antibody binding site containing an IgG-binding peptide that specifically binds to an Fc region of IgG, and
wherein Y in the Formula (1) has a group represented by Formula I below:

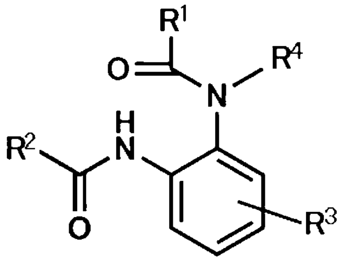

Formula I

wherein
$R^1$ is a substituent such that a pKa of $R^1$-H is 4 to 14,
$R^2$ is bound to S in the Formula (1),
$R^3$ is bound to B in the Formula (1), and
$R^4$ is H or a substituted or unsubstituted alkyl group having 1 to 8 carbon atoms.

2. The method for producing a radiolabeled antibody according to claim 1, wherein
$R^1$ is a nitrophenoxy group.

3. The method for producing a radiolabeled antibody according to claim 1 or 2, wherein

B in the Formula (1) is represented by
any one of Formulas II, III, and IV below, with the IgG-binding peptide being $R^{3a}$:

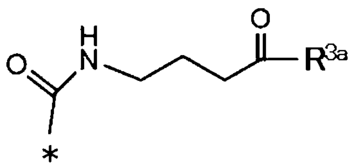

Formula II

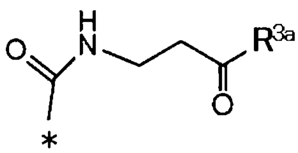

Formula III

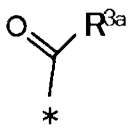

Formula IV

wherein * indicates a carbon of a benzene ring of the Formula I.

**4.** The method for producing a radiolabeled antibody according to claim 1 or 2, wherein

an amino acid sequence of the IgG-binding peptide is
an amino acid sequence shown in any one of SEQ ID NOs. 1 to 151.

**5.** The method for producing a radiolabeled antibody according to claim 1 or 2, wherein

S in the Formula (1) includes Lys-Gly-Gly, and
an amino group of a side chain of Lys is substituted with P1 in the Formula (1).

**6.** The method for producing a radiolabeled antibody according to claim 1 or 2, wherein
S in the Formula (1) is selected from the group consisting of a polyethylene glycol chain, an alkylene chain, and a combination thereof.

**7.** The method for producing a radiolabeled antibody according to claim 1 or 2, wherein

the radioisotope is
a radioactive halogen atom, $Al^{18}F$, $^{64}Cu$, $^{68}Ga$, $^{89}Zr$, $^{90}Y$, $^{99m}Tc$, $^{111}In$, $^{177}Lu$, or $^{225}Ac$.

**8.** The method for producing a radiolabeled antibody according to claim 1 or 2, wherein

the radioisotope is
a radioactive metal nuclide.

**9.** The method for producing a radiolabeled antibody according to claim 1 or 2, wherein

P1 in the Formula (1) is a labeling site labeled with a radioisotope, and
in the conjugation step,
a modified antibody represented by the following Formula (2) is obtained,

$(P1\text{-}S)_n\text{-}Ab$    Formula (2)

wherein
Ab is IgG, and
n is 1 or 2.

**10.** The method for producing a radiolabeled antibody according to claim 1 or 2, wherein

P1 in the Formula (1) is a labeling site capable of being labeled with a radioisotope, and
in the conjugation step,
a modified antibody represented by the following Formula (2) is obtained,

$(P1\text{-}S)_n\text{-}Ab$    Formula (2)

wherein
Ab is IgG, and
n is 1 or 2.

**11.** The method for producing a radiolabeled antibody according to claim 9, further comprising:
a labeling step of labeling the labeling site with the radioisotope.

**12.** The method for producing a radiolabeled antibody according to claim 1 or 2, wherein

P1 in the Formula (1) is a labeling site having a first click reactive group and capable of being labeled with a radioisotope, and
in the conjugation step,
a modified antibody represented by the following Formula (2) is obtained,

$(P1\text{-}S)_n\text{-}Ab$ Formula (2)

wherein
Ab is IgG, and
n is 1 or 2.

**13.** The method for producing a radiolabeled antibody according to claim 12, further comprising:

a complex formation step of reacting a compound represented by the following Formula (3) with the radioisotope,

Ch-L1 Formula (3)

wherein
Ch is a chelating agent, and
L1 is a linker having a second click reactive group that reacts with the first click reactive group,
to form a complex of the radioisotope and the chelating agent, thereby obtaining a labeling compound.

**14.** The method for producing a radiolabeled antibody according to claim 13, further comprising:
a labeling step of labeling the modified antibody with the radioisotope by reacting the first click reactive group possessed by the modified antibody obtained in the conjugation step with the second click reactive group possessed by the labeling compound obtained in the complex formation step.

**15.** The method for producing a radiolabeled antibody according to claim 13, wherein

the chelating agent has a structure derived from a compound represented by the following Formula V, or a salt thereof,

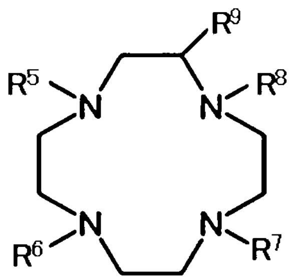

Formula V

wherein $R^5$, $R^6$, and $R^7$ are each independently - $(CH_2)_pCOOH$, $-(CH_2)_pC_5H_5N$, $-(CH_2)_pPO_3H_2$, $-(CH_2)_pCONH_2$, or - $(CHCOOH)(CH_2)_pCOOH$,
one of $R^8$ and $R^9$ is a hydrogen atom, a carboxyl group, or a carboxyalkyl group having 2 or 3 carbon atoms, and the other is a substituent for binding to S in the Formula (2),
p is an integer of 0 to 3,
when $R^8$ is a substituent for binding to S in the Formula (2), $R^9$ is a hydrogen atom, and
and when $R^8$ is not a substituent for binding to S in the Formula (2), $R^9$ is a substituent for binding to S in the Formula (2).

**16.** A radiolabeled antibody represented by Formula (4) below:

(P2-S)n-Ab Formula (4)

wherein

P2 contains a radioisotope,
S is a spacer,
Ab is IgG, and
n is 1 or 2, wherein
S in the Formula (4) includes Lys-Gly-Gly, an amino group of a side chain of Lys in S in the Formula (4) is substituted with P2, and

S in the Formula (4) is specifically bound to Lys in an Fc region of IgG.

**17.** A radiolabeled antibody represented by Formula (4) below:

(P2-S)n-Ab Formula (4)

wherein

P2 contains a radioisotope,
S is a spacer,
Ab is IgG, and
n is 1 or 2, wherein
S in the Formula (4) is selected from the group consisting of a polyethylene glycol chain, an alkylene chain, and a combination thereof, and
S in the Formula (4) is specifically bound to Lys in an Fc region of IgG.

**18.** The radiolabeled antibody according to claim 16 or 17, wherein
P2 is a complex site that contains a complex formed by complex formation between the radioisotope and a chelating agent.

**19.** The radiolabeled antibody according to claim 16 or 17, wherein

the radioisotope is
a radioactive halogen atom, $Al^{18}F$, $^{64}Cu$, $^{68}Ga$, $^{89}Zr$, $^{90}Y$, $^{99m}Tc$, $^{111}In$, $^{177}Lu$, or $^{225}Ac$.

**20.** The radiolabeled antibody according to claim 18, wherein

the chelating agent has a structure derived from a compound represented by the following Formula V or a salt thereof,

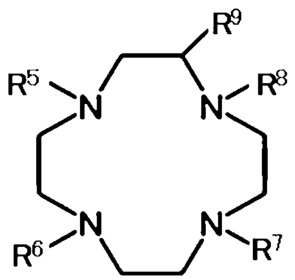

Formula V

wherein $R^5$, $R^6$, and $R^7$ are each independently - $(CH_2)_pCOOH$, $-(CH_2)_pC_5H_5N$, $-(CH_2)_pPO_3H_2$, $-(CH_2)_pCONH_2$, or - $(CHCOOH)(CH_2)_pCOOH$,
one of $R^8$ and $R^9$ is a hydrogen atom, a carboxyl group, or a carboxyalkyl group having 2 or 3 carbon atoms, and the other is a substituent for binding to S in the Formula (4),
p is an integer of 0 to 3,
when $R^8$ is a substituent for binding to S in the Formula (4), $R^9$ is a hydrogen atom, and
and when $R^8$ is not a substituent for binding to S in the Formula (4), $R^9$ is a substituent for binding to S in the Formula (4).

**21.** The radiolabeled antibody according to claim 16 or 17, wherein
in the Formula (4), n is 2.

**22.** The radiolabeled antibody according to claim 16 or 17, wherein
the IgG is trastuzumab or panitumumab.

**23.** The radiolabeled antibody according to claim 18, wherein

P2 in the Formula (4) includes a group represented by Formula (5) below:

ChM-L2 (5)

wherein ChM is the complex site, and L2 is a linker.

**24.** The radiolabeled antibody according to claim 23, wherein L2 in the Formula (5) includes

any one of the groups represented by Formulas VI-1, VI-2, or VI-3 below:

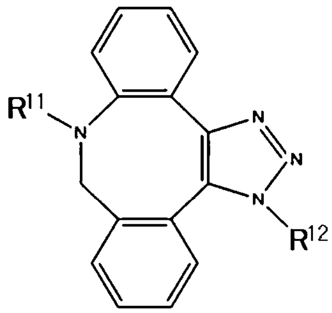

Formula VI-1

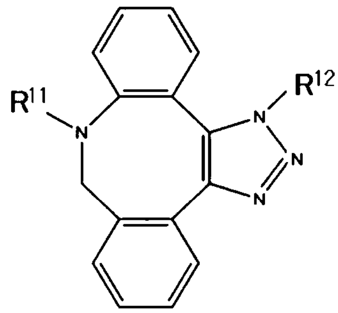

Formula VI-2

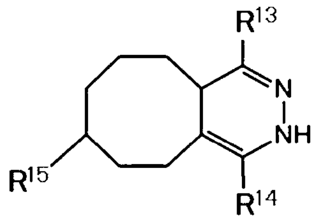

Formula VI-3

wherein, $R^{11}$ indicates a binding site on ChM side in the Formula (5), and $R^{12}$ indicates a binding site on S side in the Formula (4), and
wherein, one of $R^{13}$ and $R^{14}$ is a hydrogen atom, a methyl group, a phenyl group, or a pyridyl group, and the other indicates a binding site on ChM side in the Formula (5), and $R^{15}$ indicates a binding site on S side in the Formula (4).

**25.** A radiopharmaceutical comprising,
as an active ingredient, the radiolabeled antibody according to claim 16 or 17.

**26.** The radiopharmaceutical according to claim 25,
which is used for radionuclide therapy of cancer or for diagnosis of cancer.

# FIG. 1

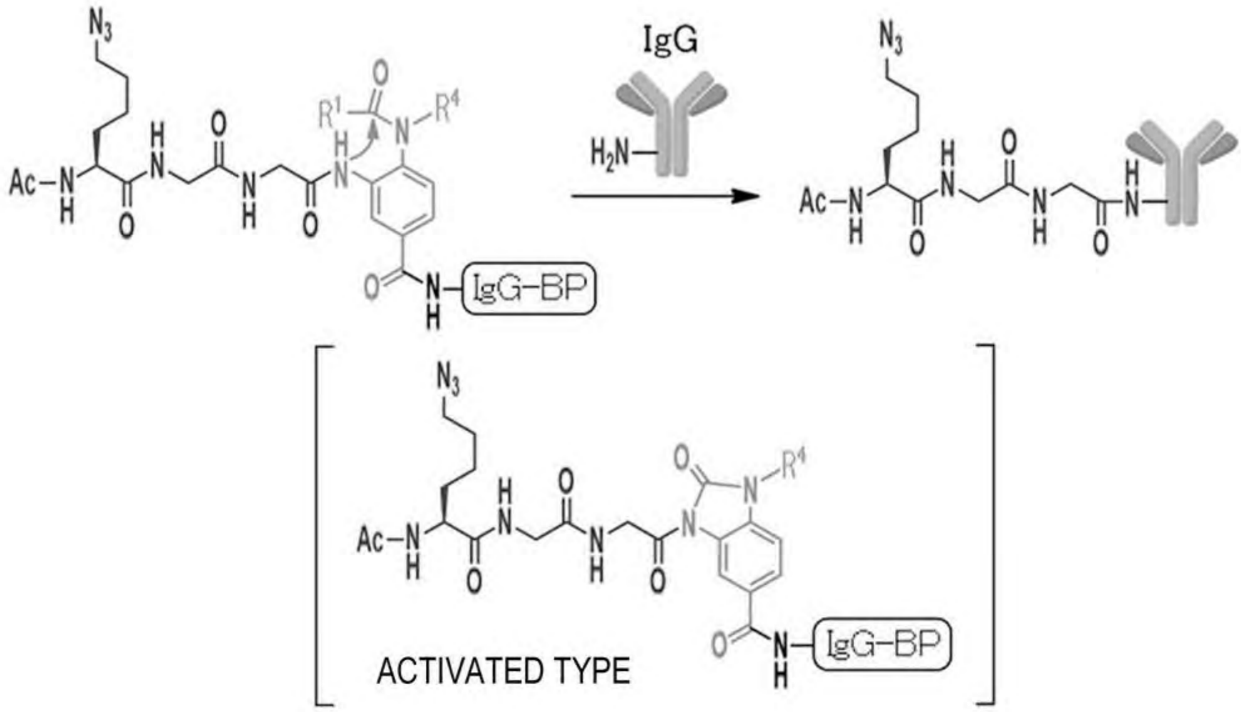
IgG
ACTIVATED TYPE

# FIG. 2

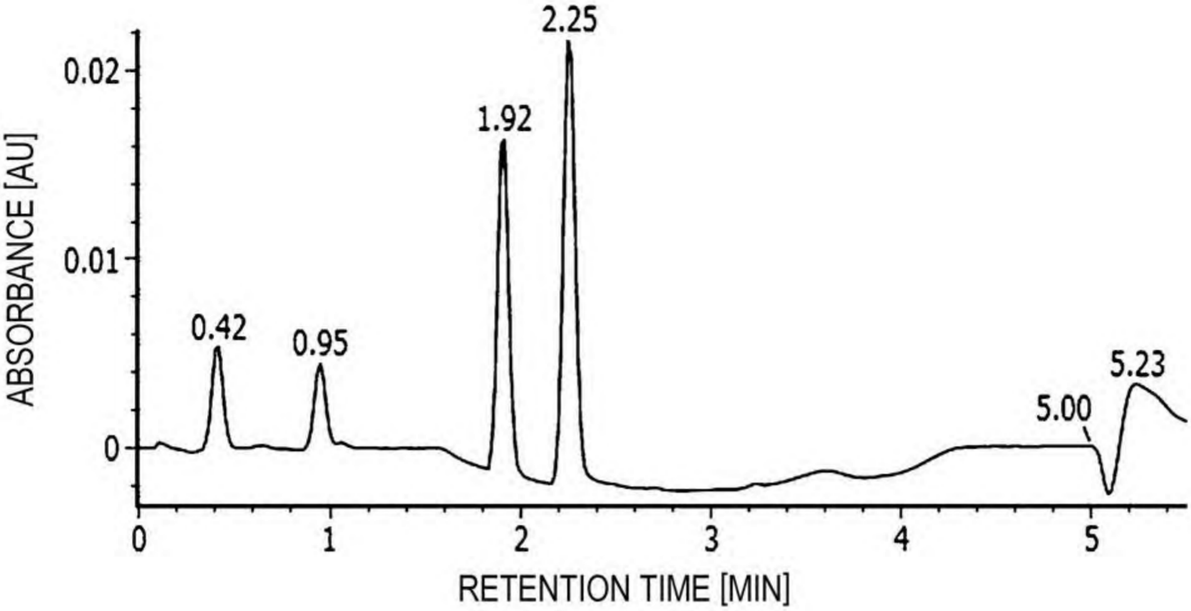
ABSORBANCE [AU]
0.02
0.01
0
2.25
1.92
0.42
0.95
5.00
5.23
0
1
2
3
4
5
RETENTION TIME [MIN]

FIG. 3A

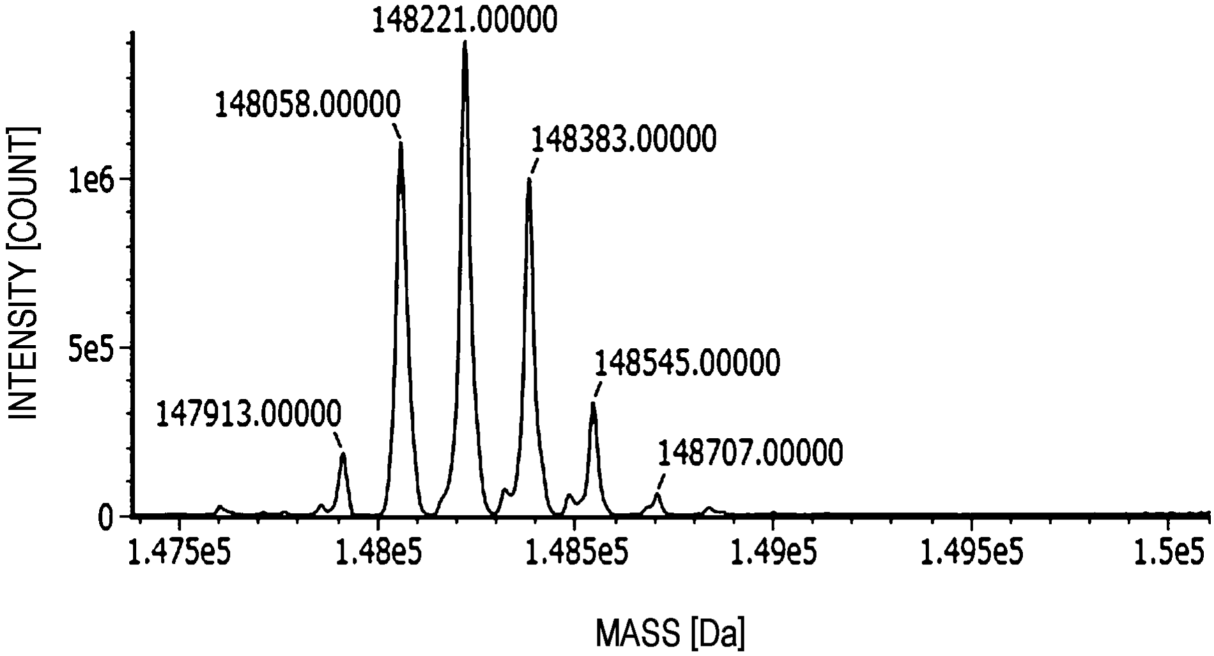
148221.00000
148058.00000
148383.00000
148545.00000
147913.00000
148707.00000
INTENSITY [COUNT]
1e6
5e5
0
1.475e5
1.48e5
1.485e5
1.49e5
1.495e5
1.5e5
MASS [Da]

FIG. 3B

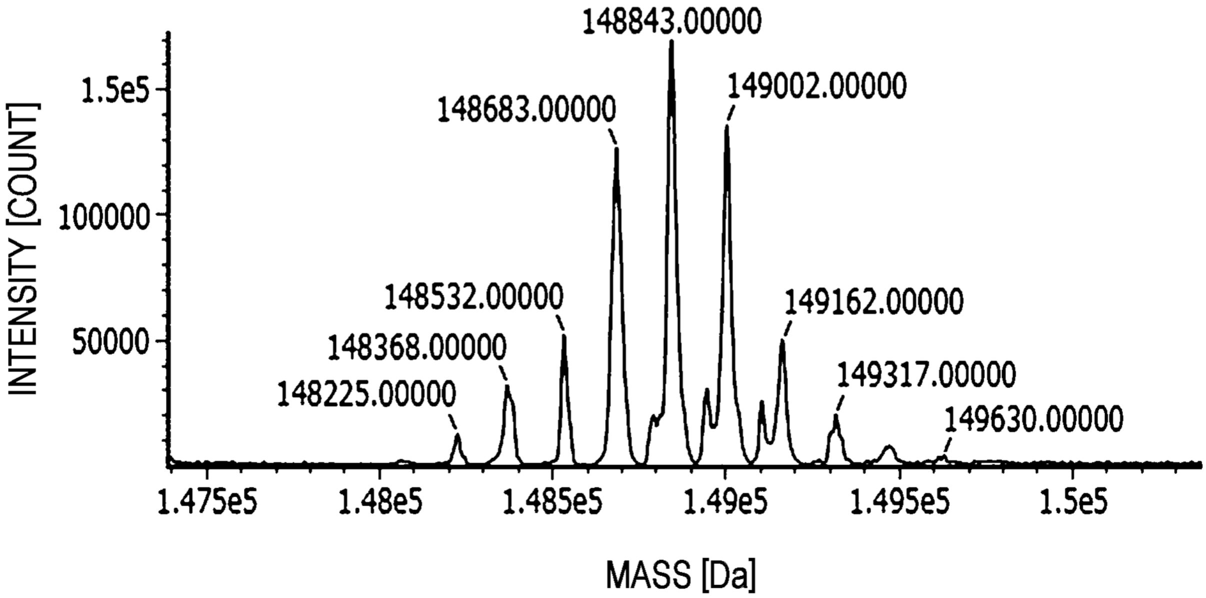
148843.00000
149002.00000
148683.00000
148532.00000
149162.00000
148368.00000
149317.00000
148225.00000
149630.00000
INTENSITY [COUNT]
1.5e5
100000
50000
1.475e5
1.48e5
1.485e5
1.49e5
1.495e5
1.5e5
MASS [Da]

FIG. 4A

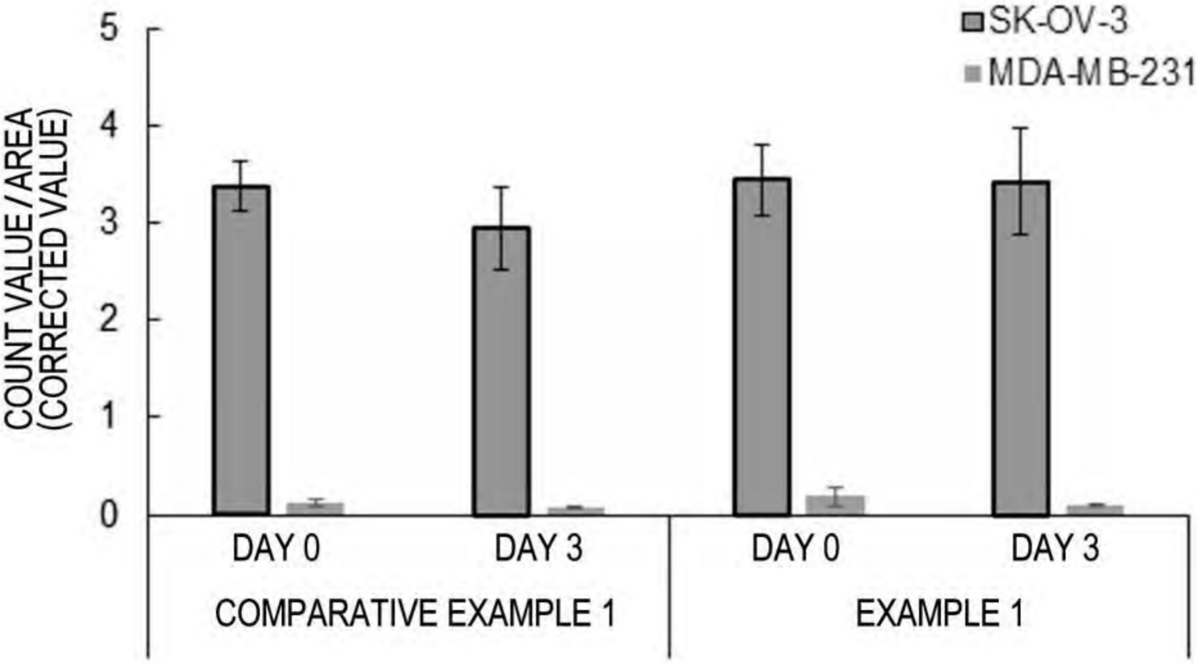
COUNT VALUE / AREA (CORRECTED VALUE)
5
4
3
2
1
0
SK-OV-3
MDA-MB-231
DAY 0
DAY 3
DAY 0
DAY 3
COMPARATIVE EXAMPLE 1
EXAMPLE 1

FIG. 4B

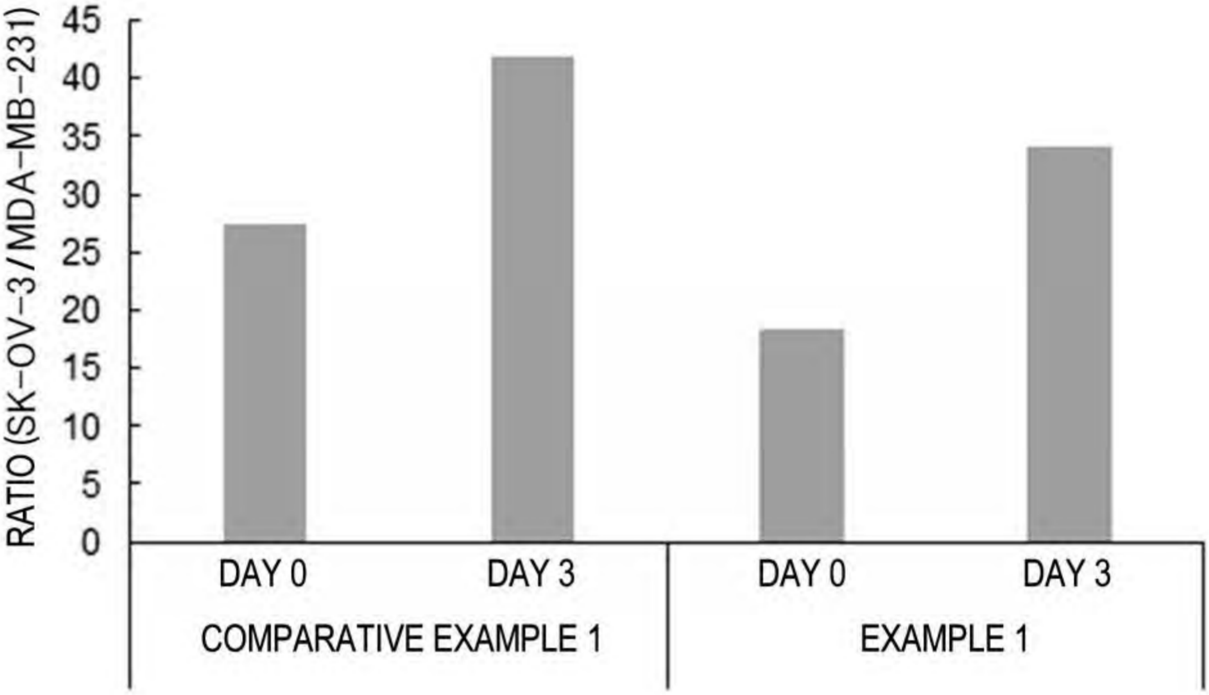
RATIO (SK−OV−3 / MDA−MB−231)
45
40
35
30
25
20
15
10
5
0
DAY 0
DAY 3
DAY 0
DAY 3
COMPARATIVE EXAMPLE 1
EXAMPLE 1

FIG. 5

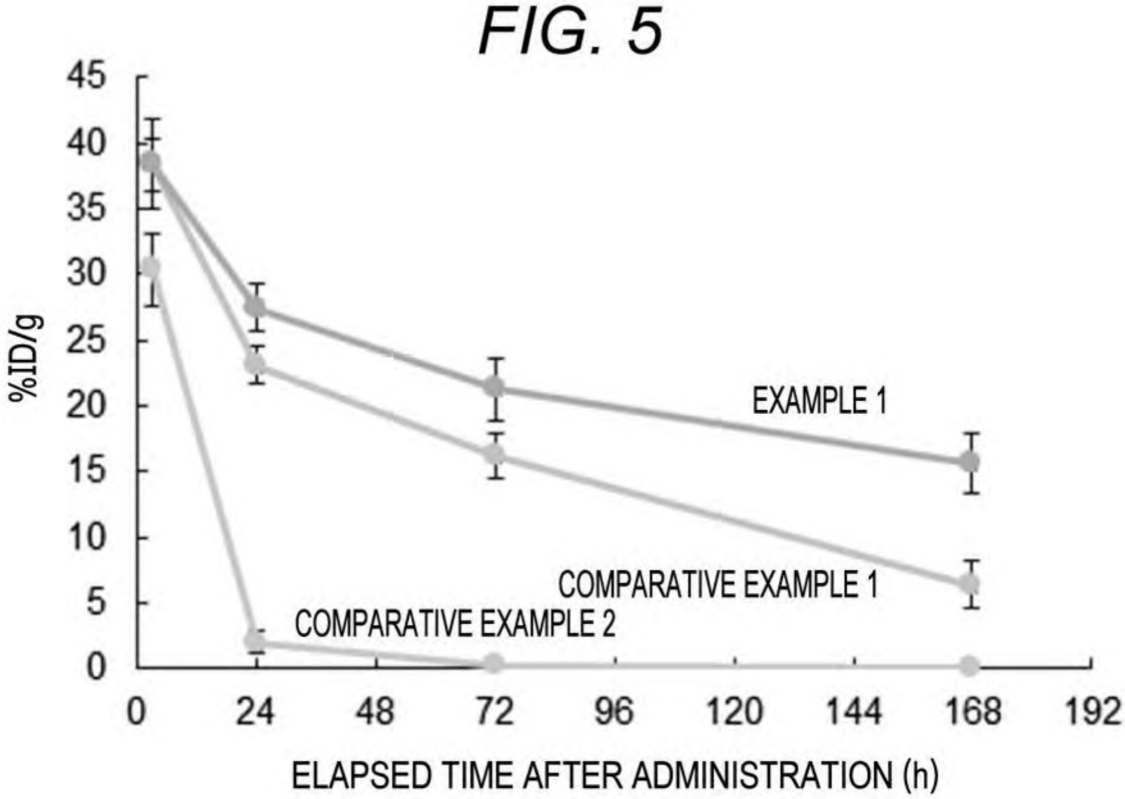
45
40
35
30
25
20
15
10
5
0
%ID/g
EXAMPLE 1
COMPARATIVE EXAMPLE 1
COMPARATIVE EXAMPLE 2
0
24
48
72
96
120
144
168
192
ELAPSED TIME AFTER ADMINISTRATION (h)

FIG. 6A

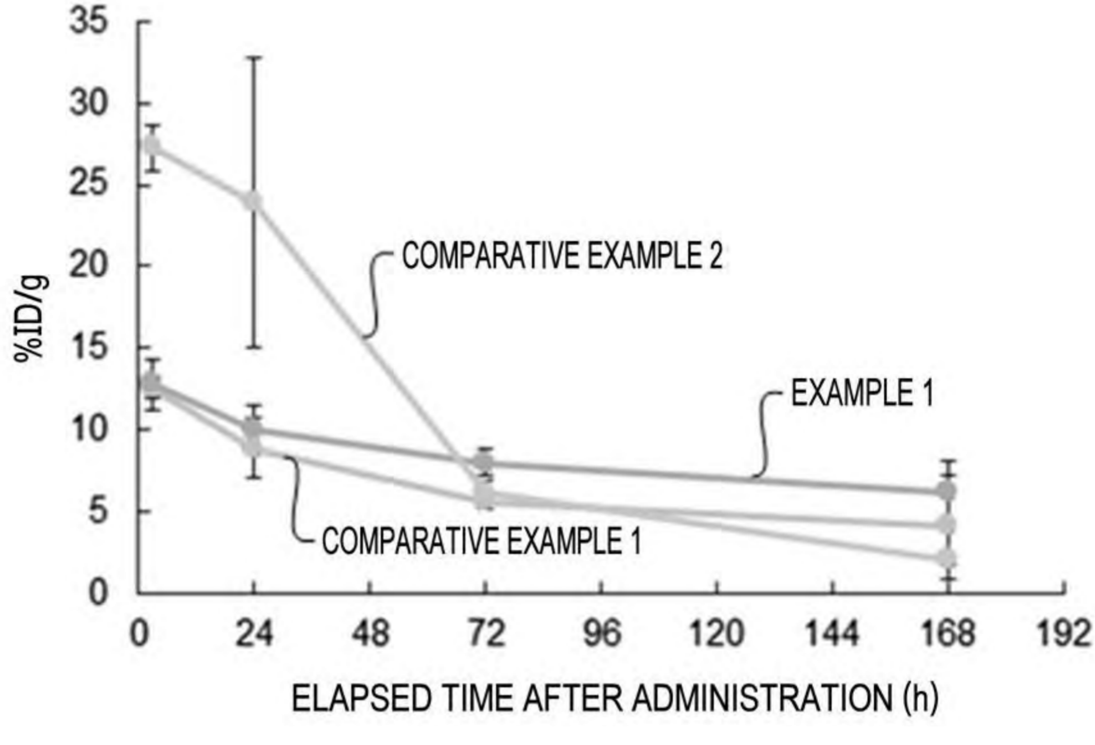
35
30
25
20
15
10
5
0
%ID/g
COMPARATIVE EXAMPLE 2
EXAMPLE 1
COMPARATIVE EXAMPLE 1
0
24
48
72
96
120
144
168
192
ELAPSED TIME AFTER ADMINISTRATION (h)

FIG. 6B

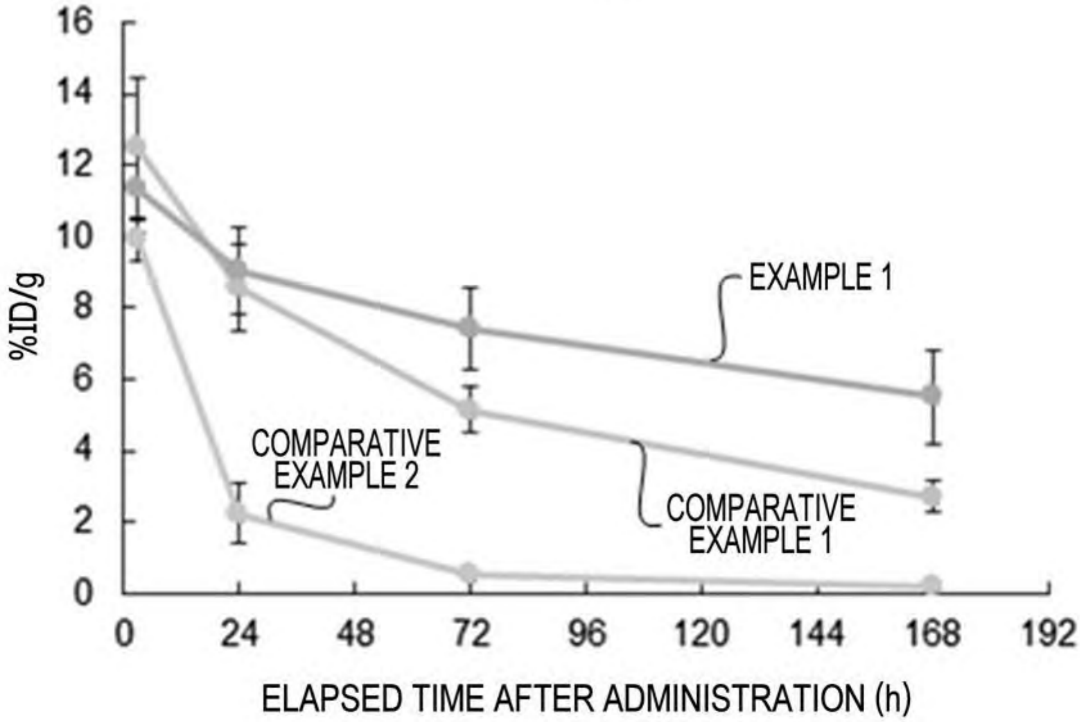
16
14
12
10
8
6
4
2
0
%ID/g
0
24
48
72
96
120
144
168
192
ELAPSED TIME AFTER ADMINISTRATION (h)
EXAMPLE 1
COMPARATIVE EXAMPLE 1
COMPARATIVE EXAMPLE 2

FIG. 6C

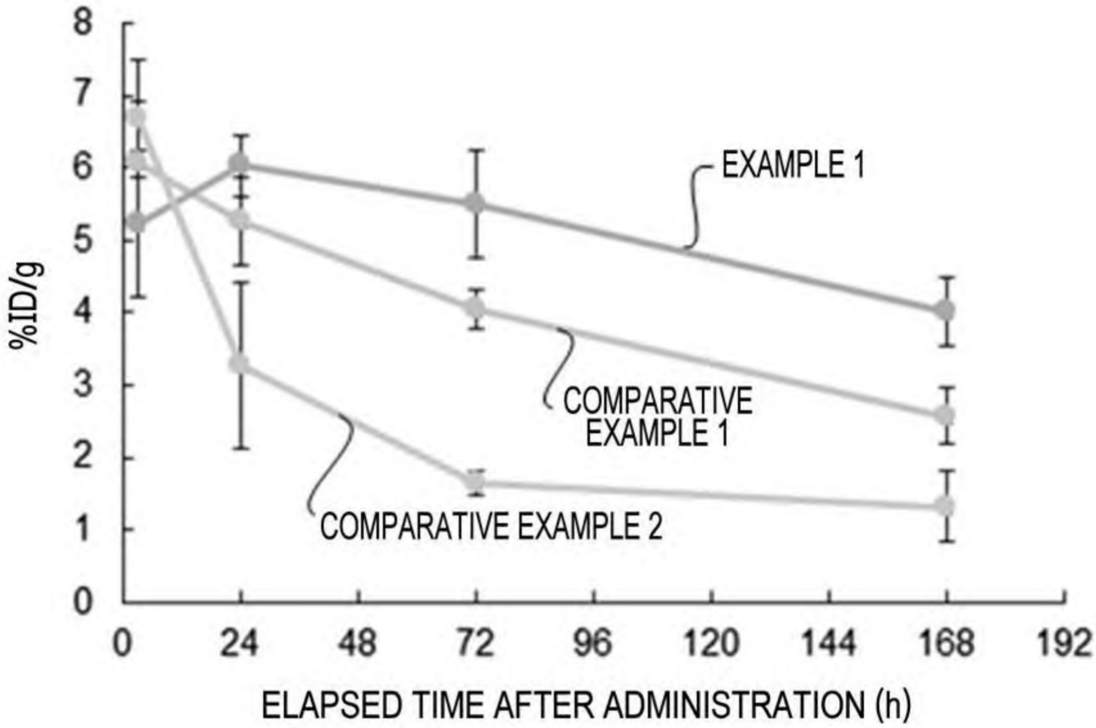
8
7
6
5
4
3
2
1
0
%ID/g
0
24
48
72
96
120
144
168
192
ELAPSED TIME AFTER ADMINISTRATION (h)
EXAMPLE 1
COMPARATIVE EXAMPLE 1
COMPARATIVE EXAMPLE 2

FIG. 6D

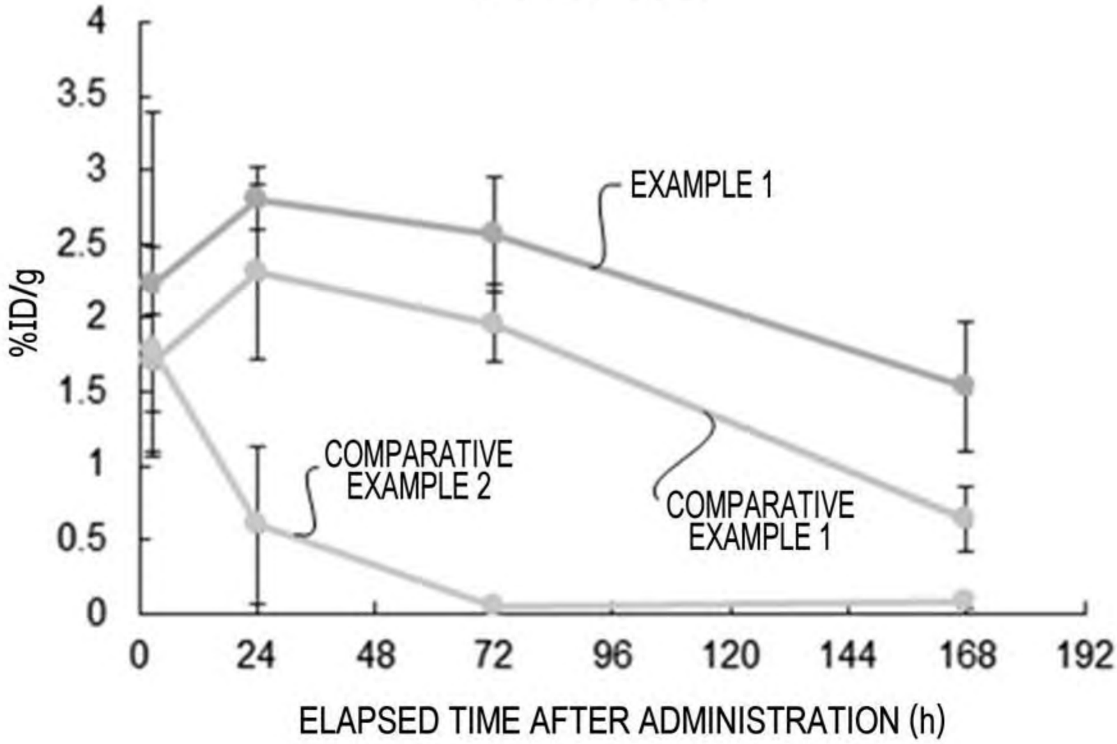
4
3.5
3
2.5
2
1.5
1
0.5
0
%ID/g
EXAMPLE 1
COMPARATIVE EXAMPLE 2
COMPARATIVE EXAMPLE 1
0
24
48
72
96
120
144
168
192
ELAPSED TIME AFTER ADMINISTRATION (h)

FIG. 6E

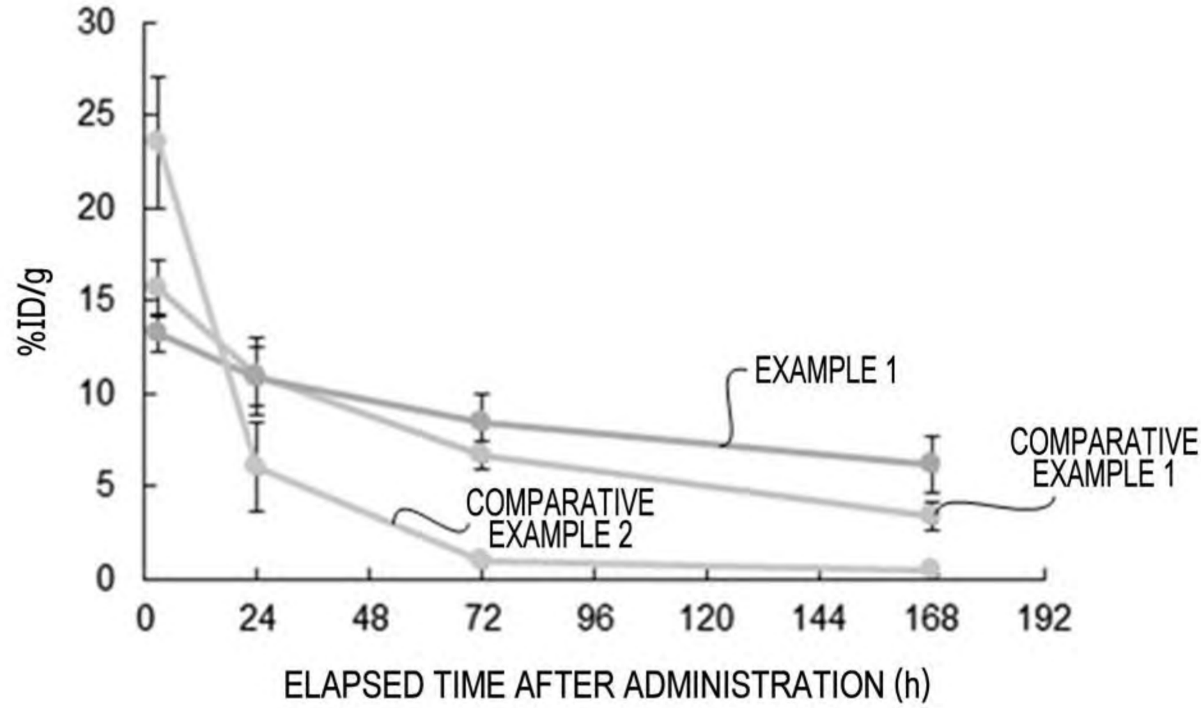
30
25
20
15
10
5
0
%ID/g
EXAMPLE 1
COMPARATIVE EXAMPLE 1
COMPARATIVE EXAMPLE 2
0
24
48
72
96
120
144
168
192
ELAPSED TIME AFTER ADMINISTRATION (h)

FIG. 7A

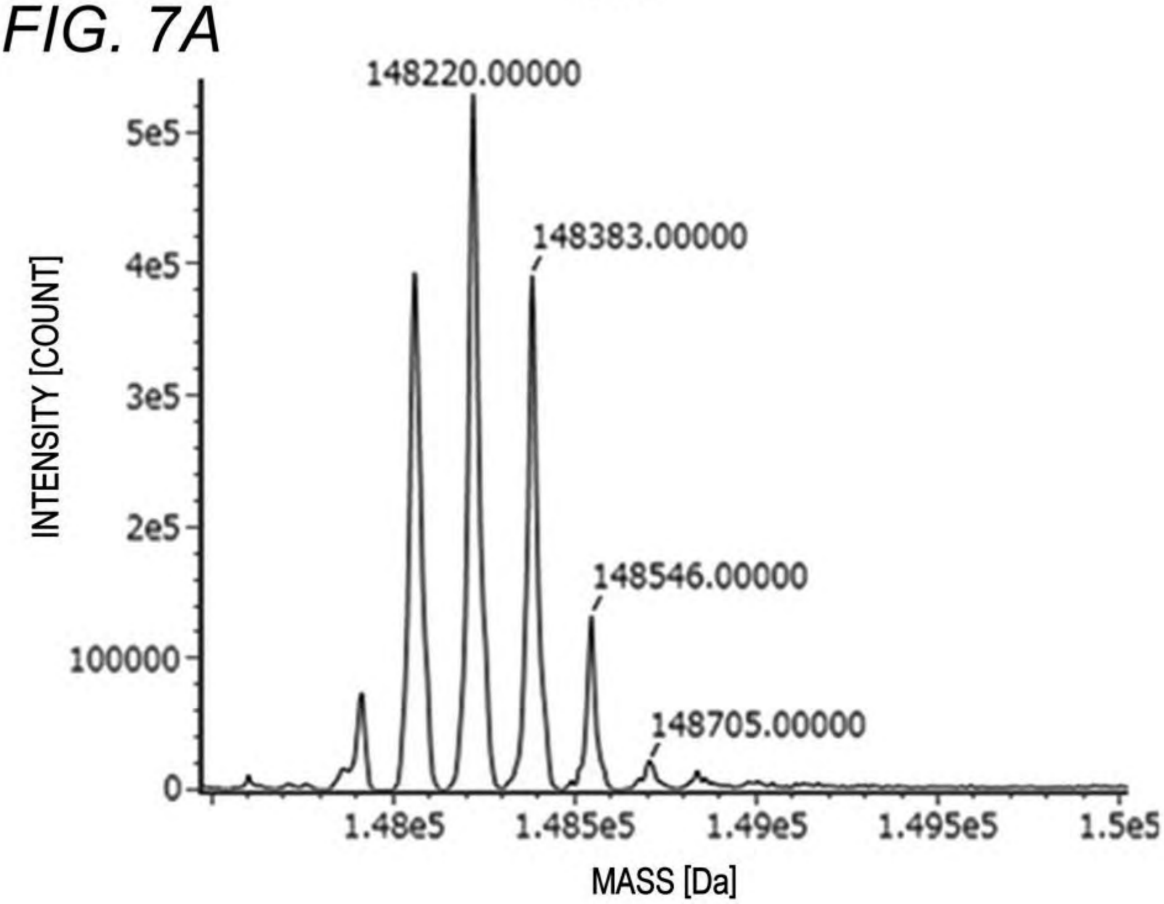
148220.00000
148383.00000
148546.00000
148705.00000
5e5
4e5
3e5
2e5
100000
0
INTENSITY [COUNT]
1.48e5
1.485e5
1.49e5
1.495e5
1.5e5
MASS [Da]

FIG. 7B

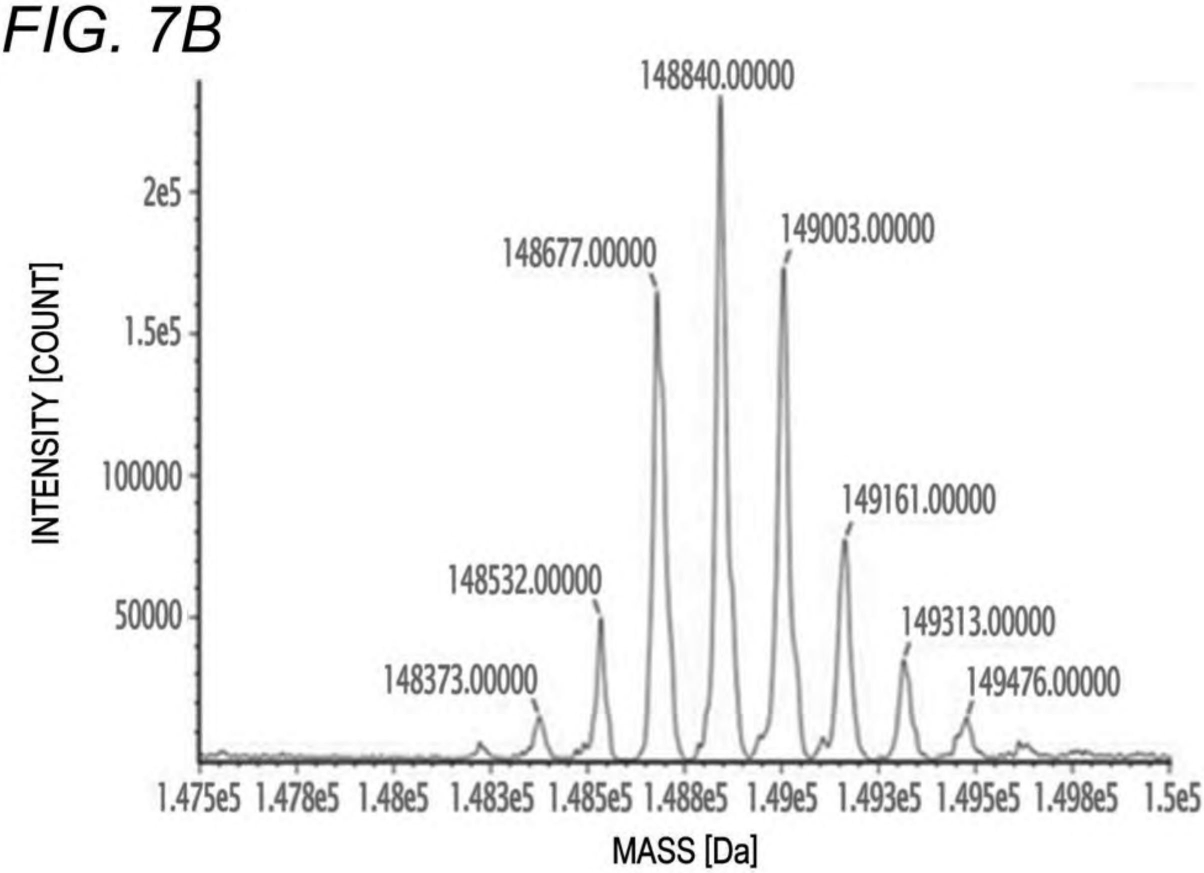
148840.00000
148677.00000
149003.00000
149161.00000
148532.00000
149313.00000
148373.00000
149476.00000
2e5
1.5e5
100000
50000
INTENSITY [COUNT]
1.475e5
1.478e5
1.48e5
1.483e5
1.485e5
1.488e5
1.49e5
1.493e5
1.495e5
1.498e5
1.5e5
MASS [Da]

FIG. 8A

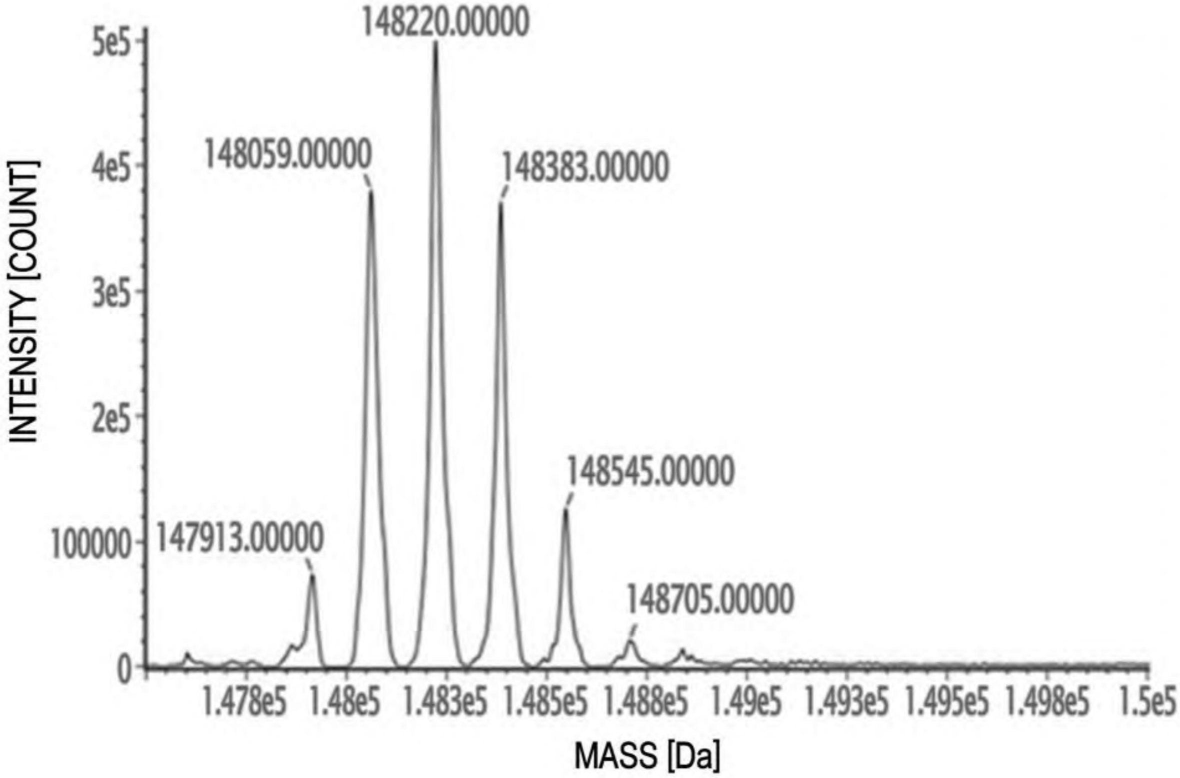
148220.00000
148059.00000
148383.00000
148545.00000
147913.00000
148705.00000
5e5
4e5
3e5
2e5
100000
0
1.478e5
1.48e5
1.483e5
1.485e5
1.488e5
1.49e5
1.493e5
1.495e5
1.498e5
1.5e5
INTENSITY [COUNT]
MASS [Da]

FIG. 8B

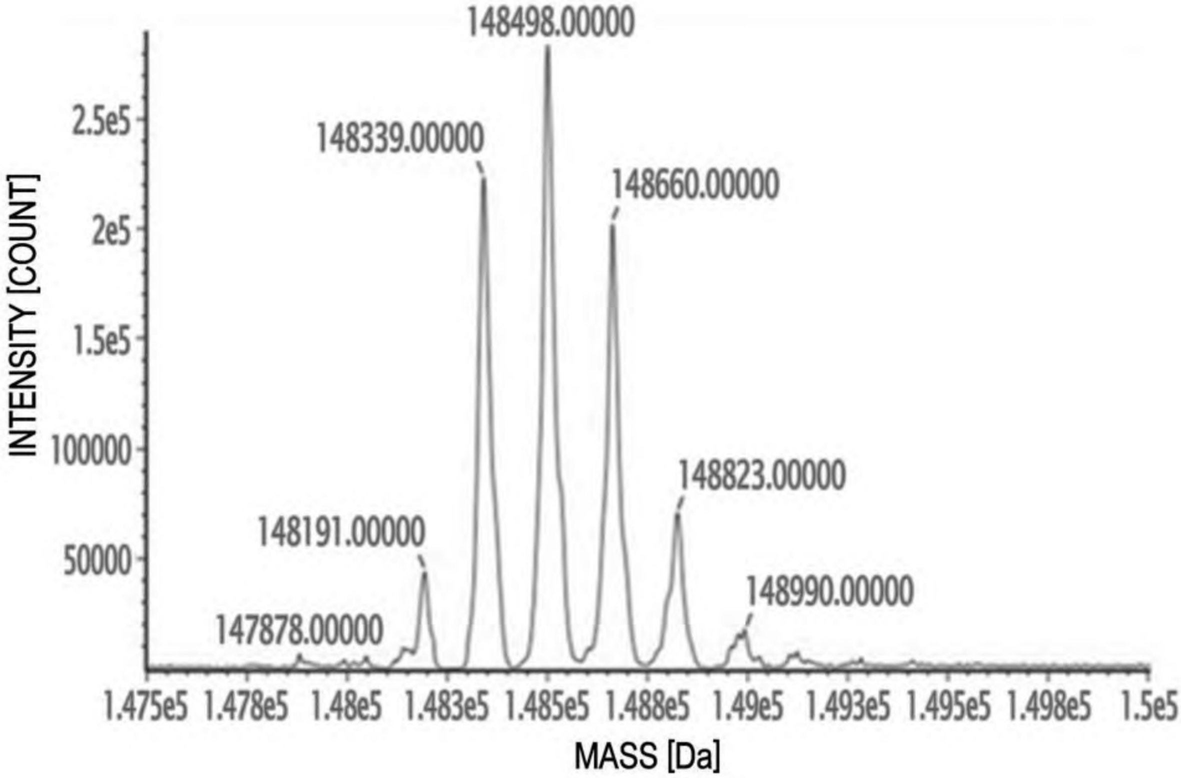
148498.00000
148339.00000
148660.00000
148823.00000
148191.00000
148990.00000
147878.00000
2.5e5
2e5
1.5e5
100000
50000
1.475e5
1.478e5
1.48e5
1.483e5
1.485e5
1.488e5
1.49e5
1.493e5
1.495e5
1.498e5
1.5e5
INTENSITY [COUNT]
MASS [Da]

FIG. 9A

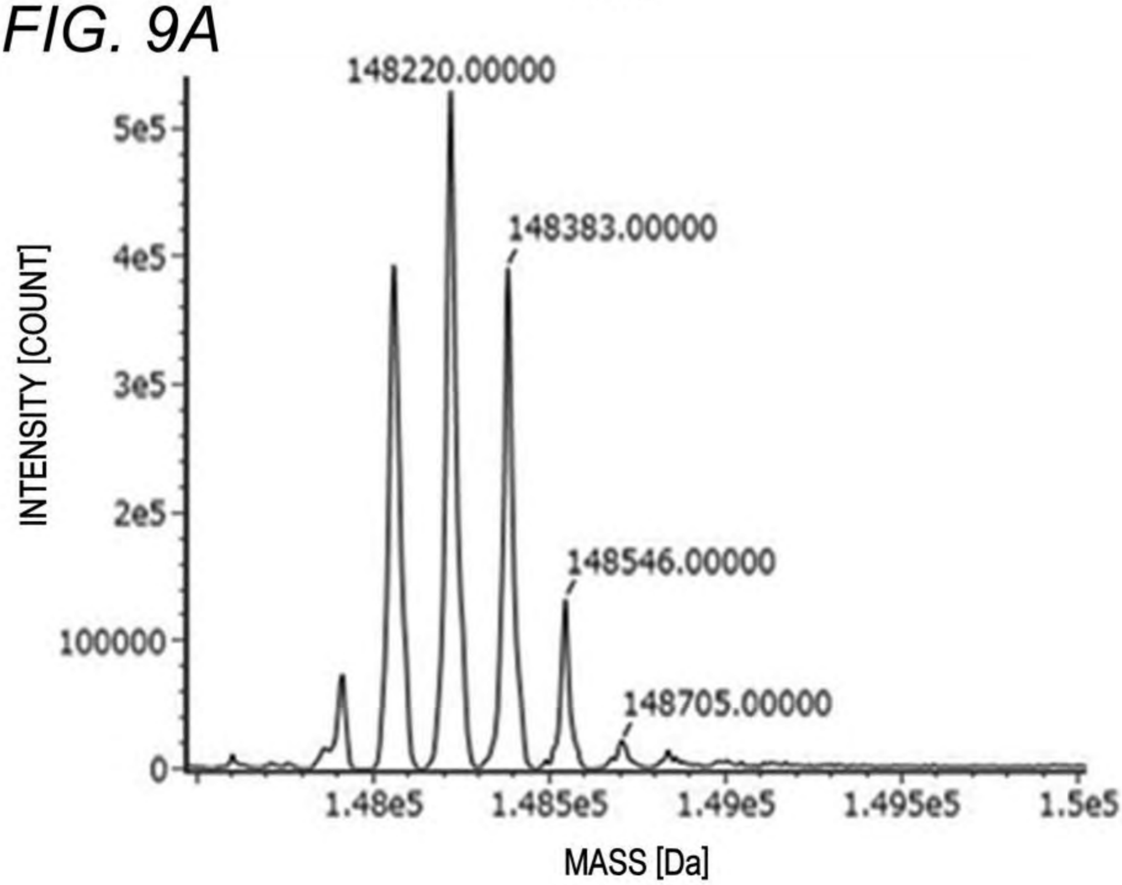
148220.00000
148383.00000
148546.00000
148705.00000
5e5
4e5
3e5
2e5
100000
0
INTENSITY [COUNT]
1.48e5
1.485e5
1.49e5
1.495e5
1.5e5
MASS [Da]

FIG. 9B

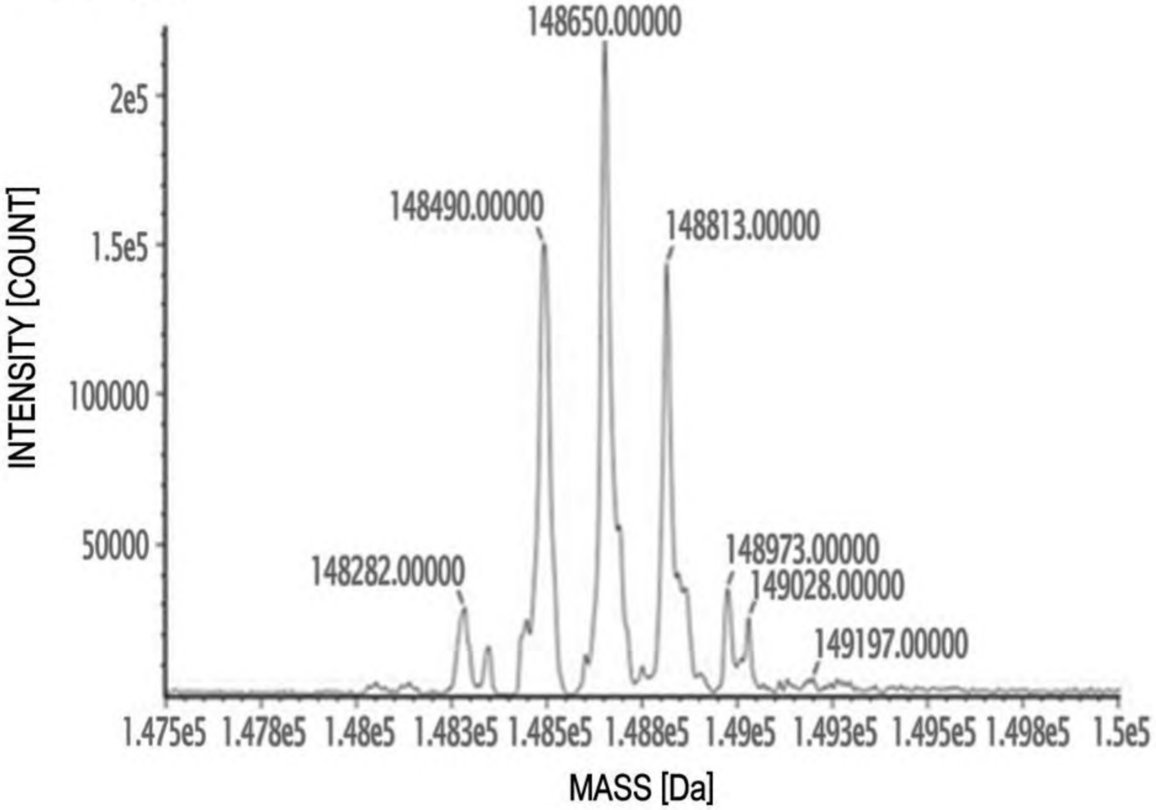
148650.00000
148490.00000
148813.00000
148282.00000
148973.00000
149028.00000
149197.00000
2e5
1.5e5
100000
50000
INTENSITY [COUNT]
1.475e5
1.478e5
1.48e5
1.483e5
1.485e5
1.488e5
1.49e5
1.493e5
1.495e5
1.498e5
1.5e5
MASS [Da]

FIG. 10A

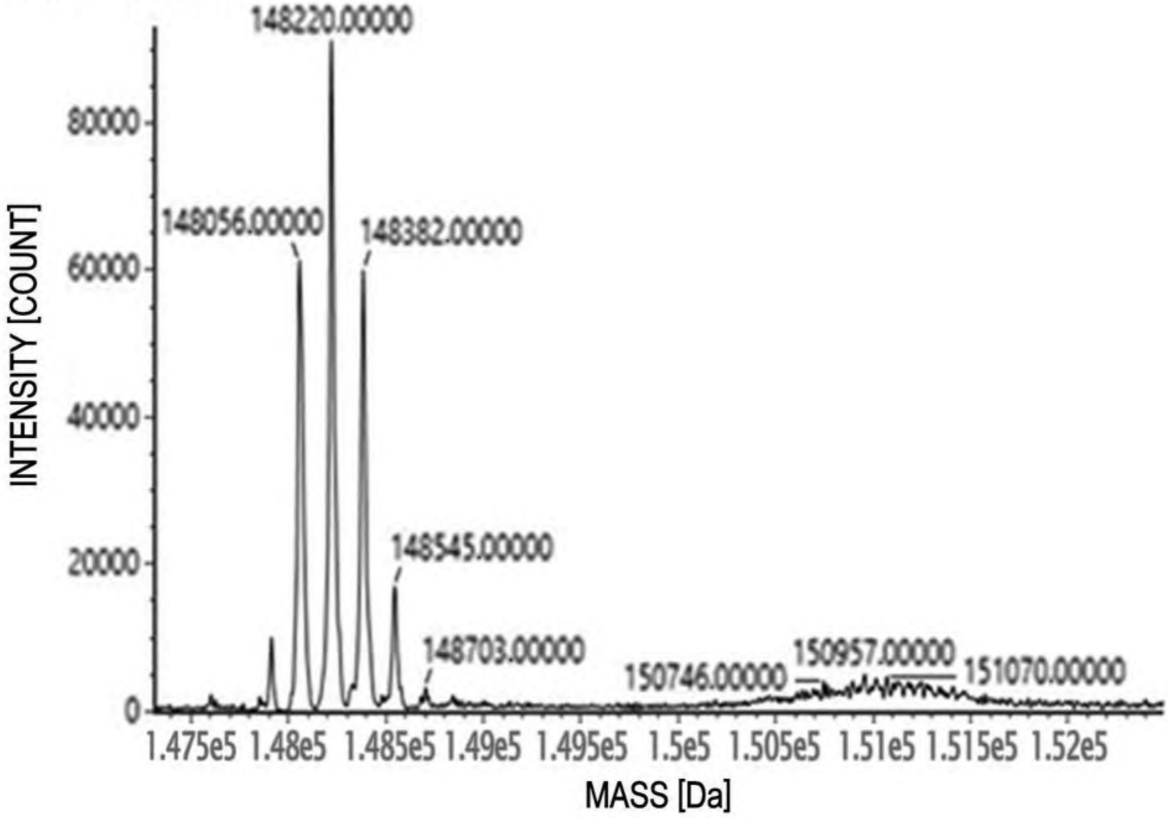


FIG. 10B

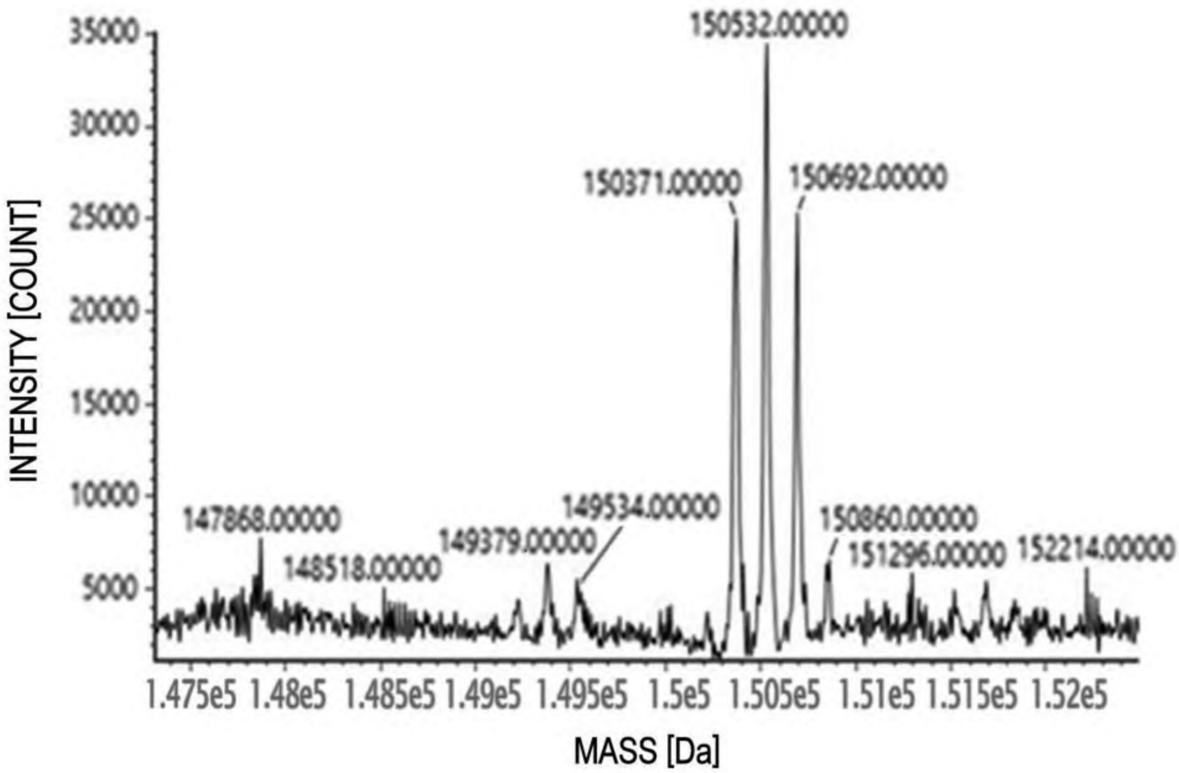

FIG. 11A

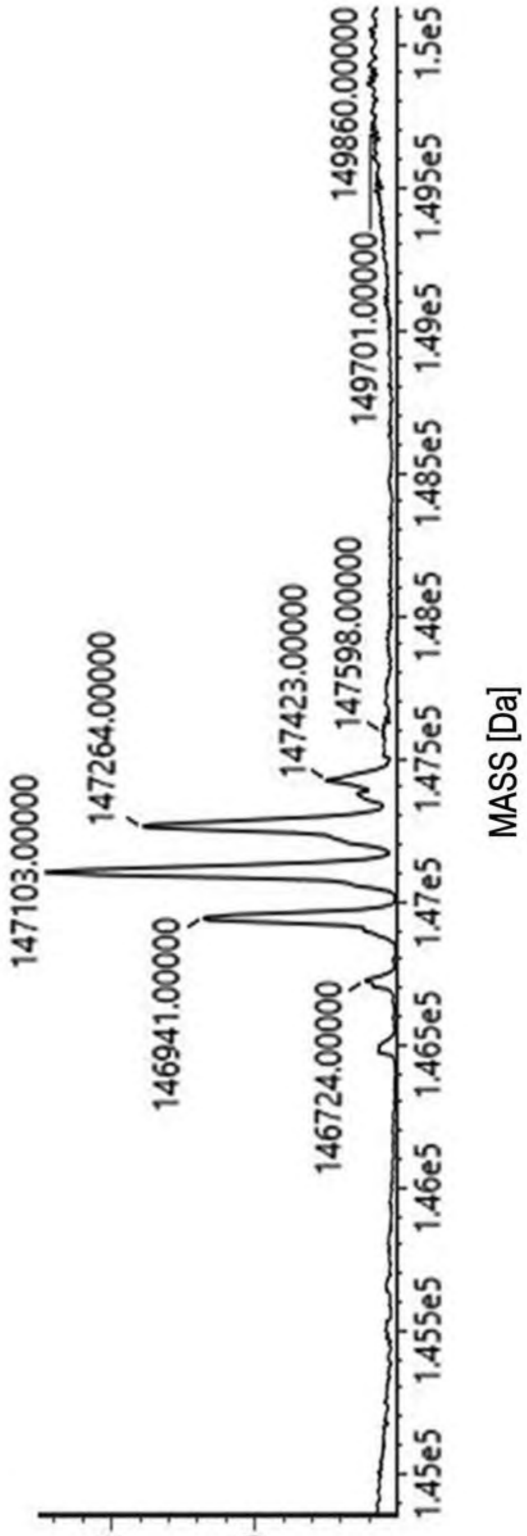
147103.00000
147264.00000
146941.00000
147423.00000
147598.00000
146724.00000
149701.00000
149860.00000
1.45e5
1.455e5
1.46e5
1.465e5
1.47e5
1.475e5
1.48e5
1.485e5
1.49e5
1.495e5
1.5e5
MASS [Da]
100000
2e5
INTENSITY [COUNT]

FIG. 11B

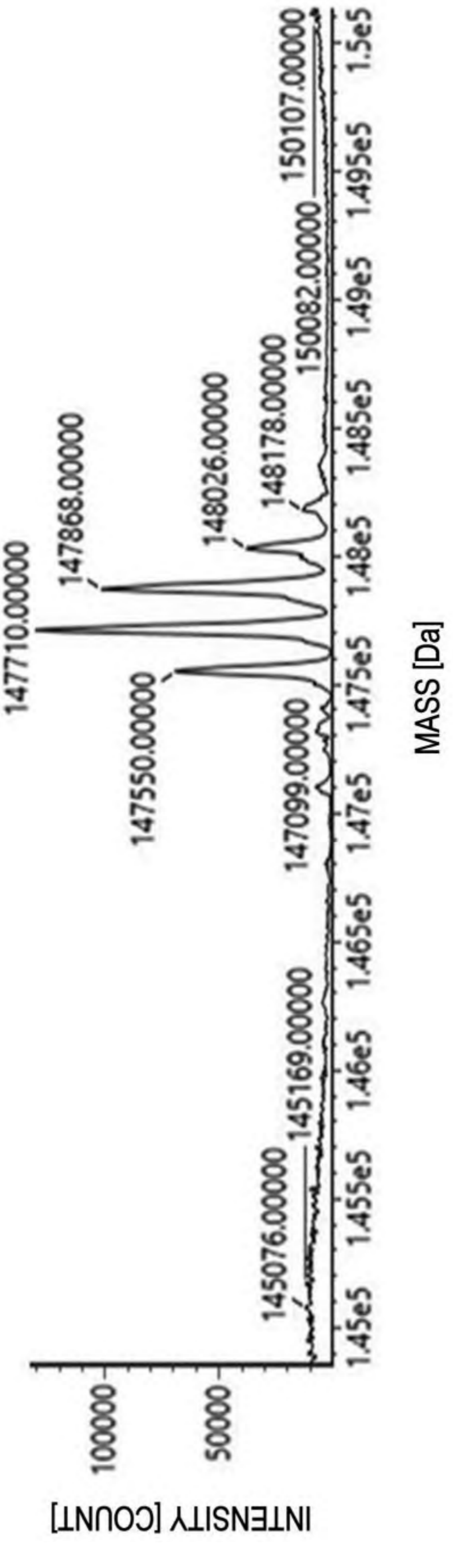
INTENSITY [COUNT]
100000
50000
145076.00000
145169.00000
147099.00000
147550.00000
147710.00000
147868.00000
148026.00000
148178.00000
150082.00000
150107.00000
1.45e5
1.455e5
1.46e5
1.465e5
1.47e5
1.475e5
1.48e5
1.485e5
1.49e5
1.495e5
1.5e5
MASS [Da]

FIG. 12A

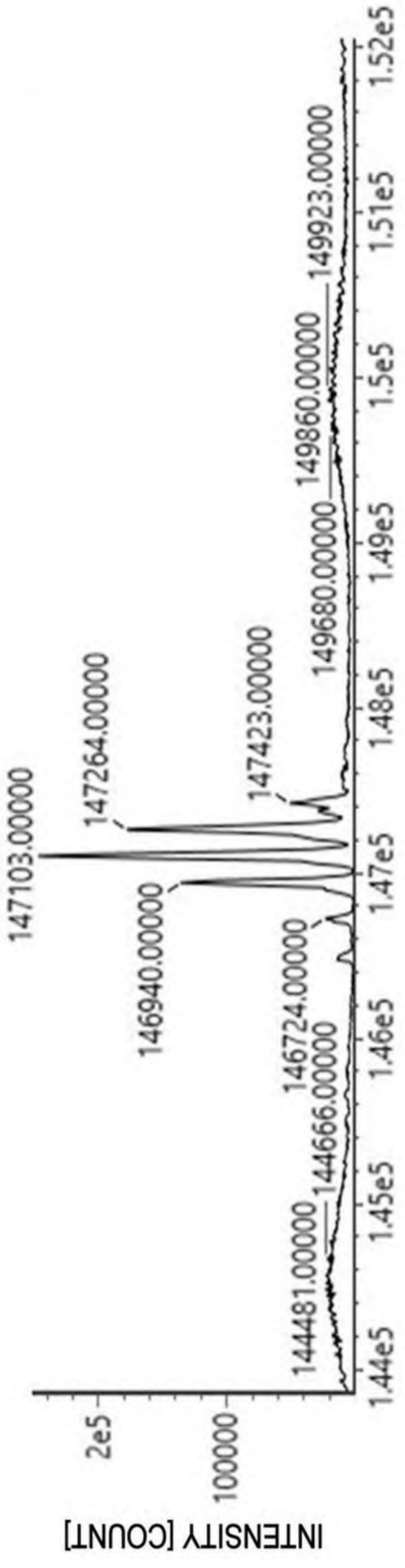
147103.00000
147264.00000
147423.00000
146940.00000
146724.00000
146666.00000
144481.00000
144666.00000
149680.00000
149860.00000
149923.00000
INTENSITY [COUNT]
2e5
100000
1.44e5
1.45e5
1.46e5
1.47e5
1.48e5
1.49e5
1.5e5
1.51e5
1.52e5
MASS [Da]

FIG. 12B

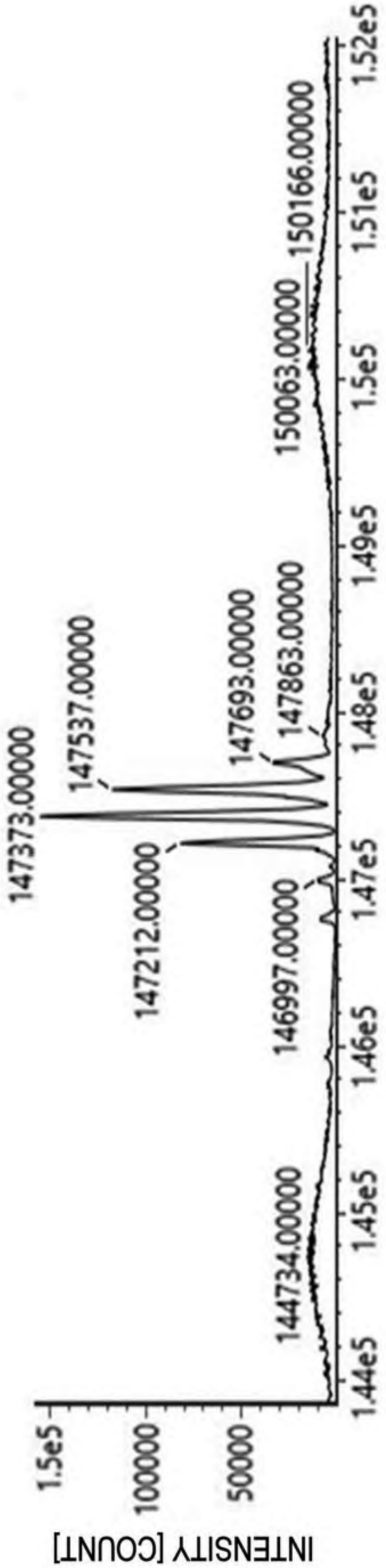
INTENSITY [COUNT]
1.5e5
100000
50000
MASS [Da]
1.44e5
1.45e5
1.46e5
1.47e5
1.48e5
1.49e5
1.5e5
1.51e5
1.52e5
144734.00000
146997.00000
147212.00000
147373.00000
147537.00000
147693.00000
147863.00000
150063.00000
150166.00000

FIG. 13A

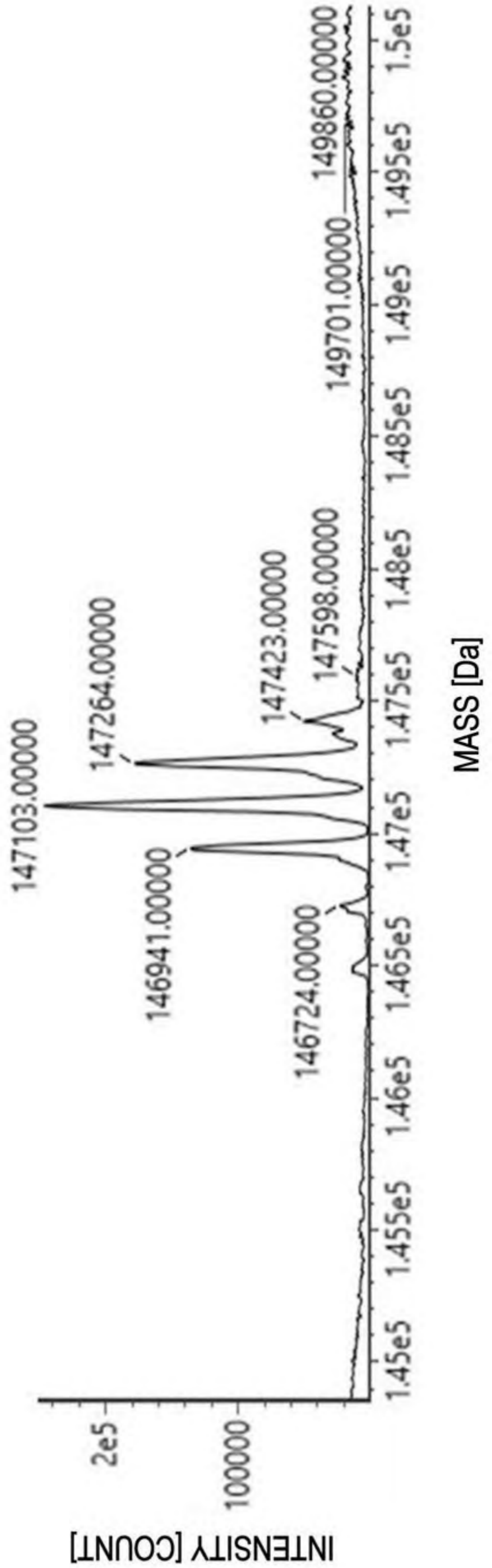
147103.00000
147264.00000
146941.00000
146724.00000
147423.00000
147598.00000
149701.00000
149860.00000
MASS [Da]
INTENSITY [COUNT]
2e5
100000
1.45e5
1.455e5
1.46e5
1.465e5
1.47e5
1.475e5
1.48e5
1.485e5
1.49e5
1.495e5
1.5e5

FIG. 13B

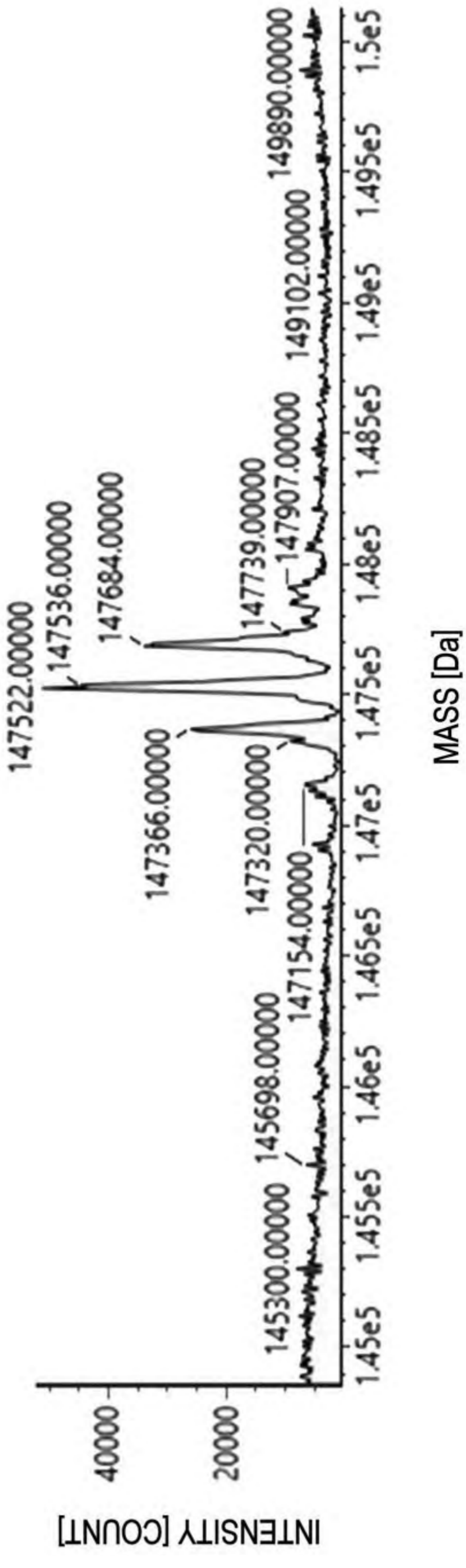

INTENSITY [COUNT]
40000
20000
MASS [Da]
1.45e5
1.455e5
1.46e5
1.465e5
1.47e5
1.475e5
1.48e5
1.485e5
1.49e5
1.495e5
1.5e5
145300.00000
145698.00000
147154.00000
147320.00000
147366.00000
147522.00000
147536.00000
147684.00000
147739.00000
147907.00000
149102.00000
149890.00000

FIG. 14

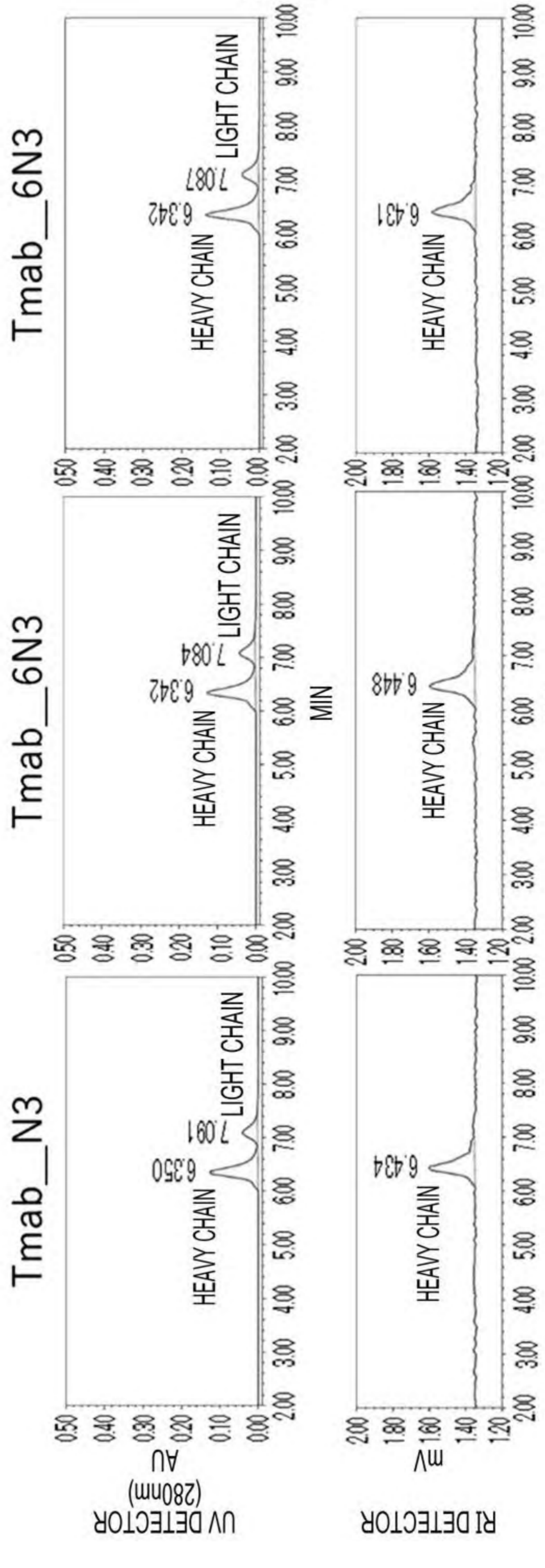
Tmab__N3
Tmab__6N3
Tmab__6N3
UV DETECTOR
(280nm)
AU
RI DETECTOR
mV
HEAVY CHAIN
LIGHT CHAIN
6.350
7.091
6.342
7.084
6.342
7.087
6.434
6.448
6.431
0.50
0.40
0.30
0.20
0.10
0.00
2.00
1.80
1.60
1.40
1.20
2.00
3.00
4.00
5.00
6.00
7.00
8.00
9.00
10.00
MIN

FIG. 15

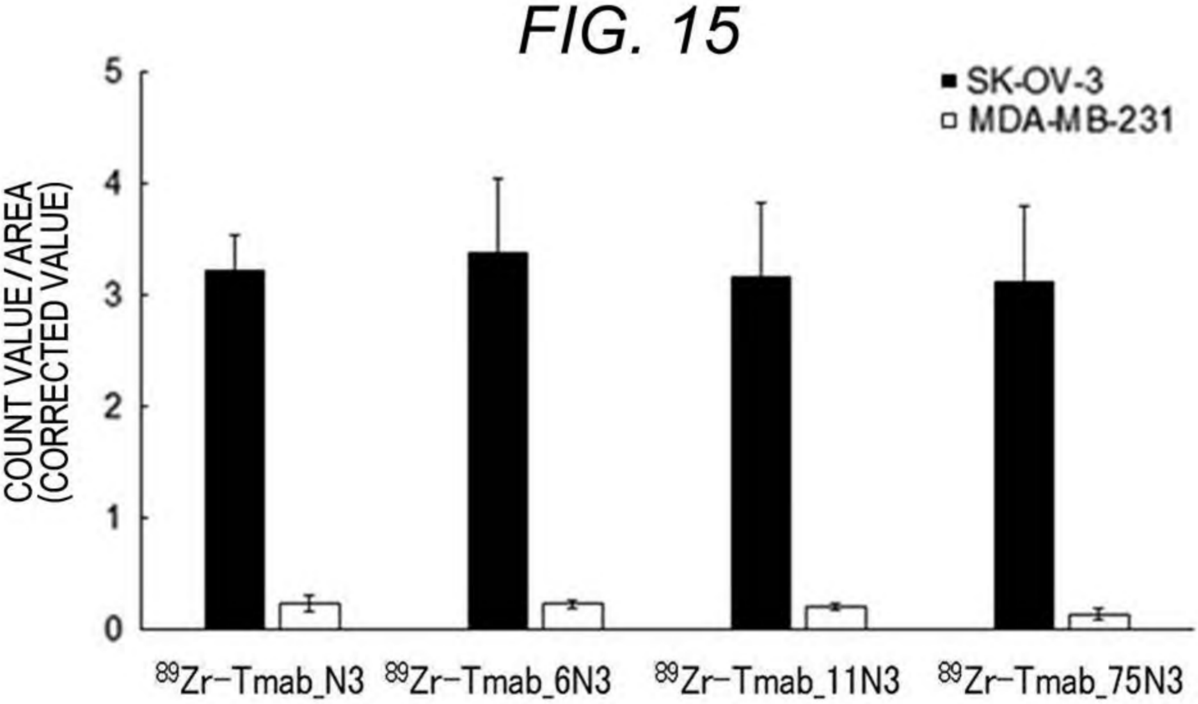


FIG. 16A

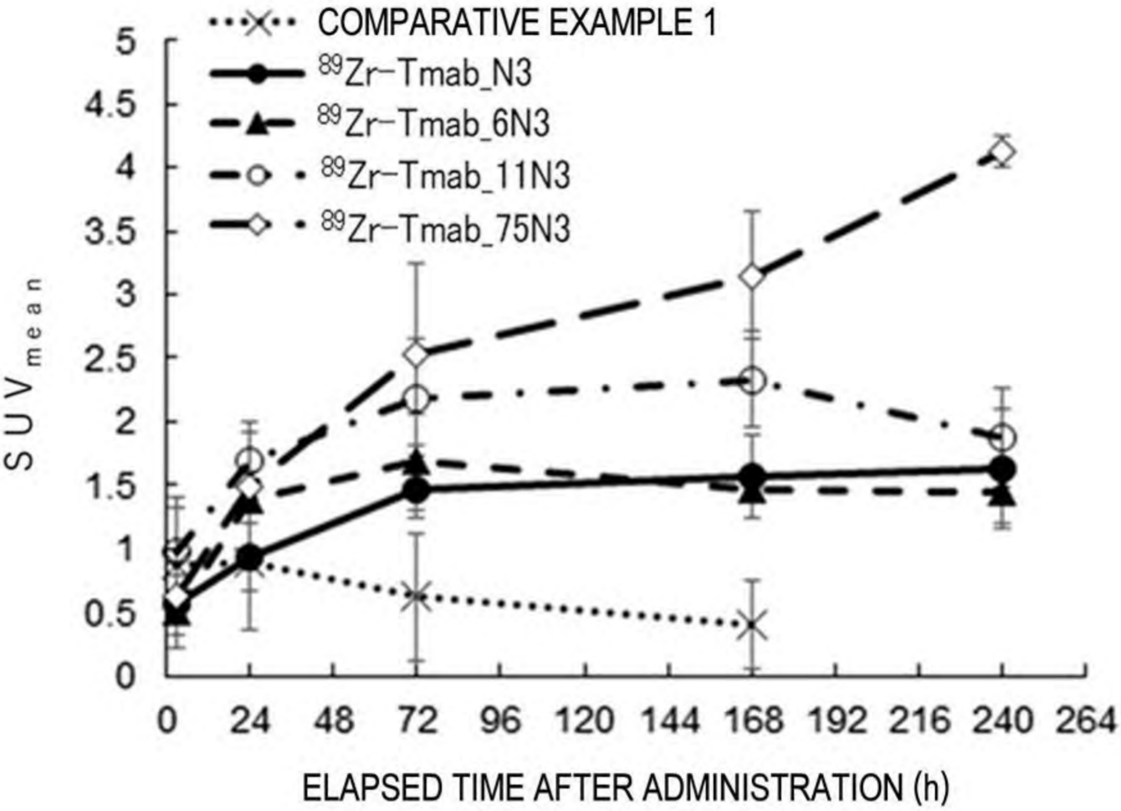

FIG. 16B

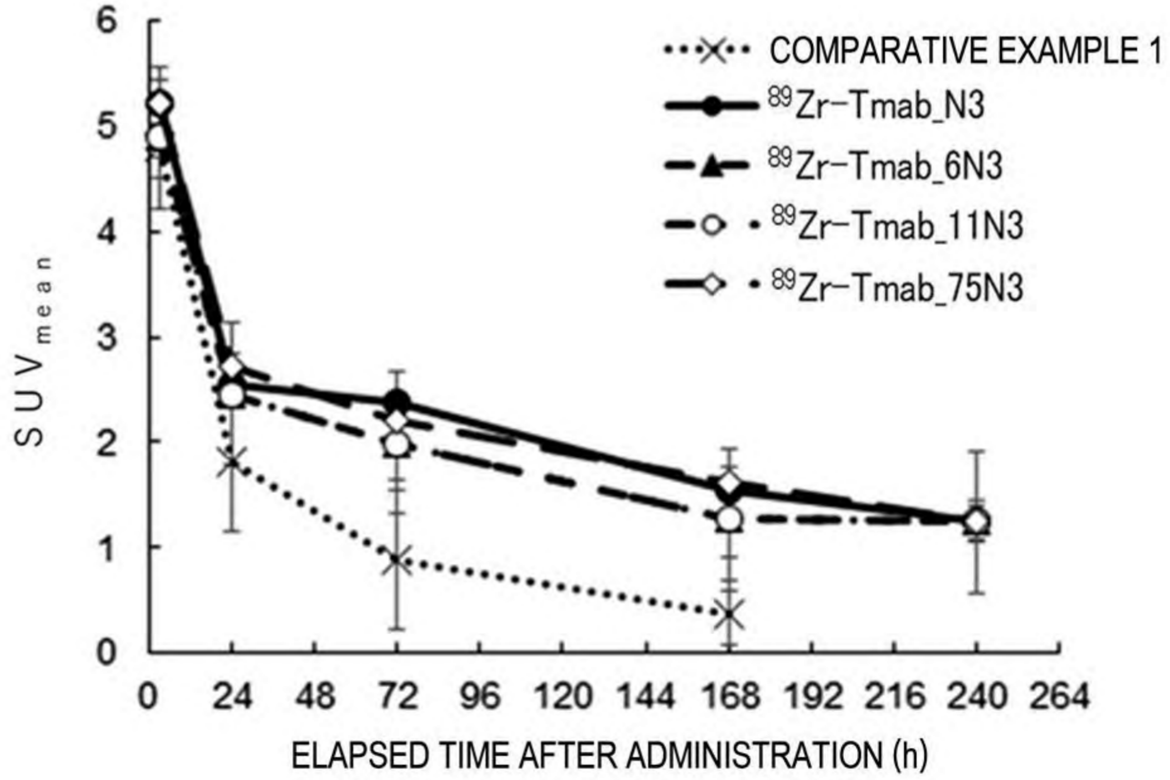


FIG. 16C

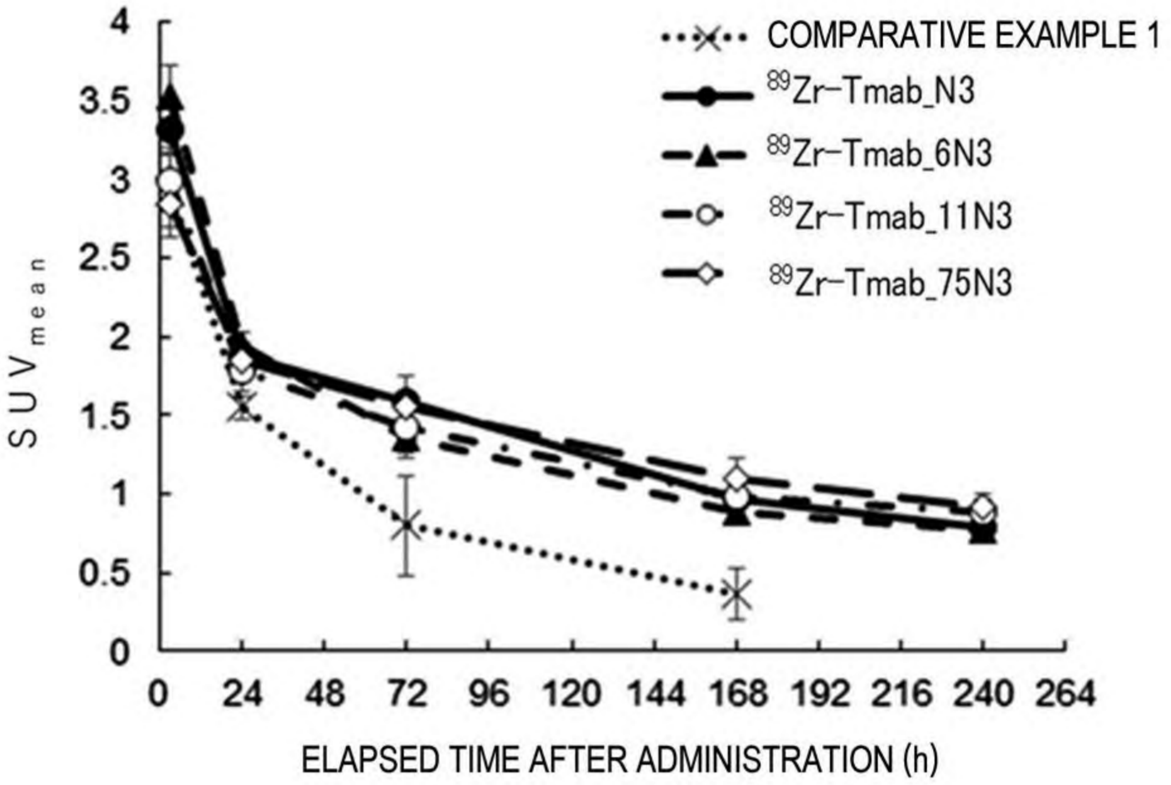

FIG. 16D

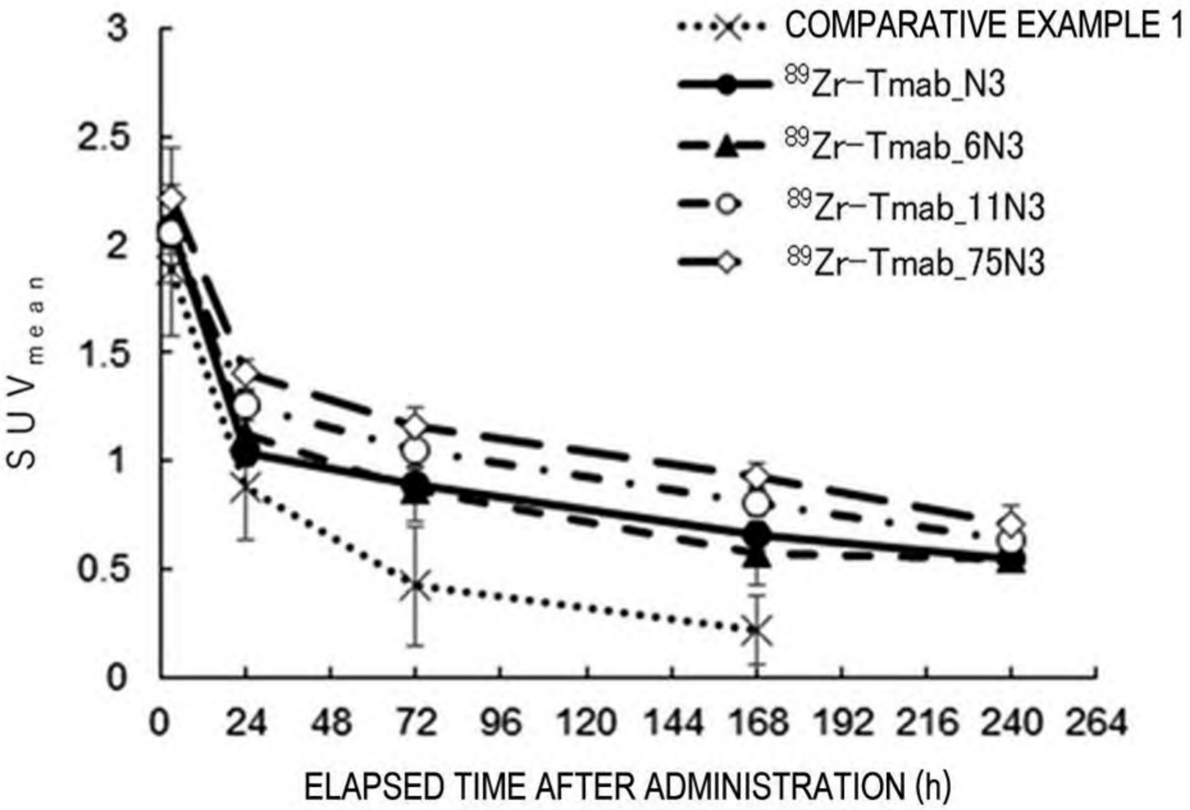


FIG. 17

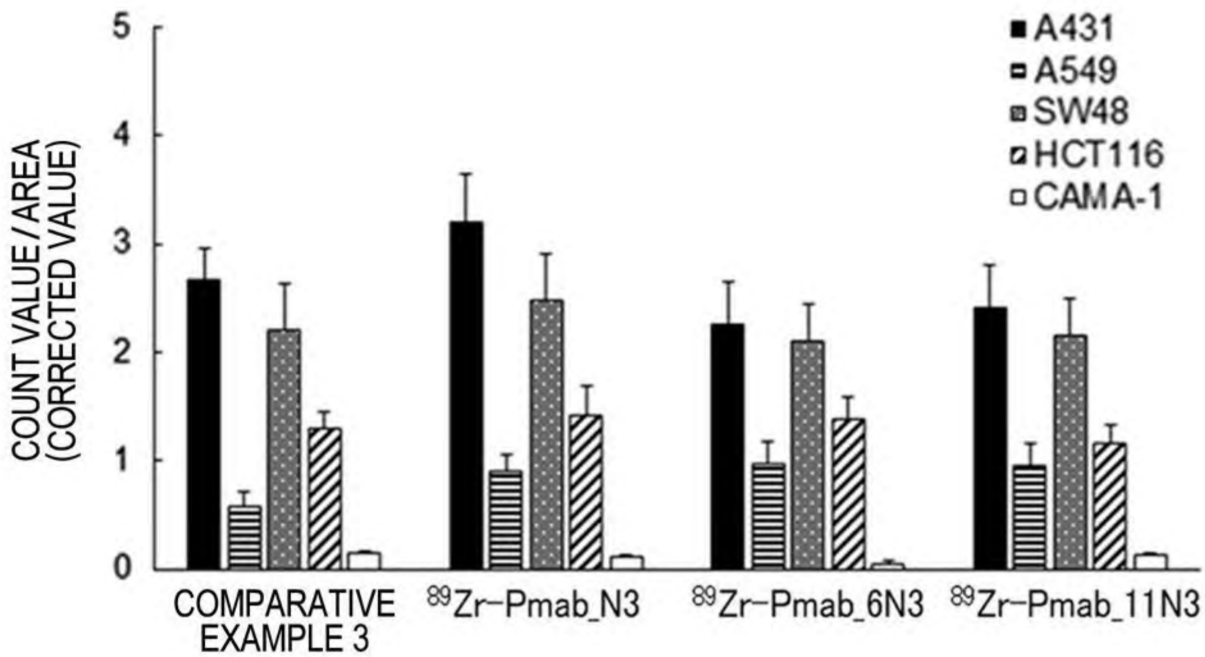

FIG. 18A

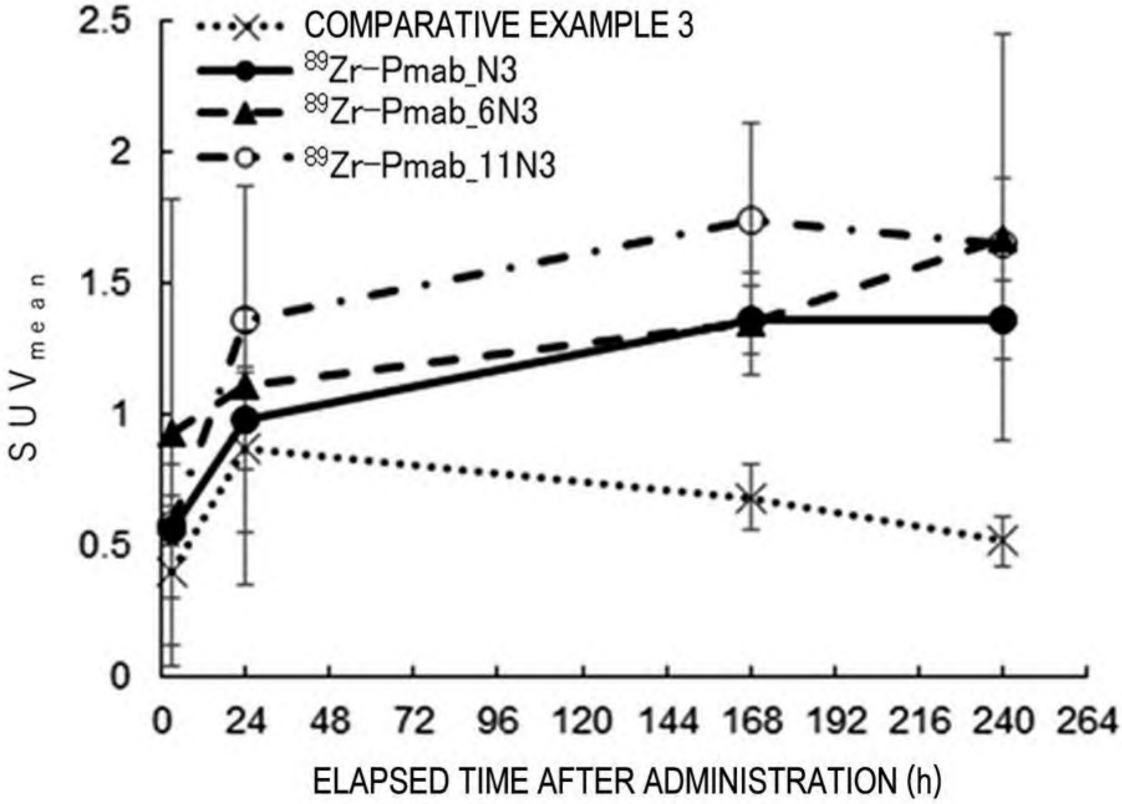


FIG. 18B

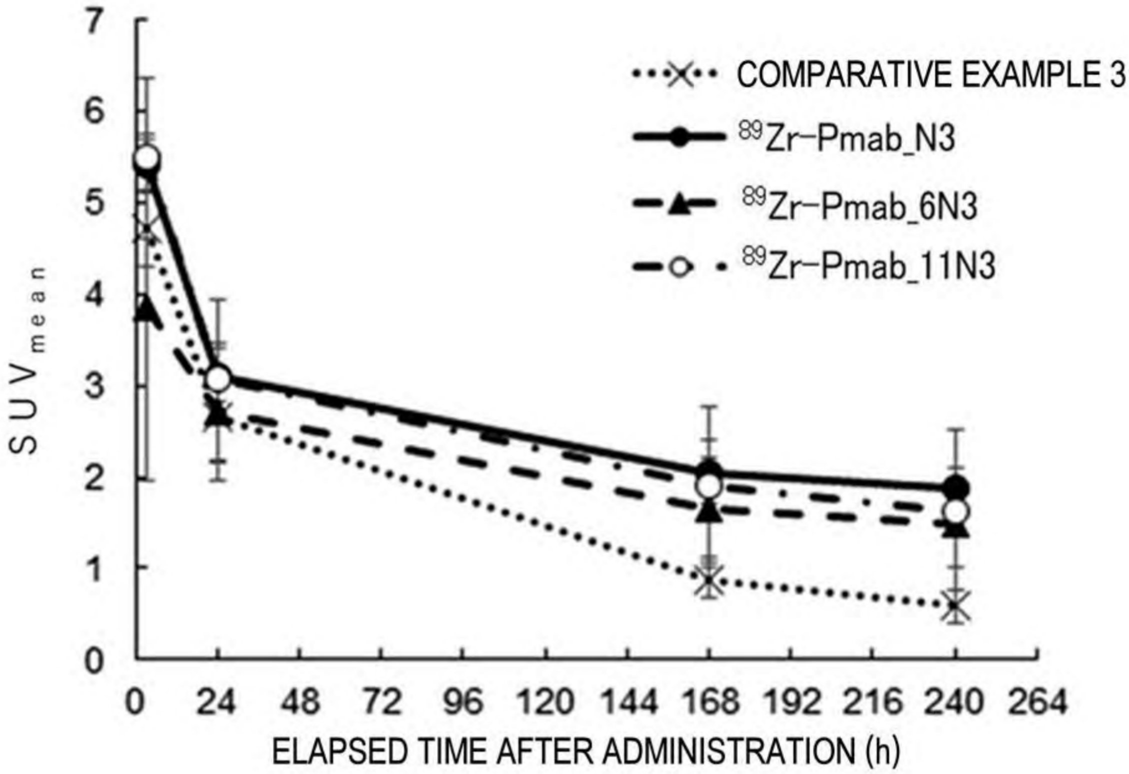

FIG. 18C

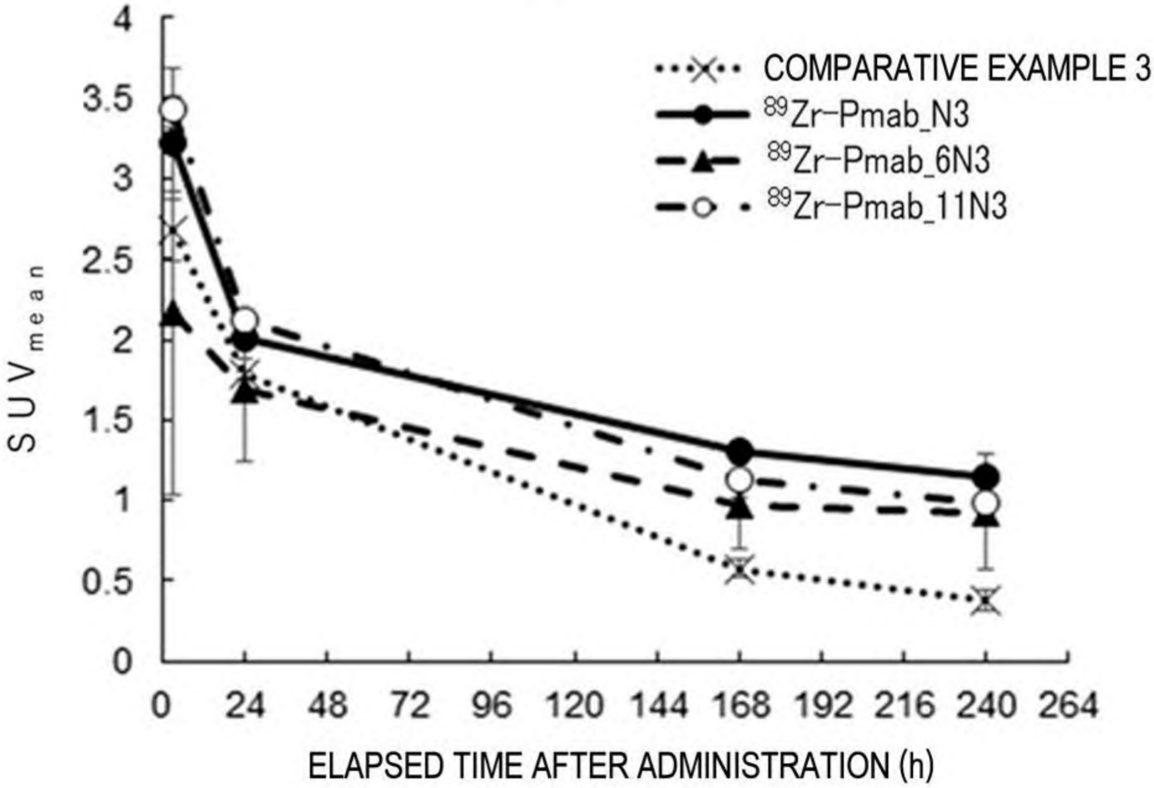


FIG. 18D

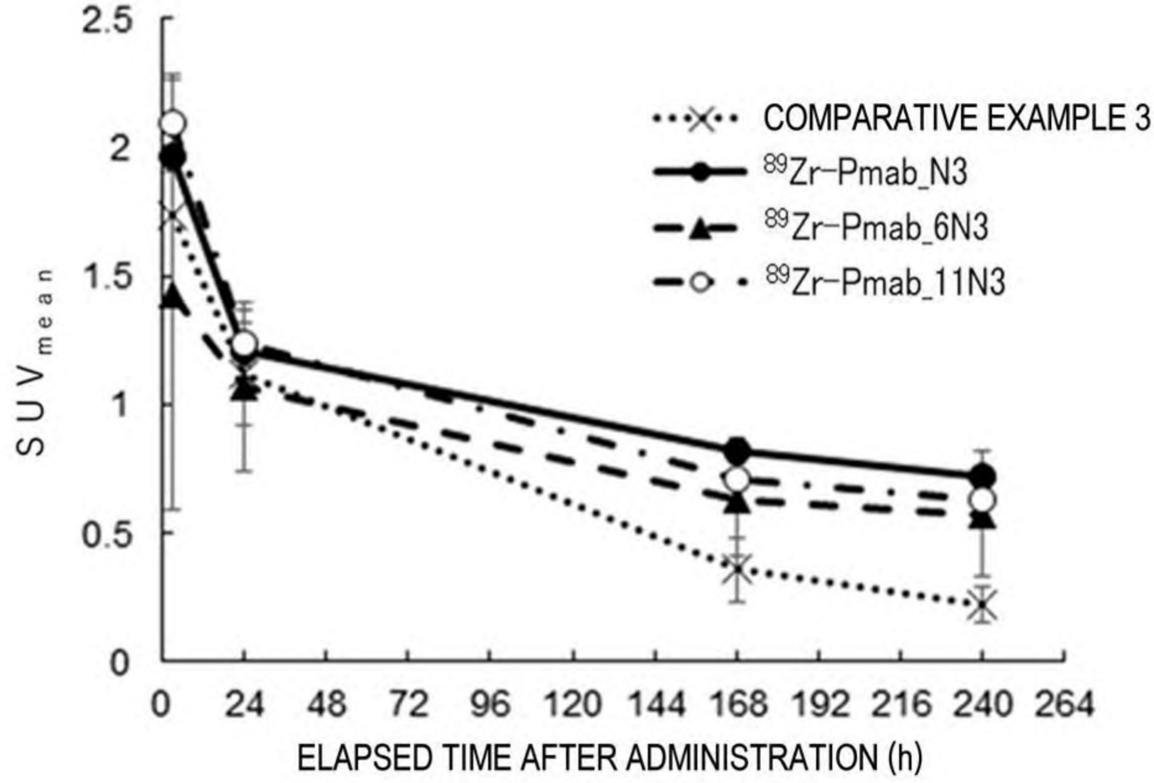

FIG. 19

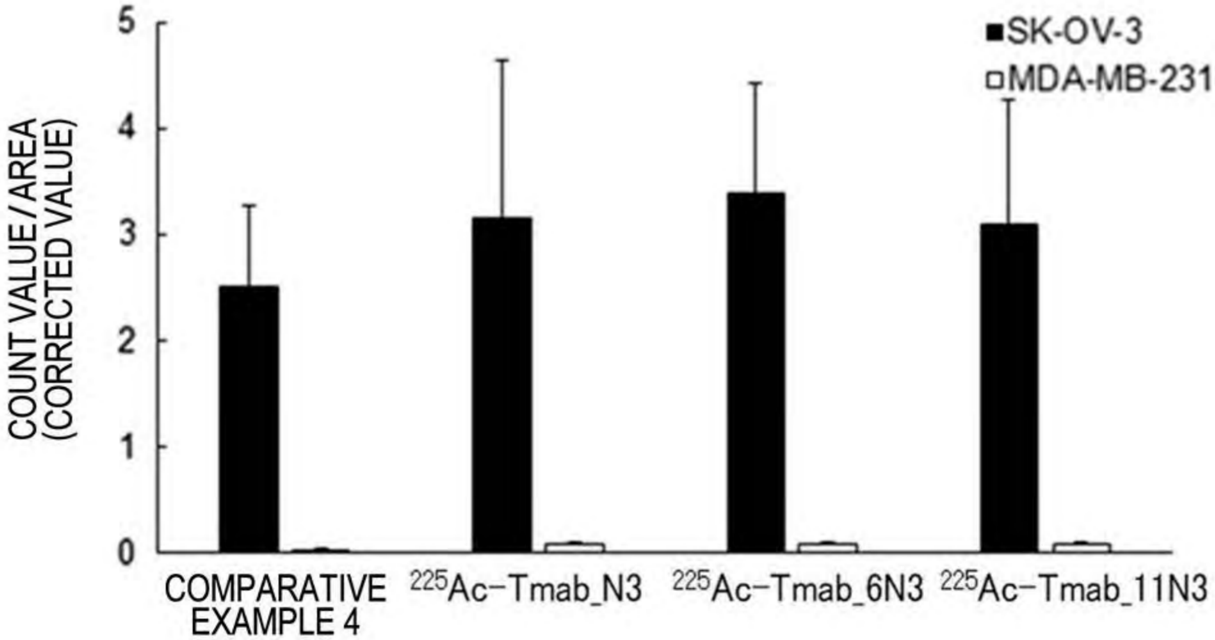
SK-OV-3
MDA-MB-231
COUNT VALUE / AREA (CORRECTED VALUE)
0
1
2
3
4
5
COMPARATIVE EXAMPLE 4
225Ac-Tmab_N3
225Ac-Tmab_6N3
225Ac-Tmab_11N3

FIG. 20

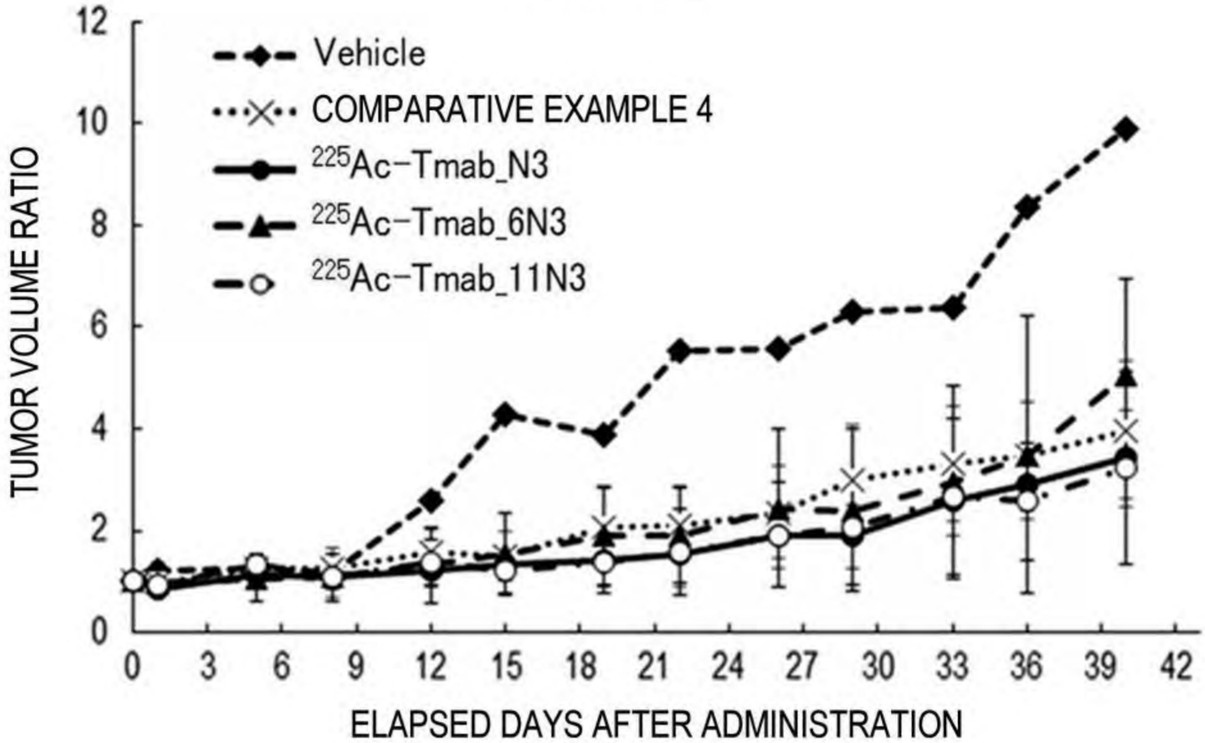
Vehicle
COMPARATIVE EXAMPLE 4
225Ac-Tmab_N3
225Ac-Tmab_6N3
225Ac-Tmab_11N3
TUMOR VOLUME RATIO
0
2
4
6
8
10
12
0 3 6 9 12 15 18 21 24 27 30 33 36 39 42
ELAPSED DAYS AFTER ADMINISTRATION

**INTERNATIONAL SEARCH REPORT**

International application No.
**PCT/JP2024/010181**

**A. CLASSIFICATION OF SUBJECT MATTER**

***C07K 19/00***(2006.01)i; ***C07K 7/08***(2006.01)i; ***C07K 16/00***(2006.01)i
FI: C07K19/00; C07K7/08; C07K16/00 ZNA

According to International Patent Classification (IPC) or to both national classification and IPC

**B. FIELDS SEARCHED**

Minimum documentation searched (classification system followed by classification symbols)

C07K19/00; C07K7/08; C07K16/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2023/033022 A1 (NIHON MEDI-PHYSICS CO., LTD.) 09 March 2023 (2023-03-09) claims 1-16, paragraphs [0015], [0019], [0021]-[0024], [0039]-[0043], [0074], [0075], [0086] | 16-26<br>1-15 |
| X<br>A | WO 2018/199337 A1 (AJINOMOTO CO., INC.) 01 November 2018 (2018-11-01) claims 1-117, paragraphs [0068], [0293], [0294], examples 5-54, fig. 1, 2, tables 3-7 | 16-26<br>1-15 |
| A | PALA-PUJADAS, J. et al. Peptide ligations by using aryloxycarbonyl-o-methylaminoanilides: Chemical synthesis of palmitoylated sonic hedgehog., ANGEWANDTE CHEMIE, INTERNATIONAL EDITION, 08 November 2018, vol. 57, no. 49, pp. 16120-16125, DOI: 10.1002/anie.201810712 abstract, schemes 1, 2, table 1 | 1-26 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/010181**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/010181**

| Patent document cited in search report | Publication date (day/month/year) | Patent family member(s) | Publication date (day/month/year) |
|---|---|---|---|
| WO 2023/033022 A1 | 09 March 2023 | TW 202325343 A | |
| WO 2018/199337 A1 | 01 November 2018 | US 2020/0190165 A1<br>claims 1-20, examples 5-54, fig. 1, 2, tables 3-7<br>EP 3617235 A1<br>KR 10-2020-0002858 A<br>CN 110637036 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016186206 A
- WO 2017217347 A
- WO 2018230257 A
- WO 2021080008 A
- WO 2008054030 A
- WO 2013027796 A